# NEW EUROPEAN PATENT SPECIFICATION

(11) **EP 1 737 969 B2**
(45) Date of publication and mention of the opposition decision: **12.09.2018**
(45) Mention of the grant of the patent: 17.06.2015
(21) Application number: 05732293.5
(22) Date of filing: 15.04.2005
(51) Int. Cl.: C12N 9/10, C12N 9/90, C12P 21/00

(54) **PRODUCTION OF GALACTOSYLATED GLYCOPROTEINS IN LOWER EUKARYOTES**
PRODUKTION GALAKTOSYLIERTER GLYKOPROTEINE IN NIEDEREN EUKARYONTEN
PRODUCTION DE GLYCOPROTEINES GALACTOSYLATEES DANS DES EUCARYOTES INFERIEURS

(30) Priority: 15.04.2004 US 562424 P
(43) Date of publication of application: 03.01.2007
(73) Proprietor: GlycoFi, Inc., Lebanon, NH 03766 (US)
(72) Inventor: DAVIDSON, Robert, Enfield, NH 03748 (US); GERNGROSS, Tillman, Hanover, NH 03755 (US); WILDT, Stefan, Lebanon, NH 03766 (US); CHOI, Byung-Kwon, Norwich, VT 05055 (US); NETT, Juergen, Grantham, NH 03753 (US); BOBROWICZ, Piotr, White River Junction, VT 05001 (US); HAMILTON, Stephen, Enfield, NH 03748 (US)
(74) Representative: Böhles, Elena
(86) International application number: PCT/IB2005/051249
(87) International publication number: WO 2005/100584

(56) References cited:
- WO-A1-01/31045
- WO-A1-99/64618
- WO-A1-03/056914
- WO-A1-2000/034490
- WO-A1-2001/031045
- WO-A2-02/00879
- US-A1- 2004 018 590
- LOPEZ-AVALOS MARIA DOLORES ET AL: "The UDPase activity of the Kluyveromyces lactis Golgi GDPase has a role in uridine nucleotide sugar transport into Golgi vesicles", GLYCOBIOLOGY, vol. 11, no. 5, May 2001 (2001-05), pages 413-422, XP002648814, ISSN: 0959-6658
- DATABASE UniProt [Online] 1 November 1999 (1999-11-01), "RecName: Full=Uncharacterized protein C365.14c;", XP002648815, retrieved from EBI accession no. UNIPROT:Q9Y7X5 Database accession no. Q9Y7X5
- VERVECKEN W ET AL: "IN VIVO SYNTHESIS OF MAMMALIAN-LIKE, HYBRID-TYPE N-GLYCANS IN PICHIA PASTORIS", APPLIED AND ENVIRONMENTAL MICROBIOLOGY, AMERICAN SOCIETY FOR MICROBIOLOGY, US, vol. 70, no. 5, 1 May 2004 (2004-05-01), pages 2639-2646, XP008032109, ISSN: 0099-2240, DOI: DOI:10.1128/AEM.70.5.2639-2646.2004
- BOBROWICZ P ET AL: "Engineering of an artificial glycosylation pathway blocked in core oligosaccharide assembly in the yeast Pichia pastoris: production of complex humanized glycoproteins with terminal galactose", GLYCOBIOLOGY, OXFORD UNIVERSITY PRESS, US, vol. 14, no. 9, 9 June 2004 (2004-06-09), pages 757-766, XP002354412, ISSN: 0959-6658, DOI: DOI:10.1093/GLYCOB/CWH104
- CHOI B.W. ET AL.: 'Use of Combinational genetic libraries to humanize N-linked glycosylation in the yeast Pichia pastoris' PNAS vol. 100, no. 9, April 2003, pages 5022 - 5027, XP002267831
- MISAKI R. ET AL: 'Plant cultured cells expressing human beta1,,4-galactosyltransferase secrete glycoproteins with galactose-extended N-linked glycans' GLYCOBIOLOGY vol. 13, no. 3, 2003, pages 199 - 205
- BAKKER H. ET AL: 'Galactose-extended glycans of antibodies produced by transgenic plants' PROC. NATL. ACA. SCI. USA vol. 98, no. 5, 27 February 2001, pages 2899 - 2904
- PALACPAC N.Q. ET AL: 'Stable expression of human beta1,4-galactosyltransferase in plant cells modifies N-linked glycosylation patterns' PROC. NATL. ACAD. SCI. USA vol. 96, April 1999, pages 4692 - 4697

### SEQUENCE LISTING

<110> GlycoFi, Inc.
   Davidson, Robert
   Gerngross, Tillman
   Wildt, Stefan
   Choi, Byung-Kwon
   Nett, Juergen
   Bobrowicz, Piotr
Hamilton, Stephen
<120> Production of Galactosyiated Glycoproteins in Lower Eukaryotes
<130> GFI - 12
<160> 57
<170> PatentIn version 3.3
<210> 1
   <211> 27
   <212> DNA
   <213> Artificial
<220>
   <223> degenerate oligonucleotide primer
<400> 1
   gccgcgacct gagccgcctg ccccaac 27
<210> 2
   <211> 27
   <212> DNA
   <213> Artificial
<220>
   <223> degenerate oligonucleotide primer
<400> 2
   ctagctcggt gtcccgatgt ccactg 27
<210> 3
   <211> 36
   <212> DNA
   <213> Artificial
<220>
   <223> degenerate oligonucleotide primer
<400> 3
   cttaggcgcg ccggccgcga cctgagccgc ctgccc 36
<210> 4
   <211> 21
   <212> DNA
   <213> Artificial
<220>
   <223> degenerate oligonucleotide primer
<400> 4
   ggggcatatc tgccgcccat c 21
<210> 5
   <211> 21
   <212> DNA
   <213> Artificial
<220>
   <223> degenerate oligonucleotide primer
<400> 5
   gatgggcggc agatatgccc c 21
<210> 6
   <211> 36
   <212> DNA
   <213> Artificial
<220>
   <223> degenerate oligonucleotide primer
<400> 6
   cttcttaatt aactagctcg gtgtcccgat gtccac 36
<210> 7
   <211> 39
   <212> DNA
   <213> Artificial
<220>
   <223> degenerate oligonucleotide primer
<400> 7
   ccttgcggcc gcatggctgt caagggcgac gatgtcaaa 39
<210> 8
   <211> 40
   <212> DNA
   <213> Artificial
<220>
   <223> degenerate oligonucleotide primer
<400> 8
   attcgagaat agttaagtgt caaaatcaat gcactatttt 40
<210> 9
   <211> 40
   <212> DNA
   <213> Artificial
<220>
   <223> degenerate oligonucleotide primer
<400> 9
   aaaatagtgc attgattttg acacttaact attctcgaat 40
<210> 10
   <211> 39
   <212> DNA
   <213> Artificial
<220>
   <223> degenerate oligonucleotide primer
<400> 10
   ccttttaatt aattaatgct tatgatcaac gtccttagc 39
<210> 11
   <211> 49
   <212> DNA
   <213> Artificial
<220>
   <223> degenerate oligonucleotide primer
<400> 11
   ggctcgagcg gccgccacca tgaatagcat acacatgaac gccaatacg 49
<210> 12
   <211> 47
   <212> DNA
   <213> Artificial
<220>
   <223> degenerate oligonucleotide primer
<400> 12
   ccctcgagtt aattaactag acgcgcggca gcagcttctc ctcatcg 47
<210> 13
   <211> 355
   <212> PRT
   <213> Schizosaccharomyces pombe
<400> 13
   Met Thr Gly Val His Glu Gly Thr Val Leu Val Thr Gly Gly Ala Gly
   1 5 10 15
   Tyr Ile Gly Ser His Thr Cys Val Val Leu Leu Glu Lys Gly Tyr Asp
   20 25 30
   Val Val Ile Val Asp Asn Leu Cys Asn Ser Arg Val Glu Ala Val His
   35 40 45
   Arg Ile Glu Lys Leu Thr Gly Lys Lys Val Ile Phe His Gln Val Asp
   50 55 60
   Leu Leu Asp Glu Pro Ala Leu Asp Lys Val Phe Ala Asn Gln Asn Ile
   65 70 75 80
   Ser Ala Val Ile His Phe Ala Gly Leu Lys Ala Val Gly Glu Ser Val
   85 90 95
   Gln Val Pro Leu Ser Tyr Tyr Lys Asn Asn Ile Ser Gly Thr Ile Asn
   100 105 110
   Leu Ile Glu Cys Met Lys Lys Tyr Asn Val Arg Asp Phe Val Phe Ser
   115 120 125
   Ser Ser Ala Thr Val Tyr Gly Asp Pro Thr Arg Pro Gly Gly Thr Ile
   130 135 140
   Pro Ile Pro Glu Ser Cys Pro Arg Glu Gly Thr Ser Pro Tyr Gly Arg
   145 150 155 160
   Thr Lys Leu Phe Ile Glu Asn Ile Ile Glu Asp Glu Thr Lys Val Asn
   165 170 175
   Lys Ser Leu Asn Ala Ala Leu Leu Arg Tyr Phe Asn Pro Gly Gly Ala
   180 185 190
   His Pro Ser Gly Glu Leu Gly Glu Asp Pro Leu Gly Ile Pro Asn Asn
   195 200 205
   Leu Leu Pro Tyr Ile Ala Gln Val Ala Val Gly Arg Leu Asp His Leu
   210 215 220
   Asn Val Phe Gly Asp Asp Tyr Pro Thr Ser Asp Gly Thr Pro Ile Arg
   225 230 235 240
   Asp Tyr Ile His Val Cys Asp Leu Ala Glu Ala His Val Ala Ala Leu
   245 250 255
   Asp Tyr Leu Arg Gln His Phe Val Ser Cys Arg Pro Trp Asn Leu Gly
   260 265 270
   Ser Gly Thr Gly Ser Thr Val Phe Gln Val Leu Asn Ala Phe Ser Lys
   275 280 285
   Ala Val Gly Arg Asp Leu Pro Tyr Lys Val Thr Pro Arg Arg Ala Gly
   290 295 300
   Asp Val Val Asn Leu Thr Ala Asn Pro Thr Arg Ala Asn Glu Glu Leu
   305 310 315 320
   Lys Trp Lys Thr Ser Arg Ser Ile Tyr Glu Ile Cys Val Asp Thr Trp
   325 330 335
   Arg Trp Gln Gln Lys Tyr Pro Tyr Gly Phe Asp Leu Thr His Thr Lys
   340 345 350
   Thr Tyr Lys
   355
<210> 14
   <211> 1068
   <212> PRT
   <213> Schizosaccharomyces pombe
<400> 14
   Ala Thr Gly Ala Cys Thr Gly Gly Thr Gly Thr Thr Cys Ala Thr Gly
   1 5 10 15
   Ala Ala Gly Gly Gly Ala Cys Thr Gly Thr Gly Thr Thr Gly Gly Thr
   20 25 30
   Thr Ala Cys Thr Gly Gly Cys Gly Gly Cys Gly Cys Thr Gly Gly Thr
   35 40 45
   Thr Ala Thr Ala Thr Ala Gly Gly Thr Thr Cys Thr Cys Ala Thr Ala
   50 55 60
   Cys Gly Thr Gly Cys Gly Thr Thr Gly Thr Thr Thr Thr Gly Thr Thr
   65 70 75 80
   Ala Gly Ala Ala Ala Ala Ala Gly Gly Ala Thr Ala Thr Gly Ala Thr
   85 90 95
   Gly Thr Thr Gly Thr Ala Ala Thr Thr Gly Thr Cys Gly Ala Thr Ala
   100 105 110
   Ala Thr Thr Thr Ala Thr Gly Cys Ala Ala Thr Thr Cys Thr Cys Gly
   115 120 125
   Cys Gly Thr Thr Gly Ala Ala Gly Cys Cys Gly Thr Gly Cys Ala Cys
   130 135 140
   Cys Gly Cys Ala Thr Thr Gly Ala Ala Ala Ala Ala Cys Thr Cys Ala
   145 150 155 160
   Cys Thr Gly Gly Gly Ala Ala Ala Ala Ala Ala Gly Thr Cys Ala Thr
   165 170 175
   Ala Thr Thr Cys Cys Ala Cys Cys Ala Gly Gly Thr Gly Gly Ala Thr
   180 185 190
   Thr Thr Gly Cys Thr Thr Gly Ala Thr Gly Ala Gly Cys Cys Ala Gly
   195 200 205
   Cys Thr Thr Thr Gly Gly Ala Cys Ala Ala Gly Gly Thr Cys Thr Thr
   210 215 220
   Cys Gly Cys Ala Ala Ala Thr Cys Ala Ala Ala Ala Cys Ala Thr Ala
   225 230 235 240
   Thr Cys Thr Gly Cys Thr Gly Thr Cys Ala Thr Thr Cys Ala Thr Thr
   245 250 255
   Thr Thr Gly Cys Thr Gly Gly Thr Cys Thr Cys Ala Ala Ala Gly Cys
   260 265 270
   Ala Gly Thr Thr Gly Gly Thr Gly Ala Ala Thr Cys Thr Gly Thr Ala
   275 280 285
   Cys Ala Gly Gly Thr Thr Cys Cys Thr Thr Thr Gly Ala Gly Thr Thr
   290 295 300
   Ala Thr Thr Ala Cys Ala Ala Ala Ala Ala Thr Ala Ala Cys Ala Thr
   305 310 315 320
   Thr Thr Cys Cys Gly Gly Thr Ala Cys Cys Ala Thr Thr Ala Ala Thr
   325 330 335
   Thr Thr Ala Ala Thr Ala Gly Ala Gly Thr Gly Cys Ala Thr Gly Ala
   340 345 350
   Ala Gly Ala Ala Gly Thr Ala Thr Ala Ala Thr Gly Thr Ala Cys Gly
   355 360 365
   Thr Gly Ala Cys Thr Thr Cys Gly Thr Cys Thr Thr Thr Thr Cys Thr
   370 375 380
   Thr Cys Ala Thr Cys Thr Gly Cys Thr Ala Cys Cys Gly Thr Gly Thr
   385 390 395 400
   Ala Thr Gly Gly Cys Gly Ala Thr Cys Cys Thr Ala Cys Thr Ala Gly
   405 410 415
   Ala Cys Cys Thr Gly Gly Thr Gly Gly Thr Ala Cys Cys Ala Thr Thr
   420 425 430
   Cys Cys Thr Ala Thr Thr Cys Cys Ala Gly Ala Gly Thr Cys Ala Thr
   435 440 445
   Gly Cys Cys Cys Thr Cys Gly Thr Gly Ala Ala Gly Gly Thr Ala Cys
   450 455 460
   Ala Ala Gly Cys Cys Cys Ala Thr Ala Thr Gly Gly Thr Cys Gly Cys
   465 470 475 480
   Ala Cys Ala Ala Ala Gly Cys Thr Thr Thr Thr Cys Ala Thr Thr Gly
   485 490 495
   Ala Ala Ala Ala Thr Ala Thr Cys Ala Thr Thr Gly Ala Gly Gly Ala
   500 505 510
   Thr Gly Ala Gly Ala Cys Cys Ala Ala Gly Gly Thr Gly Ala Ala Cys
   515 520 525
   Ala Ala Ala Thr Cys Gly Cys Thr Thr Ala Ala Thr Gly Cys Ala Gly
   530 535 540
   Cys Thr Thr Thr Ala Thr Thr Ala Cys Gly Cys Thr Ala Thr Thr Thr
   545 550 555 560
   Thr Ala Ala Thr Cys Cys Cys Gly Gly Ala Gly Gly Thr Gly Cys Thr
   565 570 575
   Cys Ala Thr Cys Cys Cys Thr Cys Thr Gly Gly Thr Gly Ala Ala Cys
   580 585 590
   Thr Cys Gly Gly Thr Gly Ala Ala Gly Ala Thr Cys Cys Thr Cys Thr
   595 600 605
   Thr Gly Gly Cys Ala Thr Cys Cys Cys Thr Ala Ala Thr Ala Ala Cys
   610 615 620
   Thr Thr Gly Cys Thr Thr Cys Cys Thr Thr Ala Thr Ala Thr Cys Gly
   625 630 635 640
   Cys Gly Cys Ala Ala Gly Thr Thr Gly Cys Thr Gly Thr Ala Gly Gly
   645 650 655
   Ala Ala Gly Ala Thr Thr Gly Gly Ala Thr Cys Ala Thr Thr Thr Gly
   660 665 670
   Ala Ala Thr Gly Thr Ala Thr Thr Thr Gly Gly Cys Gly Ala Cys Gly
   675 680 685
   Ala Thr Thr Ala Thr Cys Cys Cys Ala Cys Ala Thr Cys Thr Gly Ala
   690 695 700
   Cys Gly Gly Thr Ala Cys Thr Cys Cys Ala Ala Thr Thr Cys Gly Thr
   705 710 715 720
   Gly Ala Cys Thr Ala Cys Ala Thr Thr Cys Ala Cys Gly Thr Ala Thr
   725 730 735
   Gly Cys Gly Ala Thr Thr Thr Gly Gly Cys Ala Gly Ala Gly Gly Cys
   740 745 750
   Thr Cys Ala Thr Gly Thr Thr Gly Cys Thr Gly Cys Thr Cys Thr Cys
   755 760 765
   Gly Ala Thr Thr Ala Cys Cys Thr Gly Cys Gly Cys Cys Ala Ala Cys
   770 775 780
   Ala Thr Thr Thr Thr Gly Thr Thr Ala Gly Thr Thr Gly Cys Cys Gly
   785 790 795 800
   Cys Cys Cys Thr Thr Gly Gly Ala Ala Thr Thr Thr Gly Gly Gly Ala
   805 810 815
   Thr Cys Ala Gly Gly Ala Ala Cys Thr Gly Gly Thr Ala Gly Thr Ala
   820 825 830
   Cys Thr Gly Thr Thr Thr Thr Thr Cys Ala Gly Gly Thr Gly Cys Thr
   835 840 845
   Cys Ala Ala Thr Gly Cys Gly Thr Thr Thr Thr Cys Gly Ala Ala Ala
   850 855 860
   Gly Cys Thr Gly Thr Thr Gly Gly Ala Ala Gly Ala Gly Ala Thr Cys
   865 870 875 880
   Thr Thr Cys Cys Thr Thr Ala Thr Ala Ala Gly Gly Thr Cys Ala Cys
   885 890 895
   Cys Cys Cys Thr Ala Gly Ala Ala Gly Ala Gly Cys Ala Gly Gly Gly
   900 905 910
   Gly Ala Cys Gly Thr Thr Gly Thr Thr Ala Ala Cys Cys Thr Ala Ala
   915 920 925
   Cys Cys Gly Cys Cys Ala Ala Cys Cys Cys Cys Ala Cys Thr Cys Gly
   930 935 940
   Cys Gly Cys Thr Ala Ala Cys Gly Ala Gly Gly Ala Gly Thr Thr Ala
   945 950 955 960
   Ala Ala Ala Thr Gly Gly Ala Ala Ala Ala Cys Cys Ala Gly Thr Cys
   965 970 975
   Gly Thr Ala Gly Cys Ala Thr Thr Thr Ala Thr Gly Ala Ala Ala Thr
   980 985 990
   Thr Thr Gly Cys Gly Thr Thr Gly Ala Cys Ala Cys Thr Thr Gly Gly
   995 1000 1005
   Ala Gly Ala Thr Gly Gly Cys Ala Ala Cys Ala Gly Ala Ala Gly
   1010 1015 1020
   Thr Ala Thr Cys Cys Cys Thr Ala Thr Gly Gly Cys Thr Thr Thr
   1025 1030 1035
   Gly Ala Cys Cys Thr Gly Ala Cys Cys Cys Ala Thr Ala Cys Cys
   1040 1045 1050
   Ala Ala Gly Ala Cys Ala Thr Ala Thr Ala Ala Gly Thr Ala Ala
   1055 1060 1065
<210> 15
   <211> 32
   <212> DNA
   <213> Artificial
<220>
   <223> degenerate oligonucleotide primer
<400> 15
   tatgcggccg cggctgatga tatttgctac ga 32
<210> 16
   <211> 33
   <212> DNA
   <213> Artificial
<220>
   <223> degenerate oligonucleotide primer
<400> 16
   cctctcgagt ggacacagga gactcagaaa cag 33
<210> 17
   <211> 30
   <212> DNA
   <213> Artificial
<220>
   <223> degenerate oligonucleotide primer
<400> 17
   cttctcgagg aagtaaagtt ggcgaaactt 30
<210> 18
   <211> 33
   <212> DNA
   <213> Artificial
<220>
   <223> degenerate oligonucleotide primer
<400> 18
   cttagcggcc gcgattgttc gtttgagtag ttt 33
<210> 19
   <211> 30
   <212> DNA
   <213> Artificial
<220>
   <223> degenerate oligonucleotide primer
<400> 19
   cttctcgagg gcattcaaag aagccttggg 30
<210> 20
   <211> 33
   <212> DNA
   <213> Artificial
<220>
   <223> degenerate oligonucleotide primer
<400> 20
   cttagcggcc gctgagtggt catgtgggaa ctt 33
<210> 21
   <211> 30
   <212> DNA
   <213> Artificial
<220>
   <223> degenerate oligonucleotide primer
<400> 21
   cctggatcca acagactaca atgacaggag 30
<210> 22
   <211> 43
   <212> DNA
   <213> Artificial
<220>
   <223> degenerate oligonucleotide primer
<400> 22
   cctgcatgcc tcgagcttgc cggcgtctaa atagccgttg aag 43
<210> 23
   <211> 41
   <212> DNA
   <213> Artificial
<220>
   <223> degenerate oligonucleotide primer
<400> 23
   cctgtcgacg ctgccggcaa gctcgagttt aagcggtgct g 41
<210> 24
   <211> 34
   <212> DNA
   <213> Artificial
<220>
   <223> degenerate oligonucleotide primer
<400> 24
   cctggatcct ttggcaaaaa ccagccctgg tgag 34
<210> 25
   <211> 33
   <212> DNA
   <213> Artificial
<220>
   <223> degenerate oligonucleotide primer
<400> 25
   tccttaatta aagaaagcta gagtaaaata gat 33
<210> 26
   <211> 33
   <212> DNA
   <213> Artificial
<220>
   <223> degenerate oligonucleotide primer
<400> 26
   tccctcgagg atcatgttga tcaactgaga ccg 33
<210> 27
   <211> 45
   <212> DNA
   <213> Artificial
<220>
   <223> degenerate oligonucleotide primer
<400> 27
   ggctcgagcg gccgccacca tggcagcggt tggggctggt ggttc 45
<210> 28
   <211> 43
   <212> DNA
   <213> Artificial
<220>
   <223> degenerate oligonucleotide primer
<400> 28
   ccctcgagtt aattaatcac ttcaccagca ctgactttgg cag 43
<210> 29
   <211> 45
   <212> DNA
   <213> Artificial
<220>
   <223> degenerate oligonucleotide primer
<400> 29
   ggctcgagcg gccgccacca tggcagcggt tggggctggt ggttc 45
<210> 30
   <211> 44
   <212> DNA
   <213> Artificial
<220>
   <223> degenerate oligonucleotide primer
<400> 30
   ccctcgagtt aattaactag gaacccttca ccttggtgag caac 44
<210> 31
   <211> 36
   <212> DNA
   <213> Artificial
<220>
   <223> degenerate oligonucleotide primer
<400> 31
   tagcggccgc atgacagctc agttacaaag tgaaag 36
<210> 32
   <211> 36
   <212> DNA
   <213> Artificial
<220>
   <223> degenerate oligonucleotide primer
<400> 32
   cgttaattaa tcaggaaaat ctgtagacaa tcttgg 36
<210> 33
   <211> 29
   <212> DNA
   <213> Artificial
<220>
   <223> degenerate oligonucleotide primer
<400> 33
   gcggccgcat ggcagagaag gtgctggta 29
<210> 34
   <211> 29
   <212> DNA
   <213> Artificial
<220>
   <223> degenerate oligonucleotide primer
<400> 34
   ttaattaatc aggcttgcgt gccaaagcc 29
<210> 35
   <211> 21
   <212> DNA
   <213> Artificial
<220>
   <223> degenerate oligonucleotide primer
<400> 35
   atgactggtg ttcatgaagg g 21
<210> 36
   <211> 21
   <212> DNA
   <213> Artificial
<220>
   <223> degenerate oligonucleotide primer
<400> 36
   ttacttatat gtcttggtat g 21
<210> 37
   <211> 94
   <212> DNA
   <213> Artificial
<220>
   <223> degenerate oligonucleotide primer
<400> 37
   gcggccgcat gactggtgtt catgaaggga ctgtgttggt tactggcggc gctggttata 60
   taggttctca tacgtgcgtt gttttgttag aaaa 94
<210> 38
   <211> 29
   <212> DNA
   <213> Artificial
<220>
   <223> degenerate oligonucleotide primer
<400> 38
   ttaattaatt acttatatgt cttggtatg 29
<210> 39
   <211> 33
   <212> DNA
   <213> Artificial
<220>
   <223> degenerate oligonucleotide primer
<400> 39
   cttaggcgcg cccagcacct ggccttcttc age 33
<210> 40
   <211> 36
   <212> DNA
   <213> Artificial
<220>
   <223> degenerate oligonucleotide primer
<400> 40
   cttgttaatt aatcagcccc gagggggcca cgacgg 36
<210> 41
   <211> 35
   <212> DNA
   <213> Artificial
<220>
   <223> degenerate oligonucleotide primer
<400> 41
   cttaggcgcg cccgaagtct cagtgcccta tttgg 35
<210> 42
   <211> 36
   <212> DNA
   <213> Artificial
<220>
   <223> degenerate oligonucleotide primer
<400> 42
   cttgttaatt aatcagtgtg aacctcggag ggctgt 36
<210> 43
   <211> 24
   <212> DNA
   <213> Artificial
<220>
   <223> degenerate oligonucleotide primer
<400> 43
   tacgagattc ccaaatatga ttcc 24
<210> 44
   <211> 24
   <212> DNA
   <213> Artificial
<220>
   <223> degenerate oligonucleotide primer
<400> 44
   atagtgtctc catatggctt gttc 24
<210> 45
   <211> 35
   <212> DNA
   <213> Artificial
<220>
   <223> degenerate oligonucleotide primer
<400> 45
   cttggcgcgc catgactggt gttcatgaag ggact 35
<210> 46
   <211> 33
   <212> DNA
   <213> Artificial
<220>
   <223> degenerate oligonucleotide primer
<400> 46
   cctggatccc ttatatgtct tggtatgggt cag 33
<210> 47
   <211> 39
   <212> DNA
   <213> Artificial
<220>
   <223> degenerate oligonucleotide primer
<400> 47
   cttggatccg gtggtggccg cgacctgagc cgcctgccc 39
<210> 48
   <211> 1303
   <212> DNA
   <213> Schizosaccharomyces pombe
<400> 48
   atggtatgaa taactttttt aattaatcaa aagcattctt ttgtatgaaa atactaatta 60
   tgattcatag actggtgttc atgaagggac tgtgttggtt actggcggcg ctggttatat 120
   aggttctcat acggtacgta gagagcttga agatacagaa gaggattagt aatgtacatg 180
   taaatgtttt aagcacgcat cttttgtgaa tatagcttgc tgctctttac ttttatacaa 240
   tttcgtccat attctataaa gctctttttt gagatatttt gctaaccaca atctgcaata 300
   gtgcgttgtt ttgttagaaa aaggatatga tgttgtaatt gtcgataatt tatgcaattc 360
   tcgcgttgaa gccgtgcacc gcattgaaaa actcactggg aaaaaagtca tattccacca 420
   ggtggatttg cttgatgagc cagctttgga caaggtcttc gcaaatcaaa acatatctgc 480
   tgtcattcat tttgctggtc tcaaagcagt tggtgaatct gtacaggttc ctttgagtta 540
   ttacaaaaat aacatttccg gtaccattaa tttaatagag tgcatgaaga agtataatgt 600
   acgtgacttc gtcttttctt catctgctac cgtgtatggc gatcctacta gacctggtgg 660
   taccattcct attccagagt catgccctcg tgaaggtaca agcccatatg gtcgcacaaa 720
   gcttttcatt gaaaatatca ttgaggatga gaccaaggtg aacaaatcgc ttaatgcagc 780
   tttattacgc tattttaatc ccggaggtgc tcatccctct ggtgaactcg gtgaagatcc 840
   tcttggcatc cctaataact tgcttcctta tatcgcgcaa gttgctgtag gaagattgga 900
   tcatttgaat gtatttggcg acgattatcc cacatctgac ggtactccaa ttcgtgacta 960
   cattcacgta tgcgatttgg cagaggctca tgttgctgct ctcgattacc tgcgccaaca 1020
   ttttgttagt tgccgccctt ggaatttggg atcaggaact ggtagtactg tttttcaggt 1080
   gctcaatgcg ttttcgaaag ctgttggaag agatcttcct tataaggtca cccctagaag 1140
   agcaggggac gttgttaacc taaccgccaa ccccactcgc gctaacgagg agttaaaatg 1200
   gaaaaccagt cgtagcattt atgaaatttg cgttgacact tggagatggc aacagaagta 1260
   tccctatggc tttgacctga cccataccaa gacatataag taa 1303
<210> 49
   <211> 355
   <212> PRT
   <213> Homo sapiens
<400> 49
   Gly Arg Asp Leu Ser Arg Leu Pro Gln Leu Val Gly Val Ser Thr Pro
   1 5 10 15
   Leu Gln Gly Gly Ser Asn Ser Ala Ala Ala Ile Gly Gln Ser Ser Gly
   20 25 30
   Glu Leu Arg Thr Gly Gly Ala Arg Pro Pro Pro Pro Leu Gly Ala Ser
   35 40 45
   Ser Gln Pro Arg Pro Gly Gly Asp Ser Ser Pro Val Val Asp Ser Gly
   50 55 60
   Pro Gly Pro Ala Ser Asn Leu Thr Ser Val Pro Val Pro His Thr Thr
   65 70 75 80
   Ala Leu Ser Leu Pro Ala Cys Pro Glu Glu Ser Pro Leu Leu Val Gly
   85 90 95
   Pro Met Leu Ile Glu Phe Asn Met Pro Val Asp Leu Glu Leu Val Ala
   100 105 110
   Lys Gln Asn Pro Asn Val Lys Met Gly Gly Arg Tyr Ala Pro Arg Asp
   115 120 125
   Cys Val Ser Pro His Lys Val Ala Ile Ile Ile Pro Phe Arg Asn Arg
   130 135 140
   Gln Glu His Leu Lys Tyr Trp Leu Tyr Tyr Leu His Pro Val Leu Gln
   145 150 155 160
   Arg Gln Gln Leu Asp Tyr Gly Ile Tyr Val Ile Asn Gln Ala Gly Asp
   165 170 175
   Thr Ile Phe Asn Arg Ala Lys Leu Leu Asn Val Gly Phe Gln Glu Ala
   180 185 190
   Leu Lys Asp Tyr Asp Tyr Thr Cys Phe Val Phe Ser Asp Val Asp Leu
   195 200 205
   Ile Pro Met Asn Asp His Asn Ala Tyr Arg Cys Phe Ser Gln Pro Arg
   210 215 220
   His Ile Ser Val Ala Met Asp Lys Phe Gly Phe Ser Leu Pro Tyr Val
   225 230 235 240
   Gln Tyr Phe Gly Gly Val Ser Ala Leu Ser Lys Gln Gln Phe Leu Thr
   245 250 255
   Ile Asn Gly Phe Pro Asn Asn Tyr Trp Gly Trp Gly Gly Glu Asp Asp
   260 265 270
   Asp Ile Phe Asn Arg Leu Val Phe Arg Gly Met Ser Ile Ser Arg Pro
   275 280 285
   Asn Ala Val Val Gly Arg Cys Arg Met Ile Arg His Ser Arg Asp Lys
   290 295 300
   Lys Asn Glu Pro Asn Pro Gln Arg Phe Asp Arg Ile Ala His Thr Lys
   305 310 315 320
   Glu Thr Met Leu Ser Asp Gly Leu Asn Ser Leu Thr Tyr Gln Val Leu
   325 330 335
   Asp Val Gln Arg Tyr Pro Leu Tyr Thr Gln Ile Thr Val Asp Ile Gly
   340 345 350
   Thr Pro Ser
   355
<210> 50
   <211> 337
   <212> PRT
   <213> Homo sapiens
<400> 50
   Ala Gln His Leu Ala Phe Phe Ser Arg Phe Ser Ala Arg Gly Pro Ala
   1 5 10 15
   His Ala Leu His Pro Ala Ala Ser Ser Ser Ser Ser Ser Ser Asn Cys
   20 25 30
   Ser Arg Pro Asn Ala Thr Ala Ser Ser Ser Gly Leu Pro Glu Val Pro
   35 40 45
   Ser Ala Leu Pro Gly Pro Thr Ala Pro Thr Leu Pro Pro Cys Pro Asp
   50 55 60
   Ser Pro Pro Gly Leu Val Gly Arg Leu Leu Ile Glu Phe Thr Ser Pro
   65 70 75 80
   Met Pro Leu Glu Arg Val Gln Arg Glu Asn Pro Gly Val Leu Met Gly
   85 90 95
   Gly Arg Tyr Thr Pro Pro Asp Cys Thr Pro Ala Gln Thr Val Ala Val
   100 105 110
   Ile Ile Pro Phe Arg His Arg Glu His His Leu Arg Tyr Trp Leu His
   115 120 125
   Tyr Leu His Pro Ile Leu Arg Arg Gln Arg Leu Arg Tyr Gly Val Tyr
   130 135 140
   Val Ile Asn Gln His Gly Glu Asp Thr Phe Asn Arg Ala Lys Leu Leu
   145 150 155 160
   Asn Val Gly Phe Leu Glu Ala Leu Lys Glu Asp Ala Ala Tyr Asp Cys
   165 170 175
   Phe Ile Phe Ser Asp Val Asp Leu Val Pro Met Asp Asp Arg Asn Leu
   180 185 190
   Tyr Arg Cys Gly Asp Gln Pro Arg His Phe Ala Ile Ala Met Asp Lys
   195 200 205
   Phe Gly Phe Arg Leu Pro Tyr Ala Gly Tyr Phe Gly Gly Val Ser Gly
   210 215 220
   Leu Ser Lys Ala Gln Phe Leu Arg Ile Asn Gly Phe Pro Asn Glu Tyr
   225 230 235 240
   Trp Gly Trp Gly Gly Glu Asp Asp Asp Ile Phe Asn Arg Ile Ser Leu
   245 250 255
   Thr Gly Met Lys Ile Ser Arg Pro Asp Ile Arg Ile Gly Arg Tyr Arg
   260 265 270
   Met Ile Lys His Asp Arg Asp Lys His Asn Glu Pro Asn Pro Gln Arg
   275 280 285
   Phe Thr Lys Ile Gln Asn Thr Lys Leu Thr Met Lys Arg Asp Gly Ile
   290 295 300
   Gly Ser Val Arg Tyr Gln Val Leu Glu Val Ser Arg Gln Pro Leu Phe
   305 310 315 320
   Thr Asn Ile Thr Val Asp Ile Gly Arg Pro Pro Ser Trp Pro Pro Arg
   325 330 335
   Gly
<210> 51
   <211> 362
   <212> PRT
   <213> Homo sapiens
<400> 51
   Arg Ser Leu Ser Ala Leu Phe Gly Arg Asp Gln Gly Pro Thr Phe Asp
   1 5 10 15
   Tyr Ser His Pro Arg Asp Val Tyr Ser Asn Leu Ser His Leu Pro Gly
   20 25 30
   Arg Pro Gly Gly Pro Pro Ala Pro Gln Gly Leu Pro Tyr Cys Pro Glu
   35 40 45
   Arg Ser Pro Leu Leu Val Gly Pro Val Ser Val Ser Phe Ser Pro Val
   50 55 60
   Pro Ser Leu Ala Glu Ile Val Glu Arg Asn Pro Arg Val Glu Pro Gly
   65 70 75 80
   Ala Arg Tyr Arg Pro Ala Gly Cys Glu Pro Arg Ser Arg Thr Ala Ile
   85 90 95
   Ile Val Pro His Arg Ala Arg Glu His His Leu Arg Leu Leu Leu Tyr
   100 105 110
   His Leu His Pro Phe Leu Gln Arg Gln Gln Leu Ala Tyr Gly Ile Tyr
   115 120 125
   Val Ile His Gln Ala Gly Asn Gly Thr Phe Asn Arg Ala Lys Leu Leu
   130 135 140
   Asn Val Gly Val Arg Glu Ala Leu Arg Asp Glu Glu Trp Asp Cys Leu
   145 150 155 160
   Phe Leu His Asp Val Asp Leu Leu Pro Glu Asn Asp His Asn Leu Tyr
   165 170 175
   Val Cys Asp Pro Arg Gly Pro Arg His Val Ala Val Ala Met Asn Ser
   180 185 190
   Phe Gly Tyr Ser Leu Pro Tyr Pro Gln Tyr Phe Gly Gly Val Ser Ala
   195 200 205
   Leu Thr Pro Asp Gln Tyr Leu Lys Met Asn Gly Phe Pro Asn Glu Tyr
   210 215 220
   Trp Gly Trp Gly Gly Glu Asp Asp Asp Ile Ala Thr Arg Val Arg Leu
   225 230 235 240
   Ala Gly Met Lys Ile Ser Arg Pro Pro Thr Ser Val Gly His Tyr Lys
   245 250 255
   Met Val Lys His Arg Gly Asp Lys Gly Asn Glu Glu Asn Pro His Arg
   260 265 270
   Phe Asp Leu Leu Val Arg Thr Gln Asn Ser Trp Thr Gln Asp Gly Met
   275 280 285
   Asn Ser Leu Thr Tyr Gln Leu Leu Ala Arg Glu Leu Gly Pro Leu Tyr
   290 295 300
   Thr Asn Ile Thr Ala Asp Ile Gly Thr Asp Pro Arg Gly Pro Arg Ala
   305 310 315 320
   Pro Ser Gly Pro Arg Tyr Pro Pro Gly Ser Ser Gln Ala Phe Arg Gln
   325 330 335
   Glu Met Leu Gln Arg Arg Pro Pro Ala Arg Pro Gly Pro Leu Ser Thr
   340 345 350
   Ala Asn His Thr Ala Leu Arg Gly Ser His
   355 360
<210> 52
   <211> 348
   <212> PRT
   <213> Homo sapiens
<400> 52
   Met Ala Glu Lys Val Leu Val Thr Gly Gly Ala Gly Tyr Ile Gly Ser
   1 5 10 15
   His Thr Val Leu Glu Leu Leu Glu Ala Gly Tyr Leu Pro Val Val Ile
   20 25 30
   Asp Asn Phe His Asn Ala Phe Arg Gly Gly Gly Ser Leu Pro Glu Ser
   35 40 45
   Leu Arg Arg Val Gln Glu Leu Thr Gly Arg Ser Val Glu Phe Glu Glu
   50 55 60
   Met Asp Ile Leu Asp Gln Gly Ala Leu Gln Arg Leu Phe Lys Lys Tyr
   65 70 75 80
   Ser Phe Met Ala Val Ile His Phe Ala Gly Leu Lys Ala Val Gly Glu
   85 90 95
   Ser Val Gln Lys Pro Leu Asp Tyr Tyr Arg Val Asn Leu Thr Gly Thr
   100 105 110
   Ile Gln Leu Leu Glu Ile Met Lys Ala His Gly Val Lys Asn Leu Val
   115 120 125
   Phe Ser Ser Ser Ala Thr Val Tyr Gly Asn Pro Gln Tyr Leu Pro Leu
   130 135 140
   Asp Glu Ala His Pro Thr Gly Gly Cys Thr Asn Pro Tyr Gly Lys Ser
   145 150 155 160
   Lys Phe Phe Ile Glu Glu Met Ile Arg Asp Leu Cys Gln Ala Asp Lys
   165 170 175
   Thr Trp Asn Val Val Leu Leu Arg Tyr Phe Asn Pro Thr Gly Ala His
   180 185 190
   Ala Ser Gly Cys Ile Gly Glu Asp Pro Gln Gly Ile Pro Asn Asn Leu
   195 200 205
   Met Pro Tyr Val Ser Gln Val Ala Ile Gly Arg Arg Glu Ala Leu Asn
   210 215 220
   Val Phe Gly Asn Asp Tyr Asp Thr Glu Asp Gly Thr Gly Val Arg Asp
   225 230 235 240
   Tyr Ile His Val Val Asp Leu Ala Lys Gly His Ile Ala Ala Leu Arg
   245 250 255
   Lys Leu Lys Glu Gln Cys Gly Cys Arg Ile Tyr Asn Leu Gly Thr Gly
   260 265 270
   Thr Gly Tyr Ser Val Leu Gln Met Val Gln Ala Met Glu Lys Ala Ser
   275 280 285
   Gly Lys Lys Ile Pro Tyr Lys Val Val Ala Arg Arg Glu Gly Asp Val
   290 295 300
   Ala Ala Cys Tyr Ala Asn Pro Ser Leu Ala Gln Glu Glu Leu Gly Trp
   305 310 315 320
   Thr Ala Ala Leu Gly Leu Asp Arg Met Cys Glu Asp Leu Trp Arg Trp
   325 330 335
   Gln Lys Gln Asn Pro Ser Gly Phe Gly Thr Gln Ala
   340 345
<210> 53
   <211> 699
   <212> PRT
   <213> Saccharomyces cerevisiae
<400> 53
   Met Thr Ala Gln Leu Gln Ser Glu Ser Thr Ser Lys Ile Val Leu Val
   1 5 10 15
   Thr Gly Gly Ala Gly Tyr Ile Gly Ser His Thr Val Val Glu Leu Ile
   20 25 30
   Glu Asn Gly Tyr Asp Cys Val Val Ala Asp Asn Leu Ser Asn Ser Thr
   35 40 45
   Tyr Asp Ser Val Ala Arg Leu Glu Val Leu Thr Lys His His Ile Pro
   50 55 60
   Phe Tyr Glu Val Asp Leu Cys Asp Arg Lys Gly Leu Glu Lys Val Phe
   65 70 75 80
   Lys Glu Tyr Lys Ile Asp Ser Val Ile His Phe Ala Gly Leu Lys Ala
   85 90 95
   Val Gly Glu Ser Thr Gln Ile Pro Leu Arg Tyr Tyr His Asn Asn Ile
   100 105 110
   Leu Gly Thr Val Val Leu Leu Glu Leu Met Gln Gln Tyr Asn Val Ser
   115 120 125
   Lys Phe Val Phe Ser Ser Ser Ala Thr Val Tyr Gly Asp Ala Thr Arg
   130 135 140
   Phe Pro Asn Met Ile Pro Ile Pro Glu Glu Cys Pro Leu Gly Pro Thr
   145 150 155 160
   Asn Pro Tyr Gly His Thr Lys Tyr Ala Ile Glu Asn Ile Leu Asn Asp
   165 170 175
   Leu Tyr Asn Ser Asp Lys Lys Ser Trp Lys Phe Ala Ile Leu Arg Tyr
   180 185 190
   Phe Asn Pro Ile Gly Ala His Pro Ser Gly Leu Ile Gly Glu Asp Pro
   195 200 205
   Leu Gly Ile Pro Asn Asn Leu Leu Pro Tyr Met Ala Gln Val Ala Val
   210 215 220
   Gly Arg Arg Glu Lys Leu Tyr Ile Phe Gly Asp Asp Tyr Asp Ser Arg
   225 230 235 240
   Asp Gly Thr Pro Ile Arg Asp Tyr Ile His Val Val Asp Leu Ala Lys
   245 250 255
   Gly His Ile Ala Ala Leu Gln Tyr Leu Glu Ala Tyr Asn Glu Asn Glu
   260 265 270
   Gly Leu Cys Arg Glu Trp Asn Leu Gly Ser Gly Lys Gly Ser Thr Val
   275 280 285
   Phe Glu Val Tyr His Ala Phe Cys Lys Ala Ser Gly Ile Asp Leu Pro
   290 295 300
   Tyr Lys Val Thr Gly Arg Arg Ala Gly Asp Val Leu Asn Leu Thr Ala
   305 310 315 320
   Lys Pro Asp Arg Ala Lys Arg Glu Leu Lys Trp Gln Thr Glu Leu Gln
   325 330 335
   Val Glu Asp Ser Cys Lys Asp Leu Trp Lys Trp Thr Thr Glu Asn Pro
   340 345 350
   Phe Gly Tyr Gln Leu Arg Gly Val Glu Ala Arg Phe Ser Ala Glu Asp
   355 360 365
   Met Arg Tyr Asp Ala Arg Phe Val Thr Ile Gly Ala Gly Thr Arg Phe
   370 375 380
   Gln Ala Thr Phe Ala Asn Leu Gly Ala Ser Ile Val Asp Leu Lys Val
   385 390 395 400
   Asn Gly Gln Ser Val Val Leu Gly Tyr Glu Asn Glu Glu Gly Tyr Leu
   405 410 415
   Asn Pro Asp Ser Ala Tyr Ile Gly Ala Thr Ile Gly Arg Tyr Ala Asn
   420 425 430
   Arg Ile Ser Lys Gly Lys Phe Ser Leu Cys Asn Lys Asp Tyr Gln Leu
   435 440 445
   Thr Val Asn Asn Gly Val Asn Ala Asn His Ser Ser Ile Gly Ser Phe
   450 455 460
   His Arg Lys Arg Phe Leu Gly Pro Ile Ile Gln Asn Pro Ser Lys Asp
   465 470 475 480
   Val Phe Thr Ala Glu Tyr Met Leu Ile Asp Asn Glu Lys Asp Thr Glu
   485 490 495
   Phe Pro Gly Asp Leu Leu Val Thr Ile Gln Tyr Thr Val Asn Val Ala
   500 505 510
   Gln Lys Ser Leu Glu Met Val Tyr Lys Gly Lys Leu Thr Ala Gly Glu
   515 520 525
   Ala Thr Pro Ile Asn Leu Thr Asn His Ser Tyr Phe Asn Leu Asn Lys
   530 535 540
   Pro Tyr Gly Asp Thr Ile Glu Gly Thr Glu Ile Met Val Arg Ser Lys
   545 550 555 560
   Lys Ser Val Asp Val Asp Lys Asn Met Ile Pro Thr Gly Asn Ile Val
   565 570 575
   Asp Arg Glu Ile Ala Thr Phe Asn Ser Thr Lys Pro Thr Val Leu Gly
   580 585 590
   Pro Lys Asn Pro Gln Phe Asp Cys Cys Phe Val Val Asp Glu Asn Ala
   595 600 605
   Lys Pro Ser Gln Ile Asn Thr Leu Asn Asn Glu Leu Thr Leu Ile Val
   610 615 620
   Lys Ala Phe His Pro Asp Ser Asn Ile Thr Leu Glu Val Leu Ser Thr
   625 630 635 640
   Glu Pro Thr Tyr Gln Phe Tyr Thr Gly Asp Phe Leu Ser Ala Gly Tyr
   645 650 655
   Glu Ala Arg Gln Gly Phe Ala Ile Glu Pro Gly Arg Tyr Ile Asp Ala
   660 665 670
   Ile Asn Gln Glu Asn Trp Lys Asp Cys Val Thr Leu Lys Asn Gly Glu
   675 680 685
   Thr Tyr Gly Ser Lys Ile Val Tyr Arg Phe Ser
   690 695
<210> 54
   <211> 394
   <212> PRT
   <213> Homo sapiens
<400> 54
   Met Ala Ala Val Gly Ala Gly Gly Ser Thr Ala Ala Pro Gly Pro Gly
   1 5 10 15
   Ala Val Ser Ala Gly Ala Leu Glu Pro Gly Thr Ala Ser Ala Ala His
   20 25 30
   Arg Arg Leu Lys Tyr Ile Ser Leu Ala Val Leu Val Val Gln Asn Ala
   35 40 45
   Ser Leu Ile Leu Ser Ile Arg Tyr Ala Arg Thr Leu Pro Gly Asp Arg
   50 55 60
   Phe Phe Ala Thr Thr Ala Val Val Met Ala Glu Val Leu Lys Gly Leu
   65 70 75 80
   Thr Cys Leu Leu Leu Leu Phe Ala Gln Lys Arg Gly Asn Val Lys His
   85 90 95
   Leu Val Leu Phe Leu His Glu Ala Val Leu Val Gln Tyr Val Asp Thr
   100 105 110
   Leu Lys Leu Ala Val Pro Ser Leu Ile Tyr Thr Leu Gln Asn Asn Leu
   115 120 125
   Gln Tyr Val Ala Ile Ser Asn Leu Pro Ala Ala Thr Phe Gln Val Thr
   130 135 140
   Tyr Gln Leu Lys Ile Leu Thr Thr Ala Leu Phe Ser Val Leu Met Leu
   145 150 155 160
   Asn Arg Ser Leu Ser Arg Leu Gln Trp Ala Ser Leu Leu Leu Leu Phe
   165 170 175
   Thr Gly Val Ala Ile Val Gln Ala Gln Gln Ala Gly Gly Gly Gly Pro
   180 185 190
   Arg Pro Leu Asp Gln Asn Pro Gly Ala Gly Leu Ala Ala Val Val Ala
   195 200 205
   Ser Cys Leu Ser Ser Gly Phe Ala Gly Val Tyr Phe Glu Lys Ile Leu
   210 215 220
   Lys Gly Ser Ser Gly Ser Val Trp Leu Arg Asn Leu Gln Leu Gly Leu
   225 230 235 240
   Phe Gly Thr Ala Leu Gly Leu Val Gly Leu Trp Trp Ala Glu Gly Thr
   245 250 255
   Ala Val Ala Thr Arg Gly Phe Phe Phe Gly Tyr Thr Pro Ala Val Trp
   260 265 270
   Gly Val Val Leu Asn Gln Ala Phe Gly Gly Leu Leu Val Ala Val Val
   275 280 285
   Val Lys Tyr Ala Asp Asn Ile Leu Lys Gly Phe Ala Thr Ser Leu Ser
   290 295 300
   Ile Val Leu Ser Thr Val Ala Ser Ile Arg Leu Phe Gly Phe His Val
   305 310 315 320
   Asp Pro Leu Phe Ala Leu Gly Ala Gly Leu Val Ile Gly Ala Val Tyr
   325 330 335
   Leu Tyr Ser Leu Pro Arg Gly Ala Ala Lys Ala Ile Ala Ser Ala Ser
   340 345 350
   Ala Ser Ala Ser Gly Pro Cys Val His Gln Gln Pro Pro Gly Gln Pro
   355 360 365
   Pro Pro Pro Gln Leu Ser Ser His Arg Gly Asp Leu Ile Thr Glu Pro
   370 375 380
   Phe Leu Pro Lys Ser Val Leu Val Lys Glx
   385 390
<210> 55
   <211> 397
   <212> PRT
   <213> Homo sapiens
<400> 55
   Met Ala Ala Val Gly Ala Gly Gly Ser Thr Ala Ala Pro Gly Pro Gly
   1 5 10 15
   Ala Val Ser Ala Gly Ala Leu Glu Pro Gly Thr Ala Ser Ala Ala His
   20 25 30
   Arg Arg Leu Lys Tyr Ile Ser Leu Ala Val Leu Val Val Gln Asn Ala
   35 40 45
   Ser Leu Ile Leu Ser Ile Arg Tyr Ala Arg Thr Leu Pro Gly Asp Arg
   50 55 60
   Phe Phe Ala Thr Thr Ala Val Val Met Ala Glu Val Leu Lys Gly Leu
   65 70 75 80
   Thr Cys Leu Leu Leu Leu Phe Ala Gln Lys Arg Gly Asn Val Lys His
   85 90 95
   Leu Val Leu Phe Leu His Glu Ala Val Leu Val Gln Tyr Val Asp Thr
   100 105 110
   Leu Lys Leu Ala Val Pro Ser Leu Ile Tyr Thr Leu Gln Asn Asn Leu
   115 120 125
   Gln Tyr Val Ala Ile Ser Asn Leu Pro Ala Ala Thr Phe Gln Val Thr
   130 135 140
   Tyr Gln Leu Lys Ile Leu Thr Thr Ala Leu Phe Ser Val Leu Met Leu
   145 150 155 160
   Asn Arg Ser Leu Ser Arg Leu Gln Trp Ala Ser Leu Leu Leu Leu Phe
   165 170 175
   Thr Gly Val Ala Ile Val Gln Ala Gln Gln Ala Gly Gly Gly Gly Pro
   180 185 190
   Arg Pro Leu Asp Gln Asn Pro Gly Ala Gly Leu Ala Ala Val Val Ala
   195 200 205
   Ser Cys Leu Ser Ser Gly Phe Ala Gly Val Tyr Phe Glu Lys Ile Leu
   210 215 220
   Lys Gly Ser Ser Gly Ser Val Trp Leu Arg Asn Leu Gln Leu Gly Leu
   225 230 235 240
   Phe Gly Thr Ala Leu Gly Leu Val Gly Leu Trp Trp Ala Glu Gly Thr
   245 250 255
   Ala Val Ala Thr Arg Gly Phe Phe Phe Gly Tyr Thr Pro Ala Val Trp
   260 265 270
   Gly Val Val Leu Asn Gln Ala Phe Gly Gly Leu Leu Val Ala Val Val
   275 280 285
   Val Lys Tyr Ala Asp Asn Ile Leu Lys Gly Phe Ala Thr Ser Leu Ser
   290 295 300
   Ile Val Leu Ser Thr Val Ala Ser Ile Arg Leu Phe Gly Phe His Val
   305 310 315 320
   Asp Pro Leu Phe Ala Leu Gly Ala Gly Leu Val Ile Gly Ala Val Tyr
   325 330 335
   Leu Tyr Ser Leu Pro Arg Gly Ala Ala Lys Ala Ile Ala Ser Ala Ser
   340 345 350
   Ala Ser Ala Ser Gly Pro Cys Val His Gln Gln Pro Pro Gly Gln Pro
   355 360 365
   Pro Pro Pro Gln Leu Ser Ser His Arg Gly Asp Leu Ile Thr Glu Pro
   370 375 380
   Phe Leu Pro Lys Leu Leu Thr Lys Val Lys Gly Ser Glx
   385 390 395
<210> 56
   <211> 357
   <212> PRT
   <213> Drosophila melanogaster
<400> 56
   Met Asn Ser Ile His Met Asn Ala Asn Thr Leu Lys Tyr Ile Ser Leu
   1 5 10 15
   Leu Thr Leu Thr Leu Gln Asn Ala Ile Leu Gly Leu Ser Met Arg Tyr
   20 25 30
   Ala Arg Thr Arg Pro Gly Asp Ile Phe Leu Ser Ser Thr Ala Val Leu
   35 40 45
   Met Ala Glu Phe Ala Lys Leu Ile Thr Cys Leu Phe Leu Val Phe Asn
   50 55 60
   Glu Glu Gly Lys Asp Ala Gln Lys Phe Val Arg Ser Leu His Lys Thr
   65 70 75 80
   Ile Ile Ala Asn Pro Met Asp Thr Leu Lys Val Cys Val Pro Ser Leu
   85 90 95
   Val Tyr Ile Val Gln Asn Asn Leu Leu Tyr Val Ser Ala Ser His Leu
   100 105 110
   Asp Ala Ala Thr Tyr Gln Val Thr Tyr Gln Leu Lys Ile Leu Thr Thr
   115 120 125
   Ala Met Phe Ala Val Val Ile Leu Arg Arg Lys Leu Leu Asn Thr Gln
   130 135 140
   Trp Gly Ala Leu Leu Leu Leu Val Met Gly Ile Val Leu Val Gln Leu
   145 150 155 160
   Ala Gln Thr Glu Gly Pro Thr Ser Gly Ser Ala Gly Gly Ala Ala Ala
   165 170 175
   Ala Ala Thr Ala Ala Ser Ser Gly Gly Ala Pro Glu Gln Asn Arg Met
   180 185 190
   Leu Gly Leu Trp Ala Ala Leu Gly Ala Cys Phe Leu Ser Gly Phe Ala
   195 200 205
   Gly Ile Tyr Phe Glu Lys Ile Leu Lys Gly Ala Glu Ile Ser Val Trp
   210 215 220
   Met Arg Asn Val Gln Leu Ser Leu Leu Ser Ile Pro Phe Gly Leu Leu
   225 230 235 240
   Thr Cys Phe Val Asn Asp Gly Ser Arg Ile Phe Asp Gln Gly Phe Phe
   245 250 255
   Lys Gly Tyr Asp Leu Phe Val Trp Tyr Leu Val Leu Leu Gln Ala Gly
   260 265 270
   Gly Gly Leu Ile Val Ala Val Val Val Lys Tyr Ala Asp Asn Ile Leu
   275 280 285
   Lys Gly Phe Ala Thr Ser Leu Ala Ile Ile Ile Ser Cys Val Ala Ser
   290 295 300
   Ile Tyr Ile Phe Asp Phe Asn Leu Thr Leu Gln Phe Ser Phe Gly Ala
   305 310 315 320
   Gly Leu Val Ile Ala Ser Ile Phe Leu Tyr Gly Tyr Asp Pro Ala Arg
   325 330 335
   Ser Ala Pro Lys Pro Thr Met His Gly Pro Gly Gly Asp Glu Glu Lys
   340 345 350
   Leu Leu Pro Arg Val
   355
<210> 57
   <211> 353
   <212> PRT
   <213> Schizosaccharomyces pombe
<400> 57
   Met Ala Val Lys Gly Asp Asp Val Lys Trp Lys Gly Ile Pro Met Lys
   1 5 10 15
   Tyr Ile Ala Leu Val Leu Leu Thr Val Gln Asn Ser Ala Leu Ile Leu
   20 25 30
   Thr Leu Asn Tyr Ser Arg Ile Met Pro Gly Tyr Asp Asp Lys Arg Tyr
   35 40 45
   Phe Thr Ser Thr Ala Val Leu Leu Asn Glu Leu Ile Lys Leu Val Val
   50 55 60
   Cys Phe Ser Val Gly Tyr His Gln Phe Arg Lys Asn Val Gly Lys Glu
   65 70 75 80
   Ala Lys Leu Arg Ala Phe Leu Pro Gln Ile Phe Gly Gly Asp Ser Trp
   85 90 95
   Lys Leu Ala Ile Pro Ala Phe Leu Tyr Thr Cys Gln Asn Asn Leu Gln
   100 105 110
   Tyr Val Ala Ala Gly Asn Leu Thr Ala Ala Ser Phe Gln Val Thr Tyr
   115 120 125
   Gln Leu Lys Ile Leu Thr Thr Ala Ile Phe Ser Ile Leu Leu Leu His
   130 135 140
   Arg Arg Leu Gly Pro Met Lys Trp Phe Ser Leu Phe Leu Leu Thr Gly
   145 150 155 160
   Gly Ile Ala Ile Val Gln Leu Gln Asn Leu Asn Ser Asp Asp Gln Met
   165 170 175
   Ser Ala Gly Pro Met Asn Pro Val Thr Gly Phe Ser Ala Val Leu Val
   180 185 190
   Ala Cys Leu Ile Ser Gly Leu Ala Gly Val Tyr Phe Glu Lys Val Leu
   195 200 205
   Lys Asp Thr Asn Pro Ser Leu Trp Val Arg Asn Val Gln Leu Ser Phe
   210 215 220
   Phe Ser Leu Phe Pro Cys Leu Phe Thr Ile Leu Met Lys Asp Tyr His
   225 230 235 240
   Asn Ile Ala Glu Asn Gly Phe Phe Phe Gly Tyr Asn Ser Ile Val Trp
   245 250 255
   Leu Ala Ile Leu Leu Gln Ala Gly Gly Gly Ile Ile Val Ala Leu Cys
   260 265 270
   Val Ala Phe Ala Asp Asn Ile Met Lys Asn Phe Ser Thr Ser Ile Ser
   275 280 285
   Ile Ile Ile Ser Ser Leu Ala Ser Val Tyr Leu Met Asp Phe Lys Ile
   290 295 300
   Ser Leu Thr Phe Leu Ile Gly Val Met Leu Val Ile Ala Ala Thr Phe
   305 310 315 320
   Leu Tyr Thr Lys Pro Glu Ser Lys Pro Ser Pro Ser Arg Gly Thr Tyr
   325 330 335
   Ile Pro Met Thr Thr Gln Asp Ala Ala Ala Lys Asp Val Asp His Lys
   340 345 350
   His

## Description

### FIELD OF THE INVENTION

The present invention relates to the field of protein glycosylation engineering in Pichia pastoris, specifically the production of glycoproteins having terminal galactose residues. The present invention further relates to novel host cells comprising genes encoding enzymes involved in galactosyltransfer on glycans and production of glycoproteins that are particularly useful as therapeutic agents.

### BACKGROUND OF THE INVENTION

Yeast and filamentous fungi have both been successfully used for the production of recombinant proteins, both intracellular and secreted (Cereghino, J. L. and J. M. 20Cregg 2000 FEMS Microbiology Reviews 24(1): 45-66; Harkki, A., et al. 1989 Bio Technology 7(6): 596; Berka, R. M., et al. 1992 AbstrPapers Amer. Chem.Soc.203: 121-BIOT; Svetina, M., et al. 2000 J. Biotechnol. 76(2-3): 245-251). Various yeasts, such as K. lactis, Pichia pastors, Pichia methanolica, and Hansenula polynorpha, have played particularly important roles as eukaryotic expression systems because they are able to grow to high cell densities and secrete large quantities of recombinant protein. Likewise, filamentous fungi, such as *Aspergillus niger, Fusarium sp, Neurospora crassa* and others, have been used to efficiently produce glycoproteins in industrial scale. However, glycoproteins expressed in any of these eukaryotic microorganisms differ substantially in N-glycan structure from those in animals. This has prevented the use of yeast or filamentous fungi as hosts for the production of glycosylated therapeutic proteins.

Currently, expression systems such as yeast, filamentous fungi, plants, algae and insect cell lines (lower eukaryotes) are being investigated for the production of therapeutic proteins, which are safer, faster and yield higher product titers than mammalian systems. These systems share a common secretory pathway in N-linked oligosaccharide synthesis. Recently, it was shown that the secretory pathway of *P. pastoris* can be genetically re-engineered to perform sequential glycosylation reactions that mimic early processing of N-glycans in humans and other higher mammals (Choi et al., Proc Natl Acad Sci U S. A. 2003 Apr 29;100(9):5022-7. In addition, production of human glycoproteins with complex N-glycans lacking galactose through re engineering the secretory pathway in yeast *P. pastoris* has been shown (Hamilton et al., Science. 2003 Aug 29;301(5637):1244-6). In mammalian cells, further maturation involves galactose transfer. Consequently, the maturation of complex glycosylation pathways from yeast and lower eukaryotes requires the functional expression of β1,4-galactosyltransferase.

Recombinant expression ofUDP-Gal: βGlcNAc β1,4-galactosyltransferase (β1,4GalT) has been demonstrated in mammalian cells, insect cells (e.g., Sf-9) and yeast cells. A cDNA encoding a soluble form of the human β1,4-galactosyltransferase I (EC 2.4.1.22) (lacking the endogenous Type II membrane domain) has also been expressed in the methylotrophic yeast *P. pastoris.* Malissard et al. Biochem Biophys Re s Commun. 2000 Jan 7;267(1) :169-73. Additionally, gene fusions encoding ScMntlp fused to the catalytic domain of a human β1,4-galactosyltransferase (Gal-Tf) have been expressed showing some activity of the enzyme in the yeast Golgi albeit at very low conversion efficiency. Schwientek et al., J Biol Chem. 1996 Feb 16;271(7):3398-405. Thus, targeting a β1,4-galactosyltranferase (β1,4GalT) to the secretory pathway of a host that produces glycans containing terminal GlcNAc is expected to result in some galactose transfer. However the formation of complex glycans in higher eukaryotes involves the action of mannosidase II which in mammalian cells has been found to act in competition with GalTI (Fukuta et al., Arch Biochem Biophys. 2001 Aug 1; 392(1):79-86). The premature action of GalT is thus expected to prevent the formation of complex galactosylated glycoproteins in the secretory pathway and yield mostly hybrid glycans.

The N-glycans of mammalian glycoproteins typically include galactose, fucose, and terminal sialic acid. These sugars are not usually found on glycoproteins produced in; yeast and filamentous fungi. In humans, nucleotide sugar precursors (e.g. UDP-N acetylglucosamine, UDP-N-acetylgalactosamine, CMP-N-acetylneuraminic acid, UDP galactose, GDP-fucose, etc.) are synthesized in the cytosol and transported into the Golgi, where they are incorporated into N-glycans by glycosyltransferases (Sommers and Hirschberg, 1981 J. Cell Biol. 91(2) : A406-A406; Sommers and Hirschberg 1982 J. Biol. Chem. 257(18): 811-817; Perez and Hirschberg 1987 Methods in Enzymology 138: 709-715).

Glycosylation engineering in heterologous protein expression systems may involve expression of various enzymes that are involved in the synthesis of nucleotide sugar precursors. The enzyme UDP-galactose 4-epimerase converts the sugar nucleotide UDP-glucose to UDP-galactose via an epimerization of C4. The enzyme has been found in organisms that are able to use galactose as its sole carbon source. Recently, the bifunctional enzyme, GallOp, has been purified in *Saccharomyces cerevisiae* having both a UDP-glucose 4-epimerase and aldose 1-epimerase activity. Majumdar et al., Eur J Biochem. 2004 Feb;271(4):753-759.

The UDP-galactose transporters (UGT) transport UDP-galactose from the cytosol to the lumen of the Golgi. Two heterologous genes, *gmal2(*+*)* encoding alpha 1,2-galactosyltransferase (alpha 1,2 G. alT) from *Sclzizasaccharonzyces ponzbe* and *(hUGT2)* encoding human UDP-galactose transporter, have been functionally expressed in *S. cerevisiae* to examine the intracellular conditions required for galactosylation. Correlation between protein galactosylation and UDP-galactose transport activity indicated that an exogenous supply of UDP-Gal transporter, played a key role for I efficient galactosylation in *S. cerevisiae* (Kainuma, 1999 Glycobiology 9(2): 133-141). Likewise, a UDP-galactose transporter from *S. pombe* was cloned(Aoki, 1999 J.Biochem. 126(5): 940-950; Segawa, 1999 Febs Letters 451 (3): 295-298).

Glycosyltransfer reactions typically yield a side product which is a nucleoside diphosphate or monophosphate. While monophosphates can be directly exported in exchange for nucleoside diphosphate sugars by an antiport mechanism, diphosphonucleosides (e.g. GDP) have to be cleaved by phosphatases (e.g. GDPase) to yield nucleoside monophosphates and inorganic phosphate prior to being exported. This reaction is important for efficient glycosylation; for example, GDPase from *S. cerevisiae* has been found to be necessary for mannosylation. However that GDPase has 90% reduced activity toward UDP (Berninsone et al., 1994 J. Biol. Chem. 269(1):207-211). Lower eukaryotes typically lack UDP-specific diphosphatase activity in the Golgi since they do not utilize UDP-sugar precursors for Golgi-based glycoprotein synthesis. *S. pombe,* a yeast found to add galactose residues to cell wall polysaccharides (from UDP-galactose) has been found to have specific UDPase activity, indicating the potential requirement for such an enzyme (Berninsone et al., 1994).

UDP is known to be a potent inhibitor of glycosyltransferases and the removal of this glycosylation side product may be important to prevent glycosyltransferase inhibition in the lumen of the Golgi (Khatara et al., 1974). See Berninsone, P., et al. 1995. J. Biol. Chem. 270(24): 14564-14567; Beaudet, L., et al. 1998 Abc Transporters: Biochemical, Cellular, and MolecularAspects. 292: 397-413. What is needed, therefore, is a method to catalyze the transfer of galactose residues from a sufficient pool of UDP-galactose onto preferred acceptor substrates for use as therapeutic glycoproteins.

### Disclosure of Invention

### SUMMARY OF THE INVENTION

The present invention provides a recombinant Pichia pastoris host cell producing human-like glycoproteins characterized as having a terminal β-galactose residue and essentially lacking fucose and sialic acid residues on the glycoprotein; wherein the host cell expresses: nucleic acid molecule encoding β1,4-galactosyltransferase activity and nucleic acid molecule encoding UDP-galactose C4 epimerase activity.

The present invention discloses a recombinant Pichia pastoris host cell producing human-like glycoproteins, the host cell capable of transferring β-galactose residue onto an N-linked oligosaccharide branch of a glycoprotein comprising a terminal GlcNAc residue, the N-linked oligosaccharide branch selected from the group consisting of GlcNAcβ1,2-Manα,3; GlcNAcβ1,4-Manα1,3; GlcNAcβ1,2-Manal,6; GcNAcβ1,4-Manal,6; and GlcNAcβ1,6-Manα1,6 on a trimannose core. The present invention illustrates a recombinant Pichia pastoris host cell that produces glycoproteins that are acceptor substrates for sialic acid transfer. In another aspect of the invention, herein is provided a composition comprising a human-like glycoprotein characterized as having a terminal β-galactose residue and essentially lacking fucose and sialic acid residues on the glycoprotein. The glycoprotein may comprise N-linked oligosaccharides selected from the group consisting of: GalGlcNAcMan₃GlcNAc₂, GalGlcNAc₂, Man₃GlcNAc₂, Gal₂GlcNAc₂Man₃GlcNAc₂, GalGlcNAc₃ Man₃GlcNAc₂, Gal₂GlcNAc₃Man₃GlcNAc₂, Gal₃GlcNAc₃Man₃GlcNAc₂, GalGlcNAc₄Man₃GlcNAc₂, Gal₂GlcNAc₄ Man₃GlcNAc₂, Gal₃GlcNAc₄Man₃GlcNAc₂, Gal₄GlcNAc₄Man₃GlcNAc₂GalGlcNAcMan₅GlcNAc₂, GalGlcNAc₂Man₅ GlcNAc₂, Gal₂GlcNAc₂Man₅GlcNAc₂, GalGlcNAc₃Man₅GlcNAc₂, Gal₂GlcNAc₃Man₅GlcNAc₂ and Gal₃GlcNAc₃ Man₅GlcNAc₂.

In another embodiment, a method is provided for producing human-like glycoproteins in a Pichia pastoris host cell the method comprising the step of producing UDP-galactose above endogenous levels.

In yet another embodiment, a method is provided for producing human-like gly-I coprotein composition in Pichia pastoris host cell comprising the step of transferring a galactose residue on a hybrid or complex glycoprotein in the absence of fucose and static acid residues.

The present invention discloses at least 10%, preferably 33%, more preferably 60% or greater galactosylated glycoprotein composition is produced.
The present invention further discloses a recombinant Pichia pastoris host cell expressing GalNAc Transferase activity.

The present invention also discloses a recombinant Pichia pastoris host cell expressing a gene encoding heterologous UDPase activity.

Additionally, the present invention discloses an isolated polynucleotide comprising or consisting of a nucleic acid sequence selected from the group consisting of: (a) SEQ ID NO: 14; (b) at least about 90% similar to the amino acid residues of the donor nucleotide binding site of SEQ ID NO: 13; (c) a nucleic acid sequence at least 92%, at least 95%, at least 98%, at least 99% or at least 99.9% identical to SEQ ID NO: 14; (d) a nucleic acid sequence that encodes a conserved polypeptide having the amino acid I sequence of SEQ ID NO: 13; (e) a nucleic acid sequence that encodes a polypeptide at least 78%, at least 80%, at least 85%, at least 90%, at least 95%, at least 98%, at least 99% or at least 99.9% identical to SEQ ID NO: 13; (f) a nucleic acid sequence that hybridizes under stringent conditions to SEQ ID NO: 13; and (g) a nucleic acid sequence comprising a fragment of any one of (a)-(f) that is at least 60 contiguous nucleotides in length.

Herein is also disclosed a modified polynucleotide comprising or consisting of a nucleic acid sequence selected from the group consisting of the conserved regions of SEQ ID NO: 48-SEQ ID NO: 52 wherein the encoded polypeptide is involved in catalyzing the interconversion of UDP-glucose and UDP-galactose for production of I galactosylated glycoproteins. The present invention is provided within the claims.

### BRIEF DESCRIPTION OF THE DRAWINGS

Figure 1A-1B depicts the construction of a plasmid map of the integration vector pXB53 encoding hGalTI.

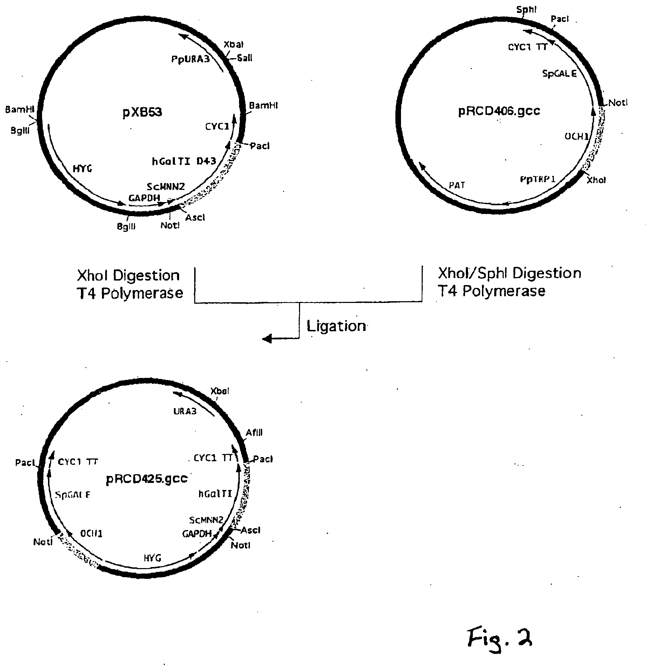

Figure 2 depicts the construction of a plasmid map of the integration vector pRCD425 encoding the S. *pombe* Gal epimerase (*Sp*GalE) and hGalTI.
Figure 3A-3B depicts the construction of a plasmid map of the integration vector pSH263 encoding the *D. melanogaster* UDP-galactose Transporter (*Dm*UGT).
Figure 4 depicts the construction of a plasmid map of the integration vector pRCD465 encoding hGalTI, *Sp*GalE and *Dm*UGT.
Figure 5 depicts the construction of a plasmid map of the integration vector pRCD461 encoding the *Sc*Mnn2/SpGalE/hGalTI fusion protein.

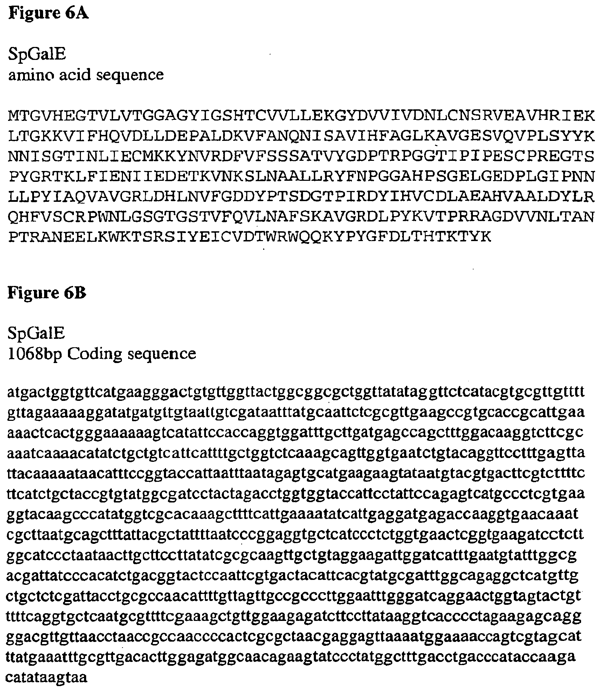

**Figure 6A** depicts the amino acid sequence of *Sp*GalE. **Figure 6B** depicts the coding sequence of *SpGALE.*
**Figure 7** shows a sequence alignment of *S. pombe*, human, *E. coli* and *S. cerevisiae* epimerases.
**Figure 8A** is a MALDI-TOF-MS analysis of N-glycans released from K3 produced in **RDP30-10** (**RDP27** transformed with **pRCD257**) displaying a peak at 1342 m/z **[A]** corresponding to the mass of the *N*-glycan GlcNAc₂Man₃GlcNAc₂.
**Figure 8B** is a MALDI-TOF-MS analysis of N-glycans released from K3 produced in **RDP37** (**RDP30-10** transformed with **pXB53**) displaying a peak at 1505 m/z **[B], which** corresponds to the mass of the *N*-glycan GalGlcNAc₂Man₃GlcNAc₂ and a peak at 1662 m/z [C], which corresponds to the mass of Gal₂GlcNAc₂Man₃GlcNAc₂.
**Figure 9A** is a MALDI-TOF-MS analysis of N-glycans released from K3 produced in **YSH-44** transformed with **pXB53** displaying a peak at 1501 m/z **[B],** which corresponds to the mass of the N-glycan GalGlcNAc₂Man₃GlcNAc₂ and a peak at 1339 m/z **[A],** which corresponds to the mass of GlcNAc₂Man₃GlcNAc₂.
**Figure 9B** is a MALDI-TOF-MS analysis of N-glycans released from K3 produced in **YSH-44** transformed with **pXB53** and **pRCD395** displaying a peak at 1501 m/z **[B],** which corresponds to the mass of the N-glycan GalGlcNAc₂Man₃GlcNAc₂ ; a peak at 1663 m/z **[C],** which corresponds to the mass of Gal₂GlcNAc₂Man₃GlnNAc₂ ; and **a peak** at 1339 m/z **[A],** which corresponds to the mass of GlcNAc₂Man₃GlcNAc₂.
**Figure 10A** is a MALDI-TOF-MS analysis of N-glycans released from K3 produced in **RDP 39-6** *(P. pastoris* **PBP-3** (US Pat. Appl. No. 20040018590)) transformed with **pRCD352** and **pXB53** displaying a predominant peak at 1622 m/z **[K],** which corresponds to the mass of the N-glycan GalGlcNAcMan₅GlcNAc₂ and a peak at 1460 m/z **[H]**, which corresponds to the mass of GlcNAcMan₅GlcNAc₂.
**Figure 10B** is a MALDI-TOF-MS analysis of N-glycans released from K3 produced in **RDP 39-6** after α1,2 and β1,4-galactosidase digest displaying a predominant peak at 1461 m/z **[H]**, which corresponds to the mass of the N-glycan GlcNAcMan₅GlcNAc₂.

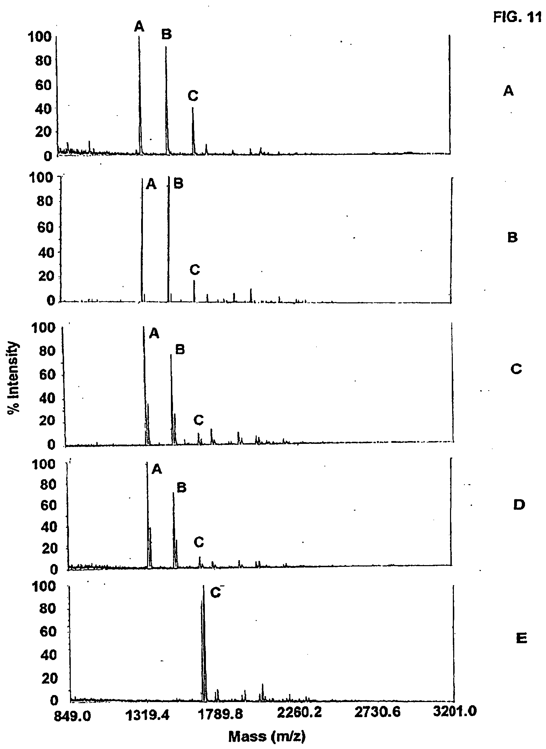

**Figure 11** is a MALDI-TOF-MS analysis of N-glycans isolated from K3 produced in various *P. pastoris* strains comparing the UDP-galactose transport activities. Panel A shows the N-glycan profile of *P. pastoris* **YSH-44** transformed with vectors **pRCD425** encoding Mnn2(s)/hGalTI and *Sp*GalE, which was designated **RDP52.** Panel **B** shows the N-glycan profile of *P. pastoris* **YSH-44** transformed with vectors **pRCD425** and **pRCD393** encoding SpUGT, which was designated as **RDP69.** Panel C shows the N-glycan profile of *P. pastoris* **YSH-44** transformed with vectors **pRCD425** and **pSH262** encoding hUGT2, which was designated as **RDP70.** Panel **D** shows the N-glycan profile of *P. pastoris* **YSH-44** transformed with vectors **pRCD425** and **pSH264** encoding hUGTI, which was designated as **RDP71.** Panel **E** shows the N-glycan profile of *P. pastoris* **YSH-44** transformed with vectors **pRCD425** and **pSH263** encoding *Dm*UGT, which was designated **RDP57.**

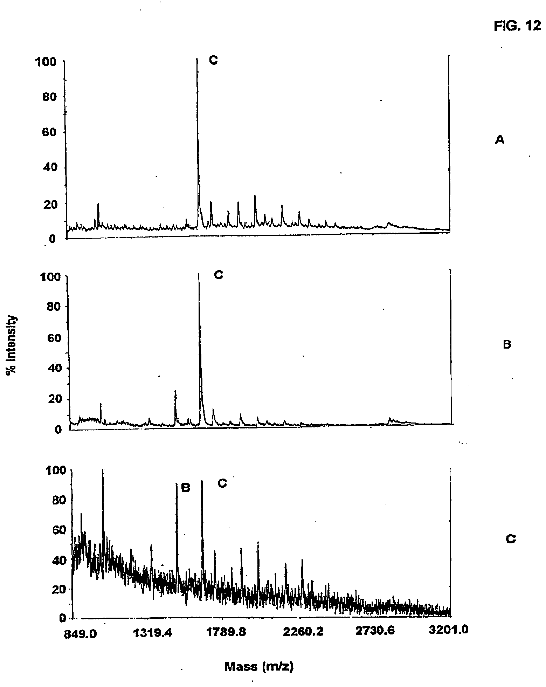

**Figure 12** is a MALDI-TOF-MS analysis of N-glycans released from K3 produced in various *P. pastoris* strains comparing the β-1,4-galactosyltransferase activities. Panel A shows the N-glycan profile of *P*. *pastoris* **YSH-44** transformed with vectors **pRCD425** and **pSH263** encoding DmUGT, which was designated as **RDP57.** Panel **B** shows the N-glycan profile of *P. pastoris* **YSH-44** transformed with vectors **pRCD440** encoding Mnn2(s)/hGalTII and SpGalE and **pSH263** encoding DmUGT, which was designated as **RDP72.** Panel **C** shows the N-glycan profile of *P. pastoris* **YSH-44** transformed with vectors **pRCD443** encoding Mnn2(s)/hGalTIII and SpGalE and **pSH263** encoding DmUGT, which was designated as **RDP73.**

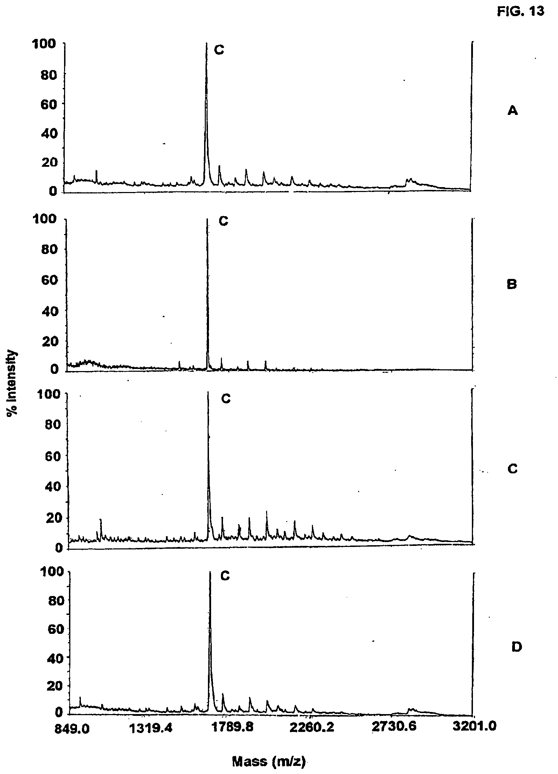

**Figure 13** is a MALDI-TOF-MS analysis of N-glycans released from K3 produced in various *P. pastoris* strains comparing epimerase activities. Panel **A** shows the N-glycan profile of *P. pastoris* **YSH-44** transformed with vectors **pRCD424** encoding Mnn2(s)/ hGalTI and ScGa110 and **pSH263** encoding DmUGT, which was designated as **RDP65.** Panel **B** shows the N-glycan profile of *P. pastoris* **YSH-44** sequentially transformed with vectors **pSH263** encoding *Dm*UGT and **pRCD425,** which was designated as **RDP74.** Panel **C** shows the N-glycan profile of *P. pastoris* **YSH-44** sequentially transformed with vectors **pRCD425** and then **pSH263** encoding DmUGT, which was designated as **RDP63.** Panel **D** shows the N-glycan profile of *P. pastoris* **YSH-44** transformed with vectors **pXB53** and **pRCD438** encoding Mnn2(s)/hGalTI and hGalE and **pSH263** encoding DmUGT, which was designated as **RDP67.**
**Figure 14A** is a MALDI-TOF-MS analysis of N-glycans released from K3 produced in **RDP80** *(P. pastoris* **YSH-44** transformed with **pRCD465**) displaying a predominant peak at 1663 m/z **[C],** which corresponds to the mass of the N-glycan Gal GlcNAc Man GlcNAc. 2 2 3 2
**Figure 14B** is a MALDI-TOF-MS analysis of N-glycans released from K3 produced in **RDP80** *(P. pastoris* **YSH-44** transformed with **pRCD465)** after β1,4-galactosidase digest displaying a predominant peak at 1340 m/z **[A],** which corresponds to the mass of the N-glycan GlcNAc₂Man₃GlcNAc₂.
**Figure 14C** is a MALDI-TOF-MS analysis of N-glycans released from K3 produced in **RDP80** and incubated with sialyltransferase *in vitro* in the presence of CMP-NANA, displaying a predominant peak at 2227 m/z **[X],** which corresponds to the mass of the N-glycan NANA₂Gal₂GlcNAc₂Man₃GlcNAc₂.
**Figure 15A** is a MALDI-TOF-MS analysis depicting the N-glycan GlcNAc₂Man₃GlcNAc₂**[A]** released from K3 produced in *P*. *pastoris* **YSH-44** (control). **Figure 15B** is a MALDI-TOF-MS analysis of N-glycans released from K3 produced in **RDP86** (*P*.*P. pastoris* YSH-44 transformed with pRCD461 (Mnn2(s)/SpGalE/hGalTI fusion) displaying a predominant peak at 1679 m/z [C], which corresponds to the mass of the N-glycan Gal₂GlcNAc₂Man₃GlcNAc₂.

### DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well known in the art. Generally, nomenclatures used in connection with, and techniques of biochemistry, enzymology, molecular and cellular biology, microbiology, genetics and protein and nucleic acid chemistry and hybridization described herein are those well known and commonly used in the art. The methods and techniques of the present invention are generally performed according to conventional methods well known in the art and as described in various general and more specific; references that are cited and discussed throughout the present specification unless otherwise indicated. See, e.g., Sambrook et al. Molecular Cloning: A Laboratory Manual, 2d ea., Cold Spring Harbor Laboratory Press, Cold Spring Harbor. N.Y.(1989); Ausubel et al., Current Protocols in Molecular Biology, Greene Publishing Associates (1992, and Supplements to 2002); Harlow and Lane Antibodies: A Laboratory Manual Cold:Spring Harbor Laboratory Press, Cold Spring Harbor, N.Y. (1990); Introduction to Glycobiology, Maureen E. Taylor, Kurt Drickamer, Oxford Univ. Press (2003); Worthington Enzyme Manual, Worthington Biochemical Corp. Freehold, NJ; Handbook of Biochemistry: Section A Proteins Vol I 1976 CRC Press; Handbook of Biochemistry: Section A Proteins Vol II 1976 CRC Press; Essentials of Glycobiology, Cold Spring Harbor Laboratory Press (1999). The nomenclatures used in connection with, and the laboratory procedures and techniques of, biochemistry and molecular biology described herein are those well known and commonly used in the; art.

The following terms, unless otherwise indicated, shall be understood to have the following meanings:

As used herein, the term 'K3' refers to the kringle 3 domain of human plasminogen.

As used herein, the term 'N-glycan' refers to an N-linked oligosaccharide, e.g., one that is attached by an asparagine-N-acetylglucosamine linkage to an asparagine residue of a polypeptide. N-glycans have a common pentasaccharide core of Man GlcNAc ('Man' refers to mannose; 'Glc' refers to glucose; end 'NAcl refers to N-acetyl; GlcNAc refers to N-acetylglucosamine). N-glycans differ with respect to the number of branches (antennae) comprising peripheral sugars (e.g., GlcNAc, galactose, fucose and sialic acid) that are added to the Man₃GlcNAc₂('Man3') core structure. N-glycans are classified according to their branched constituents (e.g., high mannose, complex or hybrid). A 'high mannose' type N-glycan has five or more mannose residues. A 'complex' type N-glycan typically has at least one GlcNAc attached to the 1,3 mannose arm and at least one GlcNAc attached to the 1,6 mannose arm of a 'trimannose' core. The 'trimannose core' is the pentasaccharide core having a Man3 structure. It is often referred to as 'paucimannose' structure. Complex N-glycans may also have galactose ('Gal') residues that are optionally modified with sialic acid or derivatives ('NeuAc', where 'Neu' refers to neuraminic acid and 'Ac' refers to acetyl). Complex N-glycans may also have intrachain substitutions comprising 'bisecting' GlcNAc and core fucose ('Fuc'). Complex N-glycans may also have multiple antennae on the 'trimannose core,' often referred to as 'multiple antennary glycans.' A 'hybrid' N-glycan has at least one GlcNAc on the terminal of the 1,3 mannose arm of the trimannose core and zero or more mannoses on the 1,6 mannose arm of the trimannose core.

Abbreviations used herein are of common usage in the art, see, e.g., abbreviations of sugars, above. Other common abbreviations include 'PNGase', which refers to peptide N-glycosidase F (EC 3.2.2.18); 'GalT', which refers to Galactosyl transferase, 'β1,4Ga1T', which refers to UDP-galactose: β-*N*-acetylglucosamine β1,4-galactosyltransferase. β-Galactosyltransferases from various species are abbreviated as follows: 'hGalT' refers to human β1,4-galactosyltransferase, 'bGalT' refers to bovine β1,4-galactosyltransferase, '*Xl*GalT' refers to *Xenopus leavis* β1,4-galactosyltransferase and '*Ce*GalT' refers to *C*. *elegans* β1,4-galactosyltransferase. 'GalNAcT' refers to UDP-GalNAc-GlcNAc β-1,4-N-acetyl-galactosaminyltransferase.

As used herein, the term 'UGT' refers to UDP-galactose transporter. The term '*Sp* GalE' refers to *S. pombe* UDP-galactose 4-epimerase, 'hGalE' refers to human UDP-galactose 4-epimerase, '*Sc*Gal10' refer to *S. cerevisiae* UDP-galactose 4-epimerase and '*Ec*GalE' refers to *E. coli* UDP-galactose 4-epimerase.

As used herein, the term 'UDP-Gal' refers to UDP-galactose and the term 'UDP-GalNAc' refers to UDP-N-acetylgalactosamine.

N-linked glycoproteins contain an N-acetylglucosamine residue linked to the amide nitrogen of an asparagine residue in the protein. The predominant sugars found on glycoproteins are glucose, galactose, mannose, fucose, N-acetylgalactosamine (GalNAc), N-acetylglucosamine (GlcNAc) and sialic acid (e.g., N-acetyl-neuraminic acid (NANA)). The processing of the sugar groups occurs cotranslationally in the lumen of the ER and continues in the Golgi apparatus for N-linked glycoproteins.

As used herein, the term 'human-like' glycoprotein refers to modified N-glycans covalently attached to a protein that are similar to the glycoproteins found in the human N-linked oligosaccharide synthesis. Complex and hybrid N-glycans are intermediates found in human glycosylation. Common to these intermediates is the Man₃GlcNAc₂ core structure also referred to as the paucimannose core, pentasaccharide core or simply Man3 or Man₃. Human-like glycoproteins, therefore, have at least the Man3 core structure.

As used herein, the term 'initiating 1,6 mannosyltransferase activity' refers to yeast specific glycan residues typically added to the Manα1,3 arm of the trimannose core in outer chain formation initiated by Ochlp with an α1,6 linkage.

The mole % transfer of galactose residue onto N-glycans as measured by MALDI-TOF-MS in positive mode refers to mole % galactose transfer with respect to mole % total neutral N-glycans. Certain cation adducts such as K⁺ and Na⁺ are normally associated with the peaks eluted increasing the mass of the N-glycans by the molecular mass of the respective adducts.

As used herein, the term 'secretion pathway' refers to the assembly line of various glycosylation enzymes to which a lipid-linked oligosaccharide precursor and an N-glycan substrate are sequentially exposed, following the molecular flow of a nascent polypeptide chain from the cytoplasm to the endoplasmic reticulum (ER) and the compartments of the Golgi apparatus. Enzymes are said to be localized along this pathway. An enzyme X that acts on a lipid-linked glycan or an N-glycan before enzyme Y is said to be or to act 'upstream' to enzyme Y; similarly, enzyme Y is or acts 'downstream' from enzyme X.

As used herein, the term 'mutation' refers to any change in the nucleic acid or amino acid sequence of a gene product, e.g., of a glycosylation-related enzyme.

The term 'polynucleotide' or 'nucleic acid molecule' refers to a polymeric form of nucleotides of at least 10 bases in length. The term includes DNA molecules (e.g., cDNA or genomic or synthetic DNA) and RNA molecules (e.g., mRNA or synthetic RNA), as well as analogs of DNA or RNA containing non-natural nucleotide analogs, non-native internucleoside bonds, or both. The nucleic acid can be in any topological conformation. For instance, the nucleic acid can be single-stranded, double-stranded, triple-stranded, quadruplexed, partially double-stranded, branched, hairpinned, circular, or in a padlocked conformation. The term includes single and double stranded forms of DNA.

Unless otherwise indicated, a 'nucleic acid comprising SEQ ID NO:X' refers to a nucleic acid, at least a portion of which has either (i) the sequence of SEQ ID NO:X, or (ii) a sequence complementary to SEQ ID NO:X. The choice between the two is dictated by the context. For instance, if the nucleic acid is used as a probe, the choice between the two is dictated by the requirement that the probe be complementary to the desired target.

An 'isolated' or 'substantially pure' nucleic acid or polynucleotide (e.g., an RNA, DNA or a mixed polymer) is one which is substantially separated from other cellular components that naturally accompany the native polynucleotide in its natural host cell, e.g., ribosomes, polymerases, and genomic sequences with which it is naturally associated. The term embraces a nucleic acid or polynucleotide that (1) has been removed from its naturally occurring environment, (2) is not associated with all or a portion of a polynucleotide in which the 'isolated polynucleotide' is found in nature, (3) is operatively linked to a polynucleotide which it is not linked to in nature, or (4) does not occur in nature. The term 'isolated' or 'substantially pure' also can be used in reference to recombinant or cloned DNA isolates, chemically synthesized polynucleotide analogs, or polynucleotide analogs that are biologically synthesized by heterologous systems.

However, 'isolated' does not necessarily require that the nucleic acid or polynucleotide so described has itself been physically removed from its native environment. For instance, an endogenous nucleic acid sequence in the genome of an organism is deemed 'isolated' herein if a heterologous sequence (i.e., a sequence that is not naturally adjacent to this endogenous nucleic acid sequence) is placed adjacent to the endogenous nucleic acid sequence, such that the expression of this endogenous nucleic acid sequence is altered. By way of example, a non-native promoter sequence can be substituted (e.g., by homologous recombination) for the native promoter of a gene in the genome of a human cell, such that this gene has an altered expression pattern. This gene would now become 'isolated' because it is separated from at least some of the sequences that naturally flank it.

A nucleic acid is also considered 'isolated' if it contains any modifications that do not naturally occur to the corresponding nucleic acid in a genome. For instance, an endogenous coding sequence is considered 'isolated' if it contains an insertion, deletion or a point mutation introduced artificially, e.g., by human intervention. An 'isolated nucleic acid' also includes a nucleic acid integrated into a host cell chromosome at a heterologous site, a nucleic acid construct present as an episome. Moreover, an 'isolated nucleic acid' can be substantially free of other cellular material, or substantially free of culture medium when produced by recombinant techniques, or substantially free of chemical precursors or other chemicals when chemically synthesized.

As used herein, the phrase 'degenerate variant' of a reference nucleic acid sequence encompasses nucleic acid sequences that can be translated, according to the standard genetic code, to provide an amino acid sequence identical to that translated from the reference nucleic acid sequence.

The term 'percent sequence identity' or 'identical' in the context of nucleic acid sequences refers to the residues in the two sequences which are the same when aligned for maximum correspondence. The length of sequence identity comparison may be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides. There are a number of different algorithms known in the art which can be used to measure nucleotide sequence identity. For instance, polynucleotide sequences can be compared using FASTA, Gap or Bestfit, which are programs in Wisconsin Package Version 10.0, Genetics Computer Group (GCG), Madison, Wisconsin. FASTA provides alignments and percent sequence identity of the regions of the best overlap between the query and search sequences (Pearson, 1990, (herein incorporated by reference). For instance, percent sequence identity between nucleic acid sequences can be determined using FASTA with its default parameters (a word size of 6 and the NOPAM factor for the scoring matrix) or using Gap with its default parameters as provided in GCG Version 6.1, herein incorporated by reference.

The term 'substantial homology' or 'substantial similarity,' when referring to a nucleic acid or fragment thereof, indicates that, when optimally aligned with appropriate nucleotide insertions or deletions with another nucleic acid (or its complementary strand), there is nucleotide sequence identity in at least about 50%, more preferably 60% of the nucleotide bases, usually at least about 70%, more usually at least about 80%, preferably at least about 90%, and more preferably at least about 95%, 96%, 97%, 98% or 99% of the nucleotide bases, as measured by any well-known algorithm of sequence identity, such as FASTA, BLAST or Gap, as discussed above.

Alternatively, substantial homology or similarity exists when a nucleic acid or fragment thereof hybridizes to another nucleic acid, to a strand of another nucleic acid, or to the complementary strand thereof, under stringent hybridization conditions. 'Stringent hybridization conditions' and 'stringent wash conditions' in the context of nucleic acid hybridization experiments depend upon a number of different physical parameters. Nucleic acid hybridization will be affected by such conditions as salt concentration, temperature, solvents, the base composition of the hybridizing species, length of the complementary regions, and the number of nucleotide base mismatches between the hybridizing nucleic acids, as will be readily appreciated by those skilled in the art. One having ordinary skill in the art knows how to vary these parameters to achieve a particular stringency of hybridization.

In general, 'stringent hybridization' is performed at about 25 °C below the thermal melting point (Tₘ) for the specific DNA hybrid under a particular set of conditions. 'Stringent washing' is performed at temperatures about 5 °C lower than the Tₘ for the specific DNA hybrid under a particular set of conditions. The Tₘ is the temperature at which 50% of the target sequence hybridizes to a perfectly matched probe. See Sambrook et al., *sura,* page 9.51, hereby incorporated by reference. For purposes herein, 'high stringency conditions' are defined for solution phase hybridization as aqueous hybridization (i.e., free of formamide) in 6X SSC (where 20X SSC contains 3.0 M NaCl and 0.3 M sodium citrate), 1% SDS at 65°C for 8-12 hours, followed by two washes in 0.2X SSC, 0.1 % SDS at 65°C for 20 minutes. It will be appreciated by the skilled worker that hybridization at 65 °C will occur at different rates depending on a number of factors including the length and percent identity of the sequences which are hybridizing.

The nucleic acids (also referred to as polynucleotides) of this invention may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The term 'mutated' when applied to nucleic acid sequences means that nucleotides in a nucleic acid sequence may be inserted, deleted or changed compared to a reference nucleic acid sequence. A single alteration may be made at a locus (a point mutation) or multiple nucleotides may be inserted, deleted or changed at a single locus. In addition, one or more alterations may be made at any number of loci within a nucleic acid sequence. A nucleic acid sequence may be mutated by any method known in the art including but not limited to mutagenesis techniques such as 'error-prone PCR' (a process for performing PCR under conditions where the copying fidelity of the DNA polymerase is low, such that a high rate of point mutations is obtained along the entire length of the PCR product. See, e.g., Leung, D. W., et al., Technique, 1, pp. 11-15 (1989) and Caldwell, R. C. & Joyce G. F., PCR Methods Applic., 2, pp. 28-33 (1992)); and 'oligonucleotide-directed mutagenesis' (a process which enables the generation of site-specific mutations in any cloned DNA segment of interest. See, e.g., Reidhaar-Olson, J. F. & Sauer, R. T., et al., Science, 241, pp. 53-57 (1988)).

The term 'vector' as used herein is intended to refer to a nucleic acid molecule capable of transporting another nucleic acid to which it has been linked. One type of vector is a 'plasmid', which refers to a circular double stranded DNA loop into which additional DNA segments may be ligated. Other vectors include cosmids, bacterial artificial chromosomes (BAC) and yeast artificial chromosomes (YAC). Another type of vector is a viral vector, wherein additional DNA segments may be ligated into the viral genome (discussed in more detail below). Certain vectors are capable of autonomous replication in a host cell into which they are introduced (e.g., vectors having an origin of replication which functions in the host cell). Other vectors can be integrated into the genome of a host cell upon introduction into the host cell, and are thereby replicated along with the host genome. Moreover, certain preferred vectors are capable of directing the expression of genes to which they are operatively linked. Such vectors are referred to herein as 'recombinant expression vectors' (or simply, 'expression vectors').

'Operatively linked' expression control sequences refers to a linkage in which the expression control sequence is contiguous with the gene of interest to control the gene of interest, as well as expression control sequences that act in *trans* or at a distance to control the gene of interest.

The term 'expression control sequence' as used herein refers to polynucleotide sequences which are necessary to affect the expression of coding sequences to which they are operatively linked. Expression control sequences are sequences which control the transcription, post-transcriptional events and translation of nucleic acid sequences. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (e.g., ribosome binding sites); sequences that enhance protein stability; and when desired, sequences that enhance protein secretion. The nature of such control sequences differs depending upon the host organism; in prokaryotes, such control sequences generally include promoter, ribosomal binding site, and transcription termination sequence. The term 'control sequences' is intended to include, at a minimum, all components whose presence is essential for expression, and can also include additional components whose presence is advantageous, for example, leader sequences and fusion partner sequences.

The term 'recombinant lower eukaryotic host cell' (or simply 'host cell'), as used herein, is intended to refer to a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term 'host cell' as used herein. A recombinant host cell may be an isolated cell or cell line grown in culture or may be a cell which resides in a living tissue or organism. A recombinant host cell includes yeast, fungi, collar-flagellates, microsporidia, alveolates (e.g., dinoflagellates), strameno-piles (e.g, brown algae, protozoa), rhodophyta (e.g., red algae), plants (e.g., green algae, plant cells, moss) and other protists.

The term 'peptide' as used herein refers to a short polypeptide, e.g., one that is typically less than about 50 amino acids long and more typically less than about 30 amino acids long. The term as used herein encompasses analogs and mimetics that mimic structural and thus biological function.

The term 'polypeptide' encompasses both naturally-occurring and non-naturally-occurring proteins, and fragments, mutants, derivatives and analogs thereof. A polypeptide may be monomeric or polymeric. Further, a polypeptide may comprise a number of different domains each of which has one or more distinct activities.

The term 'isolated protein' or 'isolated polypeptide' is a protein or polypeptide that by virtue of its origin or source of derivation (1) is not associated with naturally associated components that accompany it in its native state, (2) when it exists in a purity not found in nature, where purity can be adjudged with respect to the presence of other cellular material (e.g., is free of other proteins from the same species) (3) is expressed by a cell from a different species, or (4) does not occur in nature (e.g., it is a fragment of a polypeptide found in nature or it includes amino acid analogs or derivatives not found in nature or linkages other than standard peptide bonds). Thus, a polypeptide that is chemically synthesized or synthesized in a cellular system different from the cell from which it naturally originates will be 'isolated' from its naturally associated components. A polypeptide or protein may also be rendered substantially free of naturally associated components by isolation, using protein purification techniques well known in the art. As thus defined, 'isolated' does not necessarily require that the protein, polypeptide, peptide or oligopeptide so described has been physically removed from its native environment.

The term 'polypeptide fragment' as used herein refers to a polypeptide that has an amino-terminal and/or carboxy-terminal deletion compared to a full-length polypeptide. In a preferred embodiment, the polypeptide fragment is a contiguous sequence in which the amino acid sequence of the fragment is identical to the corresponding positions in the naturally-occurring sequence. Fragments typically are at least 5, 6, 7, 8, 9 or 10 amino acids long, preferably at least 12, 14, 16 or 18 amino acids long, more preferably at least 20 amino acids long, more preferably at least 25, 30, 35, 40 or 45, amino acids, even more preferably at least 50 or 60 amino acids long, and even more preferably at least 70 amino acids long.

A 'modified derivative' refers to polypeptides or fragments thereof that are substantially homologous in primary structural sequence but which include, e.g., *in vivo* or *in vitro* chemical and biochemical modifications or which incorporate amino acids that are not found in the native polypeptide. Such modifications include, for example, acetylation, carboxylation, phosphorylation, glycosylation, ubiquitination, labeling, e.g., with radionuclides, and various enzymatic modifications, as will be readily appreciated by those well skilled in the art. A variety of methods for labeling polypeptides and of substituents or labels useful for such purposes are well known in the art, and include radioactive isotopes such as ¹²¹I, ³²P, ³⁵S, and ³H, ligands which bind to labeled antiligands (e.g., antibodies), fluorophores, chemiluminescent agents, enzymes, and antiligands which can serve as specific binding pair members for a labeled ligand. The choice of label depends on the sensitivity required, ease of conjugation with the primer, stability requirements, and available instrumentation. Methods for labeling polypeptides are well known in the art. See Ausubel et al., 1992.

The term 'fusion protein' refers to a polypeptide comprising a polypeptide or fragment coupled to heterologous amino acid sequences. Fusion proteins are useful because they can be constructed to contain two or more desired functional elements from two or more different proteins. A fusion protein comprises at least 10 contiguous amino acids from a polypeptide of interest, more preferably at least 20 or 30 amino acids, even more preferably at least 40, 50 or 60 amino acids, yet more preferably at least 75, 100 or 125 amino acids. Fusion proteins can be produced recombinantly by constructing a nucleic acid sequence which encodes the polypeptide or a fragment thereof in frame with a nucleic acid sequence encoding a different protein or peptide and then expressing the fusion protein. Alternatively, a fusion protein can be produced chemically by crosslinking the polypeptide or a fragment thereof to another protein.

The term 'non-peptide analog' refers to a compound with properties that are analogous to those of a reference polypeptide. A non-peptide compound may also be termed a 'peptide mimetic' or a 'peptidomimetic'. See, e.g., Jones, (1992) Amino Acid and Peptide Synthesis, Oxford University Press; Jung, (1997) Combinatorial Peptide and Nonpeptide Libraries: A Handbook John Wiley; Bodanszky et al., (1993) Peptide Chemistry--A Practical Textbook, Springer Verlag; 'Synthetic Peptides: A Users Guide', G. A. Grant, Ed, W. H. Freeman and Co., 1992; Evans et al. J. Med. Chem. 30:1229 (1987); Fauchere, J. Adv. Drug Res. 15:29 (1986); Veber and Freidinger TINS p.392 (1985); and references sited in each of the above. Such compounds are often developed with the aid of computerized molecular modeling. Peptide mimetics that are structurally similar to useful peptides of the invention may be used to produce an equivalent effect and are therefore envisioned to be part of the invention.

A 'polypeptide mutant' or 'mutein' refers to a polypeptide whose sequence contains an insertion, duplication, deletion, rearrangement or substitution of one or more amino acids compared to the amino acid sequence of a native or wild type protein. A mutein may have one or more amino acid point substitutions, in which a single amino acid at a position has been changed to another amino acid, one or more insertions and/or deletions, in which one or more amino acids are inserted or deleted, respectively, in the sequence of the naturally-occurring protein, and/or truncations of the amino acid sequence at either or both the amino or carboxy termini. A mutein may have the same but preferably has a different biological activity compared to the naturally-occurring protein.

A mutein has at least 70% overall sequence homology to its wild-type counterpart. Even more preferred are muteins having 80%, 85% or 90% overall sequence homology to the wild-type protein. In an even more preferred embodiment, a mutein exhibits 95% sequence identity, even more preferably 97%, even more preferably 98% and even more preferably 99%, 99.5% or 99.9% overall sequence identity. Sequence homology may be measured by any common sequence analysis algorithm, such as Gap or Bestfit.

Preferred amino acid substitutions are those which: (1) reduce susceptibility to proteolysis, (2) reduce susceptibility to oxidation, (3) alter binding affinity for forming protein complexes, (4) alter binding affinity or enzymatic activity, and (5) confer or modify other physicochemical or functional properties of such analogs.

As used herein, the twenty conventional amino acids and their abbreviations follow conventional usage. *See* Immunology - A Synthesis (2nd Edition, E.S. Golub and D.R. Gren, Eds., Sinauer Associates, Sunderland, Mass. (1991)). Stereoisomers (e.g., D-amino acids) of the twenty conventional amino acids, unnatural amino acids such as α-, α-disubstituted amino acids, N-alkyl amino acids, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methyl-histidine, 5-hydroxylysine, s-N-methylarginine, and other similar amino acids and imino acids (e.g., 4-hydroxyproline). In the polypeptide notation used herein, the left-hand direction is the amino terminal direction and the right hand direction is the carboxy-terminal direction, in accordance with standard usage and convention.

A protein has 'homology' or is 'homologous' to a second protein if the nucleic acid sequence that encodes the protein has a similar sequence to the nucleic acid sequence that encodes the second protein. Alternatively, a protein has homology to a second protein if the two proteins have 'similar' amino acid sequences. (Thus, the term 'homologous proteins' is defined to mean that the two proteins have similar amino acid sequences). In a preferred embodiment, a homologous protein is one that exhibits 60% sequence homology to the wild type protein, more preferred is 70% sequence homology. Even more preferred are homologous proteins that exhibit 80%, 85% or 90% sequence homology to the wild type protein. In a yet more preferred embodiment, a homologous protein exhibits 95%, 97%, 98% or 99% sequence identity. As used herein, homology between two regions of amino acid sequence (especially with respect to predicted structural similarities) is interpreted as implying similarity in function.

When 'homologous' is used in reference to proteins or peptides, it is recognized that residue positions that are not identical often differ by conservative amino acid substitutions. A 'conservative amino acid substitution' is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of homology may be adjusted upwards to correct for the conservative nature of the substitution. Means for making this adjustment are well known to those of skill in the art (see, e.g., Pearson et al., 1994).

The following six groups each contain amino acids that are conservative substitutions for one another: 1) Serine (S), Threonine (T); 2) Aspartic Acid (D), Glutamic Acid (E); 3) Asparagine (N), Glutamine (Q); 4) Arginine (R), Lysine (K); 5) Isoleucine (I), Leucine (L), Methionine (M), Alanine (A), Valine (V), and 6) Phenylalanine (F), Tyrosine (Y), Tryptophan (W).

Sequence homology for polypeptides, which is also referred to as percent sequence identity, is typically measured using sequence analysis software. See, e.g., the Sequence Analysis Software Package of the Genetics Computer Group (GCG), University of Wisconsin Biotechnology Center, 910 University Avenue, Madison, Wisconsin 53705. Protein analysis software matches similar sequences using measure of homology assigned to various substitutions, deletions and other modifications, including conservative amino acid substitutions. For instance, GCG contains programs such as 'Gap' and 'Bestfit' which can be used with default parameters to determine sequence homology or sequence identity between closely related polypeptides, such as homologous polypeptides from different species of organisms or between a wild type protein and a mutein thereof. See, e.g., GCG Version 6.1.

A preferred algorithm when comparing a inhibitory molecule sequence to a database containing a large number of sequences from different organisms is the computer program BLAST (Altschul, S.F. et al. (1990) J. Mol. Biol. 215:403-410; Gish and States (1993) Nature Genet. 3:266-272; Madden, T.L. et al. (1996) Meth. Enzymol. 266:131-141; Altschul, S.F. et al. (1997) Nucleic Acids Res.25:3389-3402; Zhang, J. and Madden, T.L. (1997) Genome Res. 7:649-656), especially blastp or tblastn (Altschul et al., 1997). Preferred parameters for BLASTp are: Expectation value: 10 (default); Filter: seg (default); Cost to open a gap: 11 (default); Cost to extend a gap: 1 (default); Max. alignments: 100 (default); Word size: 11 (default); No. of descriptions: 100 (default); Penalty Matrix: BLOWSUM62.

The length of polypeptide sequences compared for homology will generally be at least about 16 amino acid residues, usually at least about 20 residues, more usually at least about 24 residues, typically at least about 28 residues, and preferably more than about 35 residues. When searching a database containing sequences from a large number of different organisms, it is preferable to compare amino acid sequences. Database searching using amino acid sequences can be measured by algorithms other than blastp known in the art. For instance, polypeptide sequences can be compared using FASTA, a program in GCG Version 6.1. FASTA provides alignments and percent sequence identity of the regions of the best overlap between the query and search sequences (Pearson, 1990, herein incorporated by reference). For example, percent sequence identity between amino acid sequences can be determined using FASTA with its default parameters (a word size of 2 and the PAM250 scoring matrix), as provided in GCG Version 6.1.

The term 'domain' as used herein refers to a structure of a biomolecule that contributes to a known or suspected function of the biomolecule. Domains may be coextensive with regions or portions thereof; domains may also include distinct, non-contiguous regions of a biomolecule. Examples of protein domains include, but are not limited to, an Ig domain, an extracellular domain, a transmembrane domain, and a cytoplasmic domain.

As used herein, the term 'molecule' means any compound, including, but not limited to, a small molecule, peptide, protein, sugar, nucleotide, nucleic acid, lipid, etc., and such a compound can be natural or synthetic.

Throughout this specification and its embodiments, the word 'comprise' or variations such as 'comprises' or 'comprising', will be understood to refer to the inclusion of a stated integer or group of integers but not the exclusion of any other integer or group of integers.

Unless otherwise defined, all technical and scientific terms used herein have the same meaning as commonly understood by one of ordinary skill in the art to which this invention pertains. Exemplary methods and materials are described below, although methods and materials similar or equivalent to those described herein can also be used in the practice of the present invention and will be apparent to those of skill in the art. In case of conflict, the present specification, including definitions, will control. The materials, methods, and examples are illustrative only and not intended to be limiting.

### Engineering Hosts To Produce Human-Like Galactosylated Glycoproteins

The present invention provides a recombinant Pichia pastoris host cell producing human-like glycoproteins characterized as having a terminal β-galactose residue and essentially lacking fucose and sialic acid residues on the glycoprotein; wherein the host cell expresses: nucleic acid molecule encoding β1,4-galactosyltransferase activity and nucleic acid molecule encoding UDP-galactose C4 epimerase activity. Disclosed is a Pichia pastoris host cell comprising an isolated nucleic acid molecule encoding UDP-galactose: β-N-acetylglucosamine, β1,4-galactosyltransferase (β1,4GalT) in combination with at least a second isolated nucleic acid molecule encoding a UDP-galactose transporter, an isolated nucleic acid encoding a UDP-galactose 4-epimerase or an isolated nucleic acid encoding galactokinase or galactose-1-phosphate uridyl transferase. β1,4GalT is expressed in combination with an isolated nucleic acid molecule encoding a UDP-galactose transporter and an isolated nucleic acid molecule encoding a UDP galactose 4-epimerase. Variants and fragments of the nucleic acid sequences encoding I the above enzymes, recombinant DNA molecules and expression vectors comprising the enzymes for transformation are also disclosed.

A method is disclosed to produce a human like glycoprotein in a Pichia pastoris host cell comprising the step of catalyzing the transfer of a galactose residue from UDP-galactose onto an acceptor substrate in a β linkage by expression of a β1,4GalT activity and introducing into the host a UDP galactose 4-epimerase activity, galactokinase activity, a galactose-1-phosphate uridyl transferase activity or a UDP-galactose transport activity. The acceptor substrate is preferably an oligosaccharide composition comprising a terminal GlcNAc residue, for example, GlcNAcβ1,2-Manα1,3; GlcNAcβ1,4-Manα1,3; GlcNAcp1,2-Manα1,6; GlcNAcβ31,4-Manα1,6; or GlcNAcβ1,6-Manα1,6 branch on a trimannose core.

The acceptor substrate is more preferably a complex glycan (e.g., GlcNAc₂ Man₃ GlcNAc), a hybrid glycan (e.g., GlcNAc₄Man₃GlcNAc₂) or a multiple antennary glycan (e.g., GlcNAc Man GlcNAc) that is covalently linked (N-linked) to a protein of interest. The β-galactose residue is transferred onto the acceptor substrate comprising a hydroxy group at carbon 4 of 2-acetamido-2-deoxy-D-glucose (GlcNAc) forming a β-glycosidic linkage. The N-linked acceptor substrates comprising a terminal GlcNAc residue capable of accepting a galactose residue include, without limitation, GlcNAcMa₃GcNAc₂, GlcNAc₂Man₃GlcNAc₂, GlcNAc₃Man₃GlcNAc₂, GlcNAc₄Man₃GlcNAc₂, GlcNAc₅Man₃GlcNAc₂GlcNAc₆Man₃GlcNAc₂, GlcNAcMaGlcNAc₂, GlcNAcMan₅GlcNAc₂, GlcNAc₂Man₅GlcNAc₂ and GlcNAc₃Man₅GlcNAc₂

### Cloning of β1,4-Galactosyltransferase genes

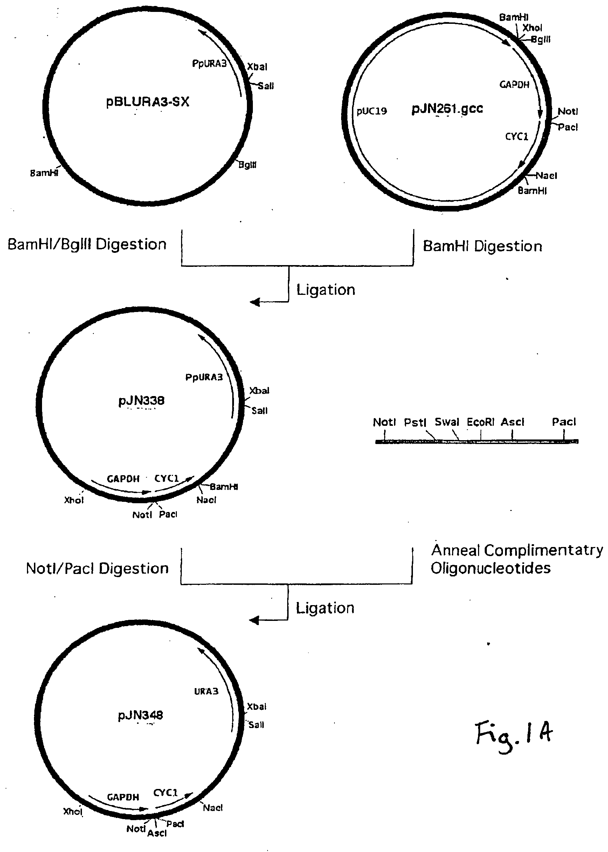

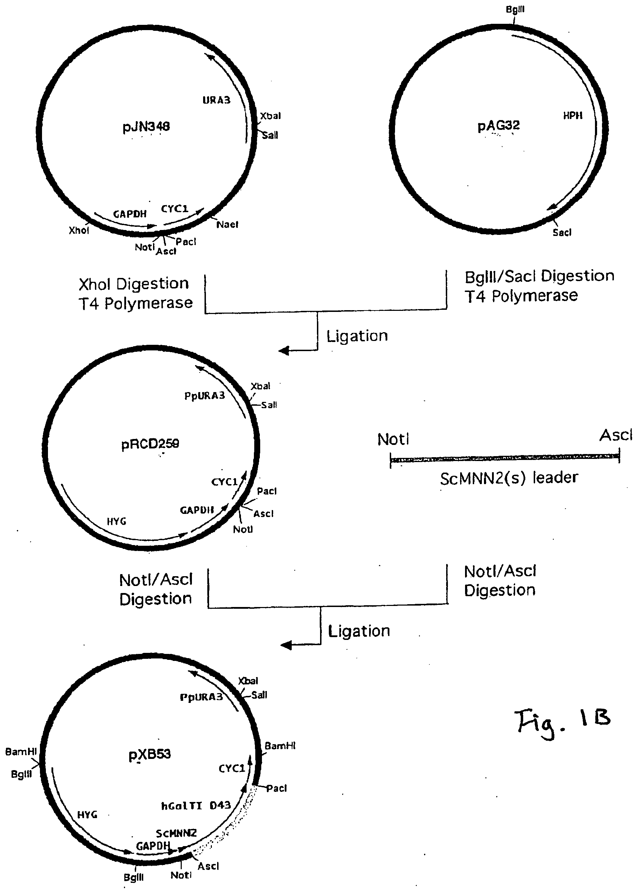

The human b-1,4-galactosyltransferase I gene (hGalTI, Genbank AH003575) was PCR amplified from human kidney cDNA (marathon ready cDNA, Clontech) using primers RCD192 (SEQ ID NO:1) and RCD186 (SEQ ID NO:2). This PCR product **was** cloned in **pCR2.1** (Invitrogen) cloned and sequenced. From this clone, a PCR overlap mutagenesis was performed. The 5' end of the gene up to the *NotI* site was amplified using primers RCD198 (SEQ ID NO:3) and RCD201 (SEQ ID NO:4) and the 3' end was amplified with primers RCD200 (SEQ ID NO:5) and RCD199 (SEQ ID NO:6). The products were overlapped together with primers RCD198 (SEQ ID NO:3) and RCD199 (SEQ ID NO:6) to resynthesize the ORF with the wild-type amino acid (except for an N-terminal deletion of 43 amino acids) sequence while eliminating the *Not*I site. The new truncated hGalTI PCR product was cloned in **pCR2.1** and sequenced. The introduced *Asc*I/*Pac*I sites were then used to subclone the fragment into plasmid **pRCD259 (****Figure 1****),** a *PpURA3*/*HY-G*^{R}roll-in vector creating **pRCD260 (****Figure 1****) (Example 4).**

The same strategy was applied in cloning the human β1,4GalTII and the human β1,4GalTIII. **Example 4** describes using gene-specific primers to amplify the human β1,4-galactosyltransferase II and III genes by PCR and cloning it then into a vector.

### Expression of β1,4-Galactosyltransferase Activity in Pichia pastoris

A gene encoding β1,4GalT activity or a recombinant nucleic acid molecule encoding β1,4-galactosyltransferase activity, a gene fusion encoding β1,4GalT activity (e.g., **pXB53**) **(****Figure 1****)** or expression from a nucleic acid molecule encoding β1,4-galactosyltransferase (Genbank AH003575) is introduced and expressed in a Pichia pastoris host cell (e.g. *P. pastoris*) to produce galactosylated glycoproteins. Alternatively, by activation of a β-galactosyltransferase activity, a Pichia pastoris host cell is engineered to produce galactosylated glycoforms. A catalytically active β1,4-galactosyltransferase domain or a part thereof catalyzes the transfer of a galactose residue from UDP-galactose onto the terminal GlcNAc residue of an oligosaccharide acceptor substrate (e.g. GlcNAc₂Man₃GlcNAc₂) forming a β1,4Gal glycosidic linkage. Complex galactosylated N-glycans that are produced according to the present invention essentially lack fucose and sialic acid (e.g., Gal₂GlcNAc₂Man₃GlcNAc₂). Such a glycoprotein composition comprising complex galactosylated, afucosylated and asialylated N-glycans are useful as therapeutic agents.

The newly formed substrates are also preferable precursors in the formation of sialylated glycoproteins produced in a Pichia pastoris host. The present invention, thus provides a method for producing human-like glycoproteins wherein the glycoproteins are characterized as having a terminal galactose residues that are acceptor substrates for the transfer of sialic acid in a lower eukaryote.

### Combinatorial DNA library of β1,4-galactosyltransferase

In a related aspect of the invention, a combinatorial DNA library of β1,4-galactosyltransferase and yeast targeting sequence transmembrane domains is created and expressed in a Pichia pastoris host cell as described in WO02/00879.

Accordingly, a sub-library of hGalTI (e.g. Genbank Accession No. X55415) fused to a sub-library of targeting peptides of lengths: short, medium and long as described in WO 02/00879 is generated. The targeting peptide sub-library includes nucleic acid sequences encoding targeting signal peptides that result in localization of a protein to a particular location within the ER, Golgi, or trans Golgi network. These targeting peptides may be selected from the host organism to be engineered as well as from other related or unrelated organisms. Generally such sequences fall into three categories: (1) N-terminal sequences encoding a cytosolic tail (ct), a transmembrane domain (tmd) and part or all of a stem region (sr), which together or individually anchor proteins to the inner (lumenal) membrane of the Golgi; (2) retrieval signals which are generally found at the C-terminus such as the HDEL or KDEL tetrapeptide; and (3) membrane spanning regions from various proteins, e.g., nucleotide sugar transporters, which are known to localize in the Golgi.

The targeting peptides are indicated herein as short (s), medium (m) and long (1) relative to the parts of a type II membrane protein. The targeting peptide sequence indicated as short (s) corresponds to the transmembrane domain (tmd) of the membrane-bound protein. The targeting peptide sequence indicated as long (1) corresponds to the length of the transmembrane domain (tmd) and the stem region (sr). The targeting peptide sequence indicated as medium (m) corresponds to the transmembrane domain (tmd) and approximately half the length of the stem region (sr). The catalytic domain regions are indicated herein by the number of nucleotide deletion with respect to its wild-type glycosylation enzyme.

In one embodiment, the library was transformed into *P. pastoris* and the transformants were selected on minimal medium containing hygromycin. The activity of β1,4-galactosyltransferase I fused to various leader sequences (as described below) was analyzed via production of galactosylated N-glycans as a readout using MALDI-TOF MS in positive mode.

### □-Galactosyltransferase Fusion Constructs

A library of the isolated yeast targeting sequence transmembrane domains (consisting of 48 leader sequences (WO 02/00879)) was ligated into the *Not*I/*Asc*I sites on **pRCD260** located upstream of the *hGalTI* gene to create plasmids **pXB20-pXB67** (each plasmid carrying one leader sequence).

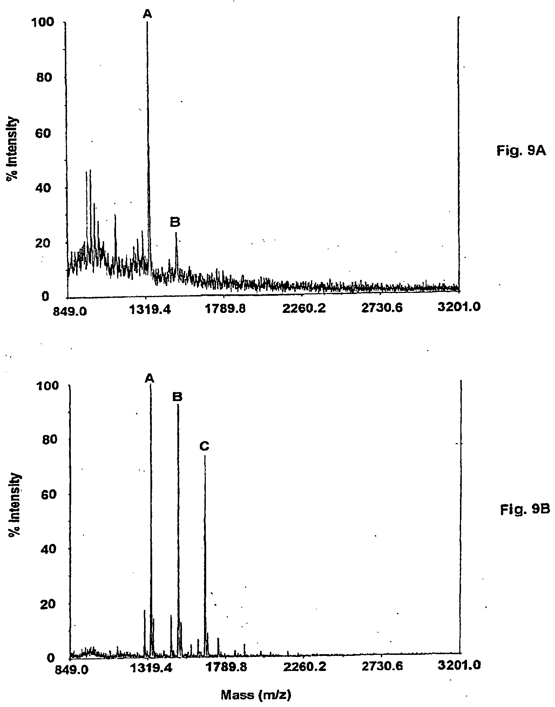

A representative example of a GalT fusion construct derived from a combinatorial DNA library of the invention is **pXB53 (****Figure 1****),** which is a truncated S. *cerevisiae* Mnn2(s) targeting peptide (1-108 nucleotides of *MNN2* from Genbank NP_009571) ligated in-frame to a 43 N-terminal amino acid deletion of a human β1,4-galactosyltransferase I (Genbank AH003575). The nomenclature used herein, thus, refers to the targeting peptide/catalytic domain region of a glycosylation enzyme as *S. cerevisiae* Mnn2(s)/hGalTI Δ43. The encoded fusion protein alone, however, is insufficient to produce N-glycans having predominantly galactosylated glycans as shown in **Figure 9A****.** Although a peak consistent with the mass of the N-glycan GalGlcNAc₂Man₃GlcNAc₂**[B]** is shown with the introduction of hGalTI in *P. pastoris* **YSH-44,** subsequent digest of the sample shows that this peak is recalcitrant to b-1,4-galactosidase **(Example 7).**

In addition, β-1,4-galactosyltransferase activity may be specific to a particular protein of interest Thus, it is to be further understood that not all targeting peptide/ galactosyltransferase catalytic domain fusion constructs function equally as well to produce the proper glycosylation on a glycoprotein of interest. Accordingly, a protein of interest may be introduced into a host cell transformed with a combinatorial DNA library to identify one or more fusion constructs which express a galactosyltransferase activity optimal for the protein of interest. One skilled in the art will be able to produce and select optimal fusion construct(s) using the combinatorial DNA library approach described herein.

It is apparent, moreover, that other such fusion constructs exhibiting localized active galactosyltransferase catalytic domains (or more generally, domains of any enzyme) may be made using techniques described herein. It will be a matter of routine experimentation for one skilled in the art to make and use the combinatorial DNA library of the present invention to optimize, for example, Gal₂GlcNAc₂Man₃GlcNAc₂ production from a library of fusion constructs in a particular expression vector introduced into a particular host cell.

### Production of Galactosylated N-glycans in Genetically Altered P. pastoris

The human-like galactosylated glycoproteins produced according to the method of present invention include GalGlcNAcMan₃GlcNAc₂, GalGlcNAc₂Man₃GlcNAc₂, Gal₂GlcNAc₂Man3GlcNAc₂, GalGlcNAc₃Man₃GlcNAc₂, Gal₂GlcNAc₃Man₃GlcNAc₂, Gal₃GlcNAc₃Man₃GlcNAc₂, GalGlcNAc₄Man₃GlcNAc₂, Gal₂GlcNAc₄Man₃GlcNAc₂, Gal₃ GlcNAc₄Man₃GlcNAc₂, Gal₄GlcNAc₄Man₃GlcNAc₂, GalGlcNAcMan₅GlcNAc₂, GalGlcNAc₂Man₅GlcNAc₂, Gal₂GlcNAc₂Man₅GlcNAc₂, GalGlcNAcMan₅GlcNAc₂, GalGlcNAc₂Man₅GlcNAc₂, Gal₂GlcNAc₂Man₅GlcNAc₂, GalGlcNAc₃Man₅GlcNAc₂, Gal₂GlcNAc₃Man₅GlcNAc₂ and Gal₃GlcNAc₃Man₅GlcNAc₂.

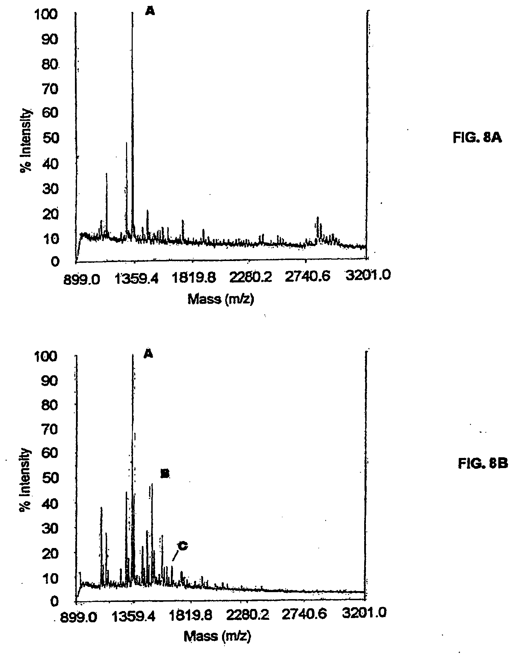

The plasmid **pXB53** comprising *MNN*2(s)1*hGalTI* was transformed in *P*. *pastoris* RDP30-10, host producing GlcNAc₂Man₃GlcNAc₂ **(Example 5).** The catalytically active β-galactosyltransferase domain catalyzes the transfer of a galactose residue onto an acceptor substrate having a terminal GlcNAc residue (e.g. GlcNAc₂Man₃GlcNAc₂) to produce a galactosylated glycoform. Using MALDI-TOF MS, the N-glycans released from the reporter protein from *P. pastoris* **RDP37** showed a peak at 1505 m/z, which corresponds to the mass of GalGlcNAc₂Man₃GlcNAc₂ **[B] (****Figure 8B****).** Transfer of a galactose residue by the fusion construct comprising human *S. cerevisiae* Mnn2(s)/β1,4-galactosyltransferase onto the acceptor substrate GlcNAc₂Man₃GlcNAc₂ producing GalGlcNAc₂Man₃GlcNAc₂ was shown to be about 10-20%. **Figure 8B** shows the corresponding mass of Gal₂GlcNAc₂Man₃GlcNAc₂ at 1662 m/z [C]. Transfer of two galactose residues onto the GlcNAc₂Man₃GlcNAc₂ substrate producing GalGlcNAc₂Man₃GlcNAc₂ was, therefore, evident. Accordingly, the host disclosed exhibits at least 10 mole % of galactosyl moiety on a human-like N-glycan.

It is recognized that GalTI is capable of transferring a second galactose residue onto an acceptor substrate having a second terminal GlcNAc residue in a host producing complex (e.g., biantennary) glycans. For example, a Mnn2(s)/hGalTI fusion, which is capable of capping the terminal GlcNAc with a galactose residue on the GlcNAcβ1,2 Manα1,3 arm of the glycan GlcNAc₂Man₃GlcNAc₂, can form at least one additional β glycosidic linkage on the other arm exposed with a terminal GlcNAc residue (e.g., GlcNAcβ1,2 Manα1,6), thereby, producing a galactosylated glycoform without the expression of subsequent galactosyltransferases. **Figure 12** displays the MALDI-TOF MS exhibiting a peak at 1663 m/z [C], which corresponds to Gal₂GlcNAc₂Man₃GlcNAc₂. The results show that substrate specificity for a particular β1,4-GalT is not limited to catalyzing the transfer of galactose residues on only the designated arm of the glycan, hence, a second galactosyltransferase may be obviated. Accordingly, in one embodiment of the present invention, expression of only one β1,4-GalT activity is capable of producing mono-, bi-, tri-or tetra-antennary galactosylated glycoforms. In such an embodiment, all glycosidic linkages between the galactose residue and the GlcNAc residue on the glycan would be the same. For instance, expression of hGalT1 in a host producing biantennary glycans would exhibit two terminal Galβ1,4 GlcNAcβ1,2 linkages.

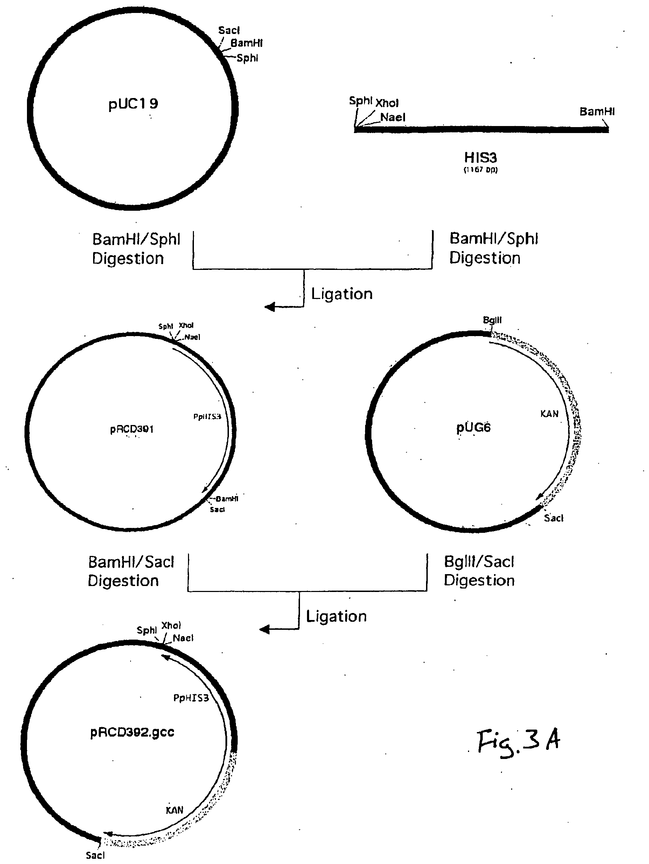

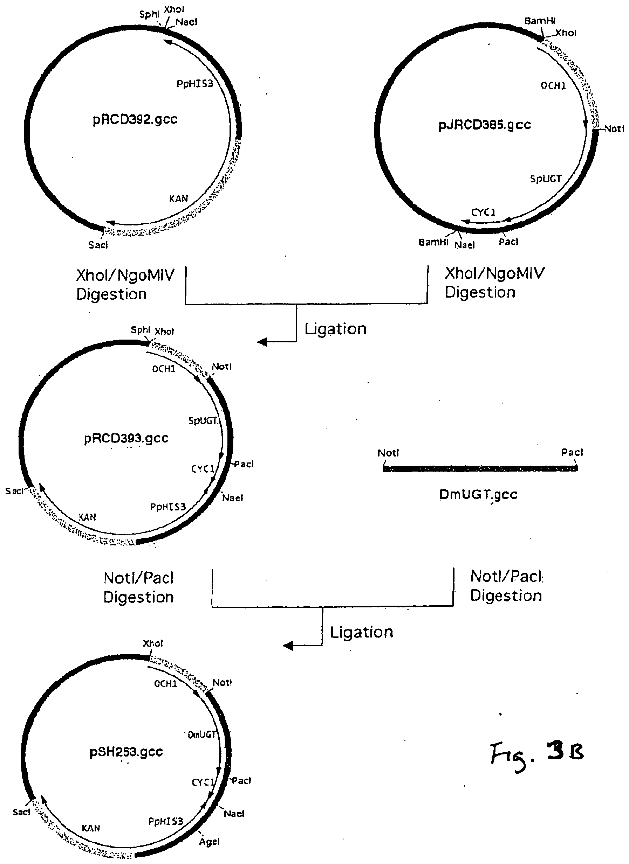

Alternatively, a different β-galactosyltransferase activity (e.g. hGalT II) or a catalytically active part thereof is expressed in a Pichia pastoris host cell. In one embodiment, a vector **pRCD440** comprising the *MNN2*(s)*lhGalTII* and *SpGALE* and the vector **pSH263 (****Figure 3B****)** comprising *Dm*UGT was transformed into a host *P. pastoris* YSH-44 **(****Figure 12B****).** The N-glycan analysis of the transformants showed the production of the Gal₂ GlcNAc₂ Man₃ GlcNAc₂ glycoform indicating that hGalTII transferred both galactose residues onto the acceptor substrate **(****Figure 12B****).** Bi galactosylated structures (Gal₂GlcNAc₂Man₃GlcNAc) are predominant. Transfer of galactosyl moiety with respect to % neutral glycans was approximately 75%.

A sequence encoding the hGalTIII is expressed in a Pichia pastoris host cell. **Figure 12C** shows galactose transfer of the combined mono-and bi-galactosylated glycans to be about 50 to 60 mole %. Comparison of hGalTI, hGalTII and hGalTIII show various level of galactose transfer **(****Figure 12A-C****).** The N-glycan profile from *P. pastoris* **RDP71 (****Figure 12A****)** shows that the transfer of galactose residue by the expression of hGalTI is optimal (about 80 mole %) for the K3 reporter protein.

### Expression of Additional β1.4-Galactosyltransferases

In another embodiment, hGalTI and hGalTII are sequentially localized and expressed using medial and late Golgi targeting sequences, respectively. For example, I the hGalT1 is localized in the medial Golgi whereas the hGalTII is localized in the late Golgi. Alternatively, to avoid substrate competition with Mannosidase II, in another embodiment, late Golgi leaders are used for β-galactosyltransferases.

Expression of galactosylkansferase activities usually generates both mono-and hi-i galactosylated glycans. Multiple antennary galactosylated glycoforms in addition to mono-galactosylated glycoforms are generally produced in host cells expressing galac tosyltransferase activity.

It will be a matter of routine experimentation for a skilled artisan to optimize galactosyltransferase activity or expression of the gene encoding the protein by using-various promoters and various expression vectors in a recombinant host cell.

### Tailored Galactosylated GlYcosidic Linkages in the Production of N-Glycans

In another feature production of multiple antennary galactosylated glycoproteins using different GalTs result in different β-glycosidic linkages. In one embodiment, desired β-glycosidic linkages of preference are generated in a lower eukaryotic host cell. For example, any one of the β1,4GalT family (e.g., hGalTI, hGalT2, hGalT3, hGalT4, hGalT5, hGalT6, hGalT7, bGalTI, XlGalT, CeGalTII) is expressed for the production of galactosylated glycoproteins characterized as having a β1,4Gal glycosidic linkage.

Alternatively, by expressing other galactosyltransferases, such as, β1,3GalT or β1,6GalT activities (enzyme, homo logs, variants, derivatives and catalytically active fragment thereof) in a Pichia pastoris host cell a galactose residue is transferred onto an intermediate oligosaccharide acceptor substrate forming a specifically desired βGal-glycosidic linkage. Various terminal galactose linkages (e.g., β13, β1,4; or β1,6) are formed as a result of the expression of a desire β-galactosyltransferase activity.

### GalNAcT Expression

GalNAc capped glycans have been observed on specific proteins in human. In another aspect a gene encoding GalNAc Transferase (GalNAcT) is expressed in a Pichia pastoris host cell, which transfers GalNAc residues onto a substrate having a terminal GlcNAc residue. In one embodiment, a gene encoding C. elegans GalNAcT (Genbank AN NP 490872) catalyzes the transfer of a GalNAc residue onto a substrate having a terminal GlcNAc residue extending the I oligosaccharide branch of the glycans produced in a host cell.

### Enhanced Galactosyl Transfer

The *hGalTI* expression comparison as shown in **Figure 12** indicates that β-galacto syltransferase expression alone may not be sufficient in the formation of βGalglycosidic linkages on acceptor substrates in a lower eukaryote. The transfer of a galactose residue is enhanced by the addition of a heterologous gene encoding an epimerase or galactokinase, a galactose-1-phosphate uridyl transferase and/or a gene encoding a UGT. Sufficient quantity of galactosylated glycoforms (e.g., Gal₂GlcNAc₂Man₃GlcNAc₂) is desirable as therapeutic glycoprotein. Accordingly, it is a feature of the present invention to enhance galactosyl transfer onto glycans by additional expression of a transport activity and/or to elevate endogenous UDP-galactose levels. In one embodiment, an epimerase activity is introduced in a host cell to increase UDP galactose levels. In another embodiment, increased UDP-galactose level is mediated by galactokinase or a galactose-1-phosphate uridyl transferase activity. The present invention, therefore, discloses a method to enhance galactosyltransfer by introducing and expressing a β-galactosyltransferase activity in combination with either a UDP-Gal transport activity and/or by elevating endogenous UDP-galactose levels via an epimerase or galactokinase or galactose-1-phosphate uridyl transferase.

### Cloning and Expression of UDP-Galactose Transporter (UGT) in Pichia pastoris Hosts in the Production of Human-like Glycoproteins

Herein the specification, is also disclosed a method to introduce and express a gene encoding a UDP-galactose transporter in a Pichia pastoris cell for the production of human-like galactosylated glycoproteins.

### Cloning and Expression of S. pombe UDP-galactose transporter

Gene-specific primers were designed to complement the homologous regions of the *S. pombe* UDP-galactose transporter gene (Genbank AL022598) and PCR amplified from *S. pombe* genomic DNA (ATCC24843) eliminating a single intron. Primers RCD164 (SEQ ID NO:7) and RCD177 (SEQ ID NO:8) were used to amplify the 5' 96bp of the gene. Primers RCD176 (SEQ ID NO:9) and RCD165 (SEQ ID NO:10) were used to amplify the 3' 966bp. Primers RCD164 (SEQ ID NO:7) and RCD165 (SEQ ID NO: 10) were used to overlap the two amplified products into a single PCR fragment containing one contiguous ORF with *NotI* and *PacI* sites introduced at the ends. The PCR product was cloned into **pCR2.1 TA** (Invitrogen) and sequenced. The gene product was subcloned into plasmid **pJN335** containing the *P. pastoris* GAPDH promoter **(Example 2).**

Accordingly, in one embodiment, a plasmid **pRCD257** encoding the S. pombe UDP-galactose transporter (Genbank AB023425) is constructed and expressed in a host producing terminal GlcNAc residues *(P. pastoris* **RDP-27** (e.g. GlcNAcMan₃; GlcNAc₂)).

### Cloning and Expression of Various UDP-galactose transporters

In a preferred embodiment, the gene encoding the *D. melanogaster* UDP-galactose transporter is introduced and expressed in a lower eukaryotic host cell. The *D. melanogaster* UGT was PCR amplified from a D. melanogaster cDNA library (UC Berkeley Drosophila Genome Project, ovary-ZAP library GM) and cloned into the pCR2.1 PCR cloning vector and sequenced. Primers DmUGT-5' (SEQ ID NO:11) and DmUGT-3' (SEQ ID NO: 12) were used to amplify the gene introducing *NotI* and *PacI* sites. The NotI and PacI sites were used to subclone this gene fused downstream of the PpOCH1 promoter at the *NotI*/*PacI* sites in **pRCD393** creating **pSH263** (**Figure 3B**).

### Example 2 describes cloning of various other UDP galactose transporters.

**Figure 11** shows UDP-transporter activity in comparison for enhanced galactose transfer. As the best mode of the present invention, the UDP-galactose transporter isolated from *D. Melanogaster* is expressed in *P. pastoris.* The activity of the human GalTI gene fusion co-expressed with the *D. Melanogaster* UDP-galactose transporter (DmUGT) is shown in **Figure 11E****.** Surprisingly, host cells expressing the *D. Melanogaster* UGT produce predominantly galactosylated glycoforms, whereas, UGTs from *S. pombe* **(****Figure 11B****),** human I **(****Figure 11C****)** and human II **(****Figure 11D****)** showed less than optimal transfer. A significant increase in the production of a bi galactosylated, afucosylated and asialylated glycoform Gal₂GlcNAc₂Man₃GlcNAc₂ is produced. The uniform peak at 1664 m/z [C] corresponds to the mass of the glycan Gal GlcNAc₂Man₃GlcNAc₂. A host cell expressing the DmUGT exhibits at least 90 mole % galactose transfer in comparison to other UDP-galactose transporters.

### UDP-Galactose Transporter Polypeptides

The invention additionally provides various combination of transporter-transferase fusions expressed in a Pichia pastoris host cell. Accordingly, in one embodiment, the present invention discloses a Pichia pastoris host comprising a UDP-galactose transporter fused in-frame to a catalytically active β-galactosyltransferase domain. In another embodiment, the host cell producing human like glycoproteins comprises a UDP-galactose transporter isolated from *S. pombe* and *S. cerevisiae* Mnn2(s) targeting peptide fused in-frame to hGalTI catalytic domain.

### Expression of UDP-Galactose 4-Epimerase in Pichia pastoris Hosts in the Production of Human-like Glycoproteins

In another aspect a method is disclosed for producing a human-like glycoprotein in Pichia pastoris by expressing a β1,4-galactosyltransferase activity and at least a UDP-galactose 4-epimerase activity (enzyme, homo logs, variants, derivatives and catalytically active fragment thereof). The epimerase is an enzyme that catalyzes the interconversion of UDP-galactose and UDP-glucose. Using well known techniques in the art, gene-specific primers are designed to complement the homologous regions of an epimerase gene (e.g. *ScGAL10, SpGALE, hGALE*:) and PCR amplified **(Example 3).** In one embodiment, a gene encoding the *S. cerevisiae* GallO activity or a recombinant nucleic acid molecule encoding an epimerase or expression from a nucleic acid molecule encoding an epimerase activity is introduced and expressed in a Pichia pastoris host cell to produce human-like glycoproteins characterized as having a terminal β galactose residue. Alternatively, by activation of an epimerase activity, a host cell is engineered to produce increased levels of galactosylated gly co forms.

### Expression of UDP-galactose 4-epimerase in the Production of Complex N-glycans

In one embodiment, a gene encoding an epimerase activity is expressed to convert UDP-glucose to UDP-galactose, generating an increased level of UDP-galactose for galactosyltransfer in host cells. The expression of an epimerase activity in addition to a β-1,4-galactosyltransferase activity increases production of galactosylated N-glycans. **Figure 9B** shows a yeast strain producing complex glycans (e.g., *P. pastoris* **YSH-44**) transformed with a Mnn2(s)/hGalTI fusion in combination with **pRCD395**, a plasmid encoding ScGallO. The addition of the ScGallO epimerase increases the available UDP-galactose for galactose transfer. A peak at 1501 m/z **[B]** corresponds to the transfer of one galactose residue on the glycan GlcNAc₂Man₃GlcNAc₂ and a peak at 1663 m/z **[C]** corresponds to the transfer of two galactose residues on the glycan GlcNAc₂Man₃GlcNAc₂. Preferably, at least 60 mole % of galactose is transferred with respect to % total neutral glycans. Accordingly, in one embodiment, a β1,4-galactosyltransferase activity in combination with an epimerase activity is expressed in a host cell to produce galactosylated glycoproteins **(Example 7).**

### Expression of UDP-galactose 4-epimerase in the Production of Hybrid N-glycans

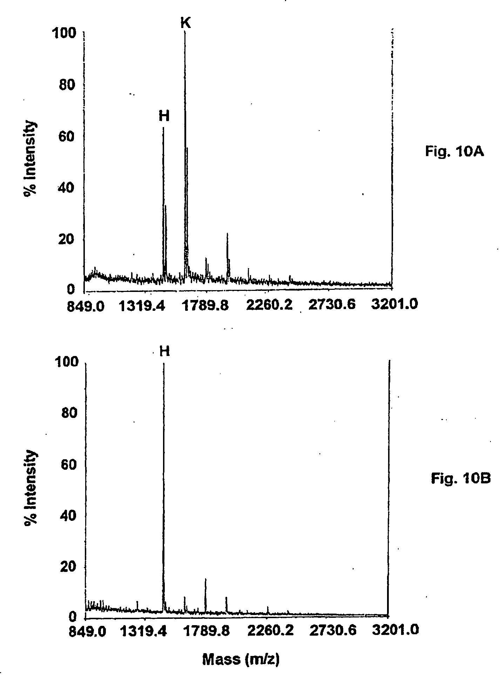

In another embodiment, the introduction and expression of *ScGAL10* increases galactose transfer on a hybrid glycoprotein in Pichia pastoris **(Example 6).** **Figure 10A** shows the *P. pastoris* strain **RDP39-6** expressing an Mnn2(m)/hGalTI fusion in combination with the ScGallO epimerase producing hybrid galactosylated N-glycans. The N-glycan analysis shows peak at 1622 m/z **[K],** which corresponds to the mass of the glycan GalGlcNAcMan₅GlcNAc₂ confirming transfer of one galactose residue, and a peak at 1460 m/z **[H],** which corresponds to the mass of the hybrid glycan GlcNAcMan₅GlcNAc₂. Subsequent β1,4-galactosidase digest confirms presence of a single galactose residue (Figure 10B). Preferably, at least 70 mole % of galactose transfer is detected with respect to % total neutral glycans.

Still other epimerases are expressed in a host cell to increase galactose transfer. **Example 3** describes construction of epimerase constructs and **Figure 13** shows the activity of various epimerases in the production of human-like N-glycans. The expression of *ScGallO* along with Mnn2(s) /hGalTI and *Dm*UGT in **Figure 13A** shows a predominant bi-galactosylated glycoform Gal₂GlcNAc₂Man₃GlcNAc₂. Similarly, the transformation of SpGalE, Mnn2(s) /hGalTI and the DmUGT in either order results in the production of the bi-galactosylated glycoform **(****Figure 13B and C** **).** The addition of hGalE has the same effect **(****Figure 13D****).** Preferably, the epimerase is selected from the group consisting of *S. cerevisiae* UDP-galactose 4-epimerase, *S. pombe* UDP-galactose 4-epimerase, E. coli UDP-galactose 4-epimerase and *H. sapiens* UDP-galactose 4-epimerase. It is contemplated that other epimerases, without limitation, can be selected and expressed in the host cell as well.

### Nucleic acid sequences encoding SpGALE

The present invention additionally discloses isolated nucleic acid molecules that include the GALE gene from *S. pombe* and variants thereof. The full-length nucleic acid sequence for this gene, which encodes the enzyme UDP-galactose 4-epimerase, has already been sequenced and identified as set forth in Genbank NC_003423.
Primers used to amplify *SpGALE* from *S. pombe* genomic DNA revealed a 175bp intron, which was eliminated (Example 3). Included within the cloned genomic sequence is a coding sequence for *S. pombe* UDP-galactose 4-epimerase. The encoded amino acid sequence is also set forth as SEQ ID NO: 13. The *Sp GALE* gene is particularly useful in generating a sufficient pool of UDP-galactose for galactose transfer onto N-glycans in a host cell. Expression of the *SpGALE* gene in Pichia pastoris provides increased and efficient galactose transfer in N-linked oligosaccharide synthesis.

In one embodiment, the invention discloses an isolated nucleic acid molecule having a nucleic acid sequence comprising or consisting of a *SpGALE* coding sequence as set forth in SEQ ID NO: 14, and homologs, variants and derivatives thereof. In a further embodiment, the invention discloses a nucleic acid molecule comprising or consisting; of a sequence which is a variant of the *SpGALE* gene having at least 53% identity to the wild-type gene. The nucleic acid sequence can preferably have at least 70%, 75% or 80% identity to the wild-type gene. Even more preferably, the nucleic acid sequence can have 85%, 90%, 95%, 98%, 99%, 99.9% or even higher identity to the wild-type gene.

In another embodiment, the nucleic acid molecule of the invention encodes a polypeptide having the amino acid sequence of SEQ ID NO:13. Also disclosed is a nucleic acid molecule encoding a polypeptide sequence that is at least 60% identical to SEQ ID NO: 13. Typically the nucleic acid molecule of the invention encodes a polypeptide sequence of at least 70%, 75% or 80% identity to SEQ ID NO:13.
Preferably, the encoded polypeptide is 85%, 90% or 95% identical to SEQ ID NO: 13, and the identity can even more preferably be 98%, 99%, 99.9% or even higher.

### Epimerase Conserved Regions involved in the interconversion of UDP-Glucose and UDP-Galactose for the Production of Galactosylated Glycoproteins

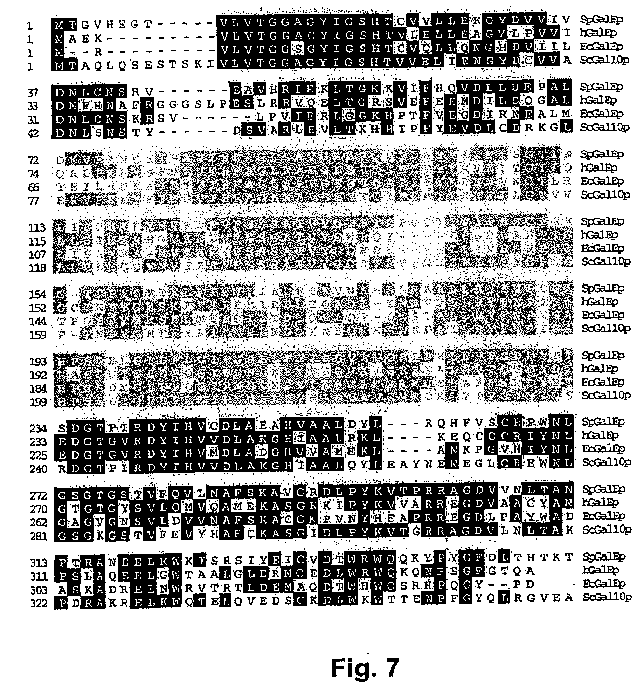

Sequence alignment of epimerases from *S. pombe*, human, E. coli and the first 362 amino acid residues of *S. cerevisiae* shows highly conserved regions indicating the presence of several motifs and a potential active site **(****Figure 7****) (Example 11).** In one embodiment, the invention discloses a polypeptide comprising the amino acid sequence of SEQ ID NO: 13, which has a potential UDP-galactose or UDP-glucose binding motif at
9-VLVTGGXGYIGSHT-22 (SEQ ID NO:48),
83-VIHFAGLKAVGESXQXPLXYY-103 (SEQ ID NO:49),
127-FSSSATVYGX-136 (SEQ ID NO:50).
184-LRYFNPXGAHXSGXXC: EDPXGIPNNLXPYXXQVAXGRX 221 (SEQ ID NO:51), or
224-LXXFGXDYXXXDGTXXRDYIHVXDLAXXHXXAX-256 (SEQ ID NO:52).

In another preferred embodiment, the amino acid residue at position 15 of the first sequence is selected from the group consisting of S and A.

In another preferred embodiment, the amino acid residue at position 96 of the second sequence is selected from the group consisting of T and V.

In another preferred embodiment, the amino acid residue at position 98 of the second sequence is selected from the group consisting of V, K and I.

In another preferred embodiment, the amino acid residue at position 101 of the second sequence is selected from the group consisting of S, D, E and R.

In another preferred embodiment, the amino acid residue at position 136 of the third sequence is selected from the group consisting of D and N.

In another preferred embodiment, the amino acid residue at position 190 of the fourth sequence is selected from the group consisting of G, T, V and I.

In another preferred embodiment, the amino acid residue at position 194 of the fourth sequence is selected from the group consisting of P and A.

In another preferred embodiment, the amino acid residue at position 197 of the fourth sequence is selected from the group consisting of E, C, D and L.

In another preferred embodiment, the amino acid residue at position 198 of the fourth sequence is selected from the group consisting of L, I and M.

In another preferred embodiment, the amino acid residue at position 203 of the fourth sequence is selected from the group consisting of L and Q.

In another preferred embodiment, the amino acid residue at position 210 of the fourth sequence is selected from the group consisting of L and M.

In another preferred embodiment, the amino acid residue at position 213 of the fourth sequence is selected from the group consisting of I, V and M.

In another preferred embodiment, the amino acid residue at position 214 of the fourth sequence is selected from the group consisting of A and S.

In another preferred embodiment, the amino acid residue at position 218 of the fourth sequence is selected from the group consisting of V and I.

In another preferred embodiment, the amino acid residue at position 221 of the fourth sequence is selected from the group consisting of L and R.

In another preferred embodiment, the amino acid residue at position 225 of the fifth sequence is selected from the group consisting of N, A and Y.

In another preferred embodiment, the amino acid residue at position 226 of the fifth sequence is selected from the group consisting of V and I.

In another preferred embodiment, the amino acid residue at position 229 of the fifth sequence is selected from the group consisting of D and N.

In another preferred embodiment, the amino acid residue at position 232 of the fifth sequence is selected from the group consisting of P and D.

In another preferred embodiment, the amino acid residue at position 233 of the fifth sequence is selected from the group consisting of T and S.

In another preferred embodiment, the amino acid residue at position 234 of the fifth sequence is selected from the group consisting of S, E and R.

In another preferred embodiment, the amino acid residue at position 238 of the fifth sequence is selected from the group consisting of P and G.

In another preferred embodiment, the amino acid residue at position 239 of the fifth sequence is selected from the group consisting of I and V.

In another preferred embodiment, the amino acid residue at position 246 of the fifth sequence is selected from the group consisting of C, V and M.

In another preferred embodiment, the amino acid residue at position 250 of the fifth sequence is selected from the group consisting of E, K and D.

In another preferred embodiment, the amino acid residue at position 251 of the fifth sequence is selected from the group consisting of A and G.

In another preferred embodiment, the amino acid residue at position 253 of the fifth I sequence is selected from the group consisting of V and I.

In another preferred embodiment, the amino acid residue at position 254 of the fifth sequence is selected from the group consisting of A and V.

In another preferred embodiment, the amino acid residue at position 256 of the fifth sequence is selected from the group consisting of L and M.

### Isolated Polypeptides

According to another aspect of the invention, isolated polypeptides (including muteins, allelic variants, fragments, derivatives, and analogs) encoded by the nucleic acid molecules of the invention are disclosed. In one embodiment, the isolated polypeptide comprises the polypeptide sequence corresponding to SEQ ID NO:13. In an alternative embodiment of the invention, the isolated polypeptide comprises a polypeptide sequence at least 60% identical to SEQ ID NO: 13. Preferably the isolated i polypeptide of the invention has at least 70%, 75% or 80% identity to SEQ ID NO: 13.More preferably, the identity is 85%, 90% or 95%, but the identity to SEQ ID NO: 13 can be 98%, 99%, 99.9% or even higher.

According to other embodiments of the invention, isolated polypeptides comprising a fragment of the above-described polypeptide sequences are disclosed. These fragments preferably include at least 20 contiguous amino acids, more preferably at least 25, 30, 35, 40, 45, 50, 60, 70, 80, 90, 100 or even more contiguous amino acids.
The polypeptides of the present invention also disclose fusions between the above described polypeptide sequences and heterologous polypeptides. The heterologous sequences can, for example, include heterologous sequences designed to facilitate purification and/or visualization of recombinantly-expressed proteins. Other non-limiting examples of protein fusions include those that permit display of the encoded protein on the surface of a phage or a cell, fusions to intrinsically fluorescent proteins, such as green fluorescent protein (GFP), and fusions to the IgG Fc region.

### UDP-Galactose 4-Epimerase / β1,4-Galactosyltransferase Fusion Polypeptides

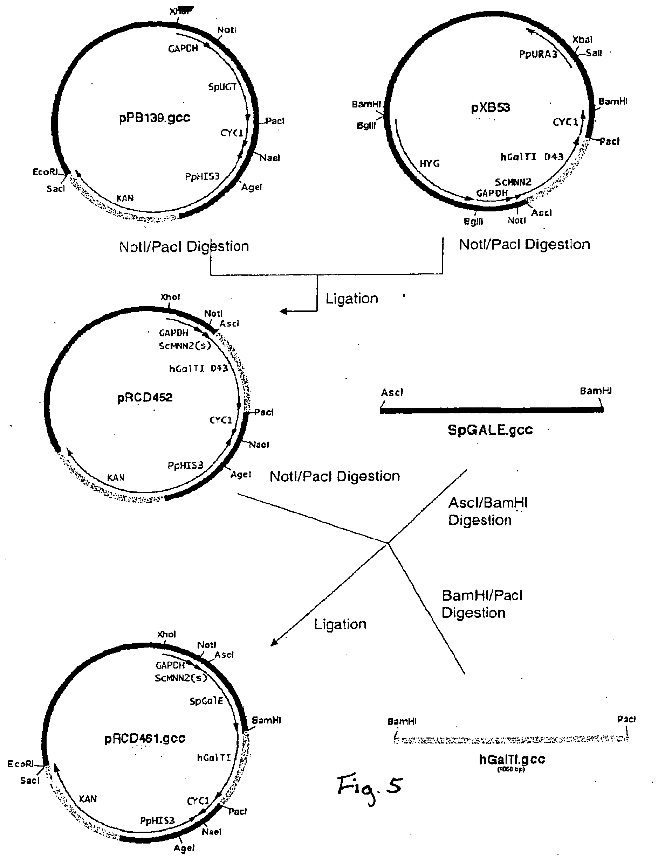

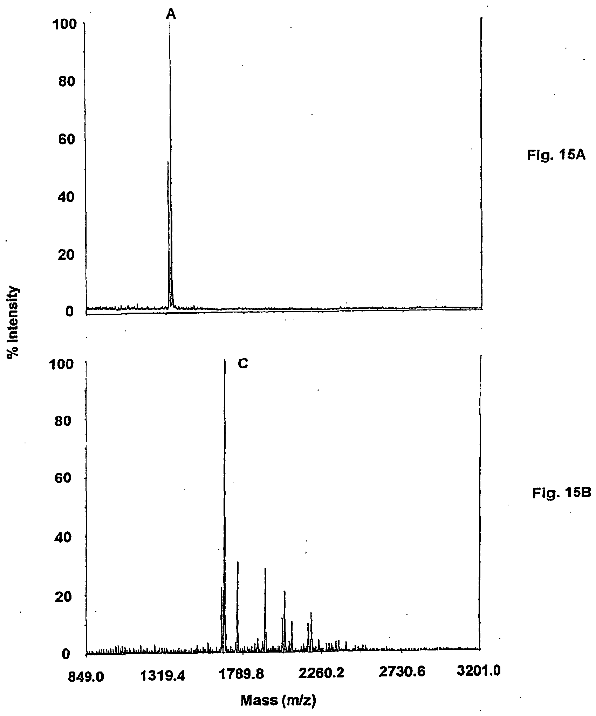

In a further aspect of the invention, a gene fusion encoding a polypeptide comprising epimerase and galactosyltransferase activities is generated. In one embodiment, a fusion polypeptide comprising a UDP-galactose 4-epimerase and β1,4-GalTI is generated and introduced in a host cell. In a more preferred embodiment, the fusion polypeptide further comprises a leader sequence. For example, a library of leader sequences encoding targeting peptides is ligated in-frame to SpGalE/hGalTI fusion. In an even more preferred embodiment, the fusion polypeptide comprises ScMnn2(s) leader, SpGalE epimerase, and hGalTI. The fusion polypeptide is inserted into a yeast integration plasmid comprising a *HYG* marker. An example of an epimerase-galactosyltransferase integration plasmid designated **pRCD461** is shown in **Figure 5** **(Example 8).** The epimerase-galactosyltransferase fusion transformant produces approximately 70% galactosylated human-like glycoprotein Gal₂GlcNAc₂Man₃GlcNAc₂ **(****Figure 15B****).**

### β1,4-Galactosyltransferase, UDP-Galactose 4-Epimerase; UDP-Galactose Transporter Polypeptides

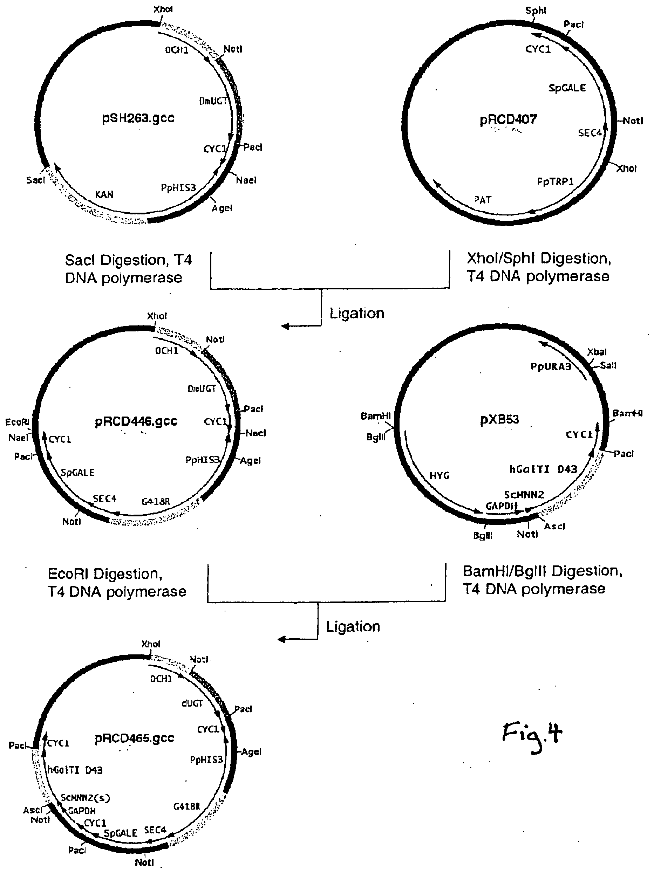

A single construct encoding polypeptides comprising a β-galaclosyltransferase, epimerase and UDP-galactose transporter activities is generated. In one embodiment, a plasmid comprising human β1,4GalT, Sp GalE and *Dm*UGT ('triple') is constructed **(Example 9).** In a preferred embodiment, the transferase polypeptide further comprises a leader sequence, for example, *Sc* Mnn2(s) ligated in-frame to *hGalTI.* All three polypeptides are inserted into a yeast integration plasmid containing a KAN^{R} marker, preferably with their own promoters and terminators. An example of this 'triple' integration plasmid, designated **pRCD465,** is shown in **Figure 4****.** In one embodiment, the 'triple' integration plasmid comprising the fusion polypeptide is introduced and expressed in a host cell producing terminal GlcNAc residues. *P. pastoris* **YSH-44** was transformed with the 'triple' integration plasmid and was denoted **RDP80.**

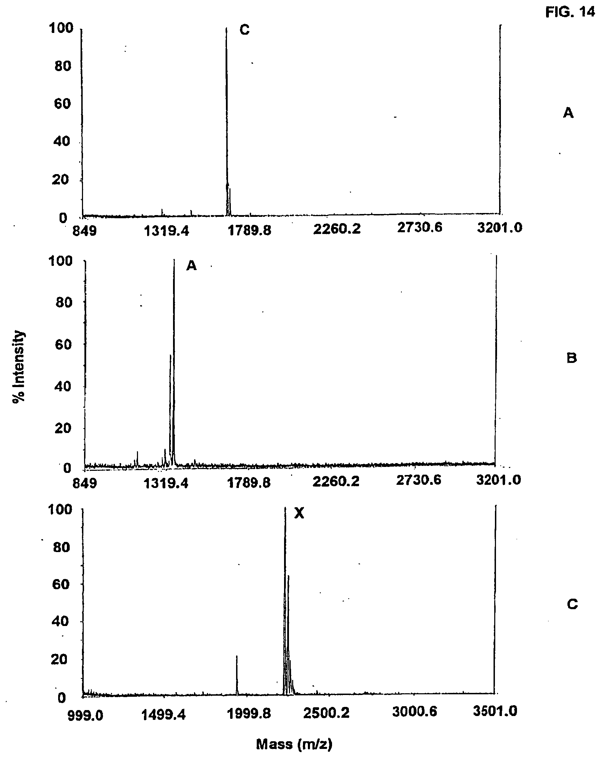

To evaluate whether the N-glycans produced in strain **RDP80** are the predicted Gal₂GlcNAc₂Man₃GlcNAc₂ species, purified K3 secreted from **RDP80** was incubated with sialyltransferase *in vitro* in the presence of CMP-NANA and the resulting N-glycans were released. The MALDI-TOF MS analysis of the N-glycans displayed a predominant peak at 2227 m/z [X], which corresponds to the mass of the complex, terminally sialylated N-glycan NANA₂ Gal₂GlcNAc₂Man₃GlcNAc₂ **(****Figure 14C****).**

### Increased Galactosylated N-glycans Production in Genetically Altered Yeast Cells

Methods to produce human-like N-glycans in yeast and fungal hosts are provided in WO00200879A3 and WO 03056914A1. The skilled artisan recognizes that routine modifications of the procedures disclosed herein in combination with the above methods may provide improved results in the production of the glycoprotein of interest.

In accordance with the methods of the present invention, *P. pastoris* transformed with at least a β-galactosyltransferase fusion construct **pXB53 (Example 4) (****Figure 12****)** produces complex galactosylated glycans in a detectable moiety. At least 10% of galactose residue is transferred onto a glycoprotein in a host cell. In another embodiment, at least 40% of galactose residue is transferred onto a glycoprotein in a host cell. The expression of an epimerase also increases the level of galactose transfer **(****Figure 13****).** Preferably, at least 60% of galactose residue is transferred onto a glycoprotein in a host cell. The expression of another heterologous glycosylation enzyme, such as UGT, further enhances the cellular production of the desired galactosylated glycoproteins. Surprisingly, expression of one such transporter, the DmUGT increases galactose transfer dramatically **(****Figure 11****).** In the best mode of the embodiment, a host cell transformed with the *DmUGT* shows at least 90% or higher galactose transfer.

Preferably, the temperature of the yeast host cell is kept at 37°C to match the temperature optimum of the enzyme.

Additionally, the method also includes isolating these glycoproteins.

### Expression of UDPase Activity

As described in WO 02/00879, in humans, nucleotide sugar precursors (e.g. UDP-N-acetylglucosamine, UDP-N-acetylgalactosamine, CMP-N-acetylneuraminic acid, UDP-galactose, etc.) are generally synthesized in the cytosol and transported into the Golgi, where they are attached to the core oligosaccharide by glycosyltransferases. To replicate this process in lower eukaryotes, sugar nucleoside specific transporters have to be expressed in the Golgi to ensure adequate levels of nucleoside sugar precursors (Sommers, 1981; Sommers, 1982; Perez, 1987). A side product of the transfer of sugars onto N-glycans is either a nucleoside diphosphate or monophosphate. While monophosphates can be directly exported in exchange for nucleoside triphosphate sugars by an antiport mechanism, diphospho nucleosides (e.g. GDP) have to be cleaved by phosphatases (e.g. GDPase) to yield nucleoside monophosphates and inorganic phosphate prior to being exported. This reaction appears to be important for efficient glycosylation, as GDPase from *S*. *cerevisiae* has been found to be necessary for mannosylation. However, the enzyme only has 10% of the activity towards UDP (Berninsone, 1994). Lower eukaryotes often do not have UDP specific diphosphatase activity in the Golgi since they do not utilize UDP-sugar precursors for glycoprotein synthesis in the Golgi.

Engineered yeast strains contain multiple transferase enzymes that utilize UDP-GlcNAc or UDP-galactose as a substrate. This requires the engineering of suitable substrate pools in the yeast Golgi, which in most species does not contain these substrates. However, the endproducts of a transferase reaction utilizing UDP-GlcNAc or UDP-galactose include free UDP. This UDP acts as a potent inhibitor of most transferases that utilize these sugar nucleotides. S. *cerevisiae* expresses two Golgi proteins with nucleoside diphosphatase activity. One, *ScGDA1*, is highly specific for GDP (Abeijon et al, 1993). The second, *ScYND1,* is an apyrase and thus capable of hydrolyzing both nucleoside tri- as well as di-phosphates and is equally specific for ADP/ ATP, GDP/GTP and UDP/UTP (Gao et al, 1999). However, because of the lack of UDP conjugated sugars in the wild-type Golgi and the concomitant lack of transferase enzymes producing UDP as an end product, the possible elevated accumulation of UDP in engineered yeast strain is a significant concern.

Because transfer of galactose residues from the cytosol to the Golgi can be hampered by the lack of UDPase, genetic manipulation to express UDPase may be required for efficient galactose transfer in a lower eukaryotic host cell. Accordingly, in another aspect of the present invention, a method is provided to express, preferably overexpress, a gene encoding for the UDPase. It is contemplated that overexpression of a gene encoding for the UDPase activity increases the availability of the sugar nucleotide UDP-galactose required for galactose transfer onto the acceptor substrates in the Golgi. To raise the level of UDPase activity in the Golgi of a yeast, several possibilities exist. In one embodiment, a gene encoding UDPase activity, e.g., *ScGDA1* (NP_010872) is overexpressed, which has some (about 10%) activity towards UDP. In another embodiment, a gene encoding nucleoside diphosphatase activity, e.g., *ScYND1* (NP_010920) is overexpressed, which has a higher activity towards UDP compared to GDP, though is not specialized for nucleotide diphosphates. Furthermore, in another embodiment, to achieve the goal of higher UPDase activity in *P. pastoris,* the *S. cerevisiae GDA1* or *YND1* is expressed or the *P. pastoris* homologs of these genes are overexpressed, which are readily identifiable via BLAST homology searches.

Additionally, organisms that utilize these sugar nucleotides are able to convert them to UMP via the action of a nucleotide diphosphatase specific for UDP. An example is the human uridine diphosphatase (UDPase) identified by Wang and Guidotti (AF016032). However, this protein contains two putative transmembrane domains; one at the C-terminus and one at the N-terminus. Accordingly, localization of this protein in the yeast Golgi thus requires fusing the catalytic domain of this protein with a yeast targeting domain.

Other yeasts including *K. lactis* and *S. pombe* utilize UDP-sugars in their Golgi to add GlcNAc and galactose, respectively, to their N-glycans. Both *K. lactis* and *S. pombe* express homologs of *ScGDA1*, designated *KIGDA1* (Lopez-Avalos et al, 2001; CAC21576) and *Spgda1* (D'Alessio et al, 2003; NP_593447), respectively, which also have UDPase activity. In case UDP accumulates in engineered yeast strains and proves to be detrimental to the engineered transferases, expression of any or more of these proteins serves to boost UDPase activity to acceptable levels.

### Binding affinity to asialoglycoprotein receptors (ASGR)

Another feature of the invention provides less binding affinity to ASGR , which are known to clear asialylated glycoproteins and reduce half-life of a therapeutic protein in the circulatory system. Previous work has shown that glycans having biantennary structures are cleared out less rapidly than glycans having tri or tetra-antennary structures (Stockert, Physiol Rev. 1995 Jul;75(3):591-609). In one aspect of the present invention provides glycans on the protein of interest having a single glycoform (e.g., bi-antennary structures) characterized as having terminal galactose residues. Such bi-antennary structures are not readily produced in mammalian cells because of other GnTs that catalyze tri- and tetra-antennary branching reactions. By capping the substrates having terminal GlcNAc residues with galactose residues, other GnTs (e.g. GnT IV, GnT V) are not present to catalyze the transfer of GlcNAcs onto the galactosylated substrates. Accordingly, the present invention provides methods for producing asialylated glycoproteins having less binding affinity to ASGR in comparison to those glycoproteins produced in mammalian hosts. In a more preferred embodiment, the asialylated glycoprotein is characterized by its increased circulatory half-life and bioactivity *in vivo* in comparison to heterogeneous glycoproteins produced in mammals.

### Integration Sites

It is preferable to integrate the nucleic acids encoding the UGT, epimerase and β1,4GalT in a locus that is responsible for mannosyltransferases such as 1,3 mannosyl-transferases (e.g. *MNN1* in *S. cerevisiae*) (Graham, 1991), 1,2 mannosyltransferases (e.g. *KTR*/*KRE*family from *S. cerevisiae*), 1,6 mannosyltransferases (*OCH1* from *S. cerevisiae* or *P. pastoris*)*,* mannosylphosphate transferases and their regulators (*MNN4, PNO1* and *MNN6* from S. *cerevisiae*), vacuolar proteinase A (*PEP4*), vacuolar protease B (*PRB1*) GPI-anchored aspartic protease *(YPS1)* and additional enzymes that are involved in aberrant, immunogenic, i.e. non-human glycosylation reactions.

The mutants with the disrupted locus give rise to a viable phenotype with reduced enzyme activity or eliminated enzyme activity completely. Preferably, the gene locus encoding the initiating α-1,6 mannosyltransferase activity is a prime target for the initial integration of genes encoding glycosyltransferase activity. In a similar manner, one can choose a range of other chromosomal integration sites that, based on a gene disruption event in that locus, are expected to: (1) improve the cell's ability to glycosylate in a more human-like fashion, (2) improve the cell's ability to secrete proteins, (3) reduce proteolysis of foreign proteins and (4) improve other characteristics of the process that facilitate purification or the fermentation process itself.

In an especially preferred embodiment, library DNA is integrated into the site of an undesired gene in a host chromosome, effecting the disruption or deletion of the gene. For example, integration into the sites of the *OCH1, MNN1,* or *MNN4* genes allows the expression of the desired library DNA while preventing the expression of enzymes involved in yeast hypermannosylation of glycoproteins. In other embodiments, library DNA may be introduced into the host via a nucleic acid molecule, plasmid, vector (e.g., viral or retroviral vector), chromosome, and may be introduced as an autonomous nucleic acid molecule or by homologous or random integration into the host genome. In any case, it is generally desirable to include with each library DNA construct at least one selectable marker gene to allow ready selection of host organisms that have been stably transformed. Recyclable marker genes such as *URA5* (Yeast. 2003 Nov;20(15):1279-90.), which can be selected for or against, are especially suitable.

### Generating Additional Sequence Diversity

The method of this embodiment is most effective when a nucleic acid, e.g., a DNA library transformed into the host contains a large diversity of sequences, thereby increasing the probability that at least one transformant will exhibit the desired phenotype. Single amino acid mutations, for example, may drastically alter the activity of glycoprotein processing enzymes (Romero et al., 2000). Accordingly, prior to transformation, a DNA library or a constituent sub-library may be subjected to one or more techniques to generate additional sequence diversity. For example, one or more rounds of gene shuffling, error prone PCR, *in vitro* mutagenesis or other methods for generating sequence diversity, may be performed to obtain a larger diversity of sequences within the pool of fusion constructs.

### Cordon Optimization

It is also contemplated that the nucleic acids of the present invention may be codon optimized resulting in one or more changes in the primary amino acid sequence, such as a conservative amino acid substitution, addition, deletion or combination thereof.

### Expression Control Sequences

In addition to the open reading frame sequences described above, it is generally preferable to provide each library construct with expression control sequences, such as promoters, transcription terminators, enhancers, ribosome binding sites, and other functional sequences as may be necessary to ensure effective transcription and translation of the fusion proteins upon transformation of fusion constructs into the host organism.

Suitable vector components, e.g., selectable markers, expression control sequences (e.g., promoter, enhancers, terminators and the like) and, optionally, sequences required for autonomous replication in a host cell, are selected as a function of which particular host cell is chosen. Selection criteria for suitable vector components for use in a particular mammalian or a lower eukaryotic host cell are routine.

Where the host is *Pichia pastoris*, suitable promoters include, for example, the *AOX1, AOX2,* GAPDH, *OCH1, SEC4,* D2 and *P40* promoters.

### Selectable Markers

It is also preferable to provide each construct with at least one selectable marker, such as a gene to impart drug resistance or to complement a host metabolic lesion. The presence of the marker is useful in the subsequent selection of transformants; for example, in yeast the *URA5, URA3, HIS4, SUC2, G418, BLA,* or *SH BLE* genes may be used. A multitude of selectable markers are known and available for use in yeast, fungi, plant, insect, mammalian and other eukaryotic host cells.

### Transformation

In yeast, any convenient method of DNA transfer may be used, such as electroporation, the lithium chloride method, or the spheroplast method. In filamentous fungi and plant cells, conventional methods include particle bombardment, electroporation and agrobacterium mediated transformation. To produce a stable strain suitable for high-density culture (e.g., fermentation in yeast), it is desirable to integrate the fusion constructs into the host chromosome. In a preferred embodiment, integration occurs via homologous recombination, using techniques well-known in the art. Preferably, stable genetic modification of *P. pastoris* occurs via a double cross-over event. Nett et al.,Yeast. 2003 Nov;20(15):1279-90. For example, the heterologous enzyme activities are provided with flanking sequences homologous to sequences of the host organism and successively transformed reusing a single marker. In this manner, integration occurs at a defined site in the host genome using a recyclable marker.

### Screening and Selection Processes

After transformation of the host strain with the heterologous enzymes, transformants displaying a desired glycosylation phenotype are selected. Selection may be performed in a single step or by a series of phenotypic enrichment and/or depletion steps using any of a variety of assays or detection methods. Phenotypic characterization may be carried out manually or using automated high-throughput screening equipment. Commonly, a host microorganism displays protein N-glycans on the cell surface, where various glycoproteins are localized.

One may screen for those cells that have the highest concentration of terminal GlcNAc on the cell surface, for example, or for those cells which secrete the protein with the highest terminal GlcNAc content. Such a screen may be based on a visual method, like a staining procedure, the ability to bind specific terminal GlcNAc binding antibodies or lectins conjugated to a marker (such lectins are available from E.Y. Laboratories Inc., San Mateo, CA), the reduced ability of specific lectins to bind to terminal mannose residues, the ability to incorporate a radioactively labeled sugar *in vitro,* altered binding to dyes or charged surfaces, or may be accomplished by using a Fluorescence Assisted Cell Sorting (FACS) device in conjunction with a fluorophore labeled lectin or antibody (Guillen, 1998).

Accordingly, intact cells may be screened for a desired glycosylation phenotype by exposing the cells to a lectin or antibody that binds specifically to the desired *N*-glycan. A wide variety of oligosaccharide-specific lectins are available commercially (e.g., from EY Laboratories, San Mateo, CA). Alternatively, antibodies to specific human or animal *N*-glycans are available commercially or may be produced using standard techniques. An appropriate lectin or antibody may be conjugated to a reporter molecule, such as a chromophore, fluorophore, radioisotope, or an enzyme having a chromogenic substrate (Guillen et al., 1998. Proc. Natl. Acad. Sci. USA 95(14): 7888-7892)).

Screening may then be performed using analytical methods such as spectrophotometry, fluorimetry, fluorescence activated cell sorting, or scintillation counting. In other cases, it may be necessary to analyze isolated glycoproteins or *N*-glycans from transformed cells. Protein isolation may be carried out by techniques known in the art. In a preferred embodiment, a reporter protein is secreted into the medium and purified by affinity chromatography (e.g. Ni-affinity or glutathione - S-transferase affinity chromatography). In cases where an isolated *N*-glycan is preferred, an enzyme such as endo- b-*N*-acetylglucosaminidase (Genzyme Co., Boston, MA; New England Biolabs, Beverly, MA) may be used to cleave the *N*-glycans from glycoproteins. Isolated proteins or *N*-glycans may then be analyzed by liquid chromatography (e.g. HPLC), mass spectroscopy, or other suitable means. U.S. Patent No. 5,595,900 teaches several methods by which cells with desired extracellular carbohydrate structures may be identified. In a preferred embodiment, MALDI-TOF mass spectrometry is used to analyze the cleaved N-glycans.

Prior to selection of a desired transformant, it may be desirable to deplete the transformed population of cells having undesired phenotypes. For example, when the method is used to engineer a functional mannosidase activity into cells, the desired transformants will have lower levels of mannose in cellular glycoprotein. Exposing the transformed population to a lethal radioisotope of mannose in the medium depletes the population of transformants having the undesired phenotype, i.e. high levels of incorporated mannose (Huffaker TC and Robbins PW., Proc Natl Acad Sci U S A. 1983 Dec;80(24):7466-70). Alternatively, a cytotoxic lectin or antibody, directed against an undesirable *N*-glycan, may be used to deplete a transformed population of undesired phenotypes (e.g., Stanley P and Siminovitch L. Somatic Cell Genet 1977 Jul;3(4):391-405). U.S. Patent No. 5,595,900 teaches several methods by which cells with a desired extracellular carbohydrate structures may be identified. Repeatedly carrying out this strategy allows for the sequential engineering of more and more complex glycans in lower eukaryotes.

To detect host cells having on their surface a high degree of the human-like N-glycan intermediate Gal₂GlcNAc₂ Man₃GlcNAc₂, for example, one may select for transformants that allow for the most efficient transfer of Galactose by GalT from UDP-Galactose in an *in vitro* cell assay. This screen may be carried out by growing cells harboring the transformed library under selective pressure on an agar plate and transferring individual colonies into a 96-well microtiter plate. After growing the cells, the cells are centrifuged, the cells resuspended in buffer, and after addition of UDP-Galactose and GalT, the release of UDP is determined either by HPLC or an enzyme linked assay for UDP. Alternatively, one may use radioactively labeled UDP-Galactose and GalT, wash the cells and then look for the release of radioactive Galactose by β-galactosidase. All this may be carried manually or automated through the use of high throughput screening equipment. Transformants that release more UDP, in the first assay, or more radioactively labeled Galactose in the second assay, are expected to have a higher degree of Gal₂GlcNAc₂Man₃GlcNAc₂ on their surface and thus constitute the desired phenotype. Similar assays may be adapted to look at the N-glycans on secreted proteins as well.

Alternatively, one may use any other suitable screen such as a lectin binding assay that is able to reveal altered glycosylation patterns on the surface of transformed cells. In this case the reduced binding of lectins specific to terminal mannoses may be a suitable selection tool. *Galantus nivalis* lectin binds specifically to terminal a-1,3 mannose, which is expected to be reduced if sufficient mannosidase II activity is present in the Golgi. One may also enrich for desired transformants by carrying out a chromatographic separation step that allows for the removal of cells containing a high terminal mannose content. This separation step would be carried out with a lectin column that specifically binds cells with a high terminal mannose content (e.g., *Galantus nivalis* lectin bound to agarose, Sigma, St.Louis, MO) over those that have a low terminal mannose content.

### Host Cells

The techniques described herein for identification and disruption of undesirable host cell glycosylation genes, e.g. *OCH1,* is understood to be applicable for these and/ or other homologous or functionally related genes in other eukaryotic host cells such as other yeast and fungal strains (*See* WO 02/00879). Additionally, other preferred host cells are deficient in Alg3p encoding for Dol-P-Man:Man₅GlcNAc₂ PP-Dol mannosyl-transferase activity (*See* WO 03/056914).

Pichia pastoris inherently lack β1,4-galactose linkages, fucose, and terminal sialic acid. Unlike the N-glycans of mammalian glycoproteins these sugars are not usually found on glycoproteins produced in yeast and filamentous fungi. The present invention provides methods for engineering host cells to produce galactose residues onto glycoproteins and essentially lack fucose and sialic acid residues on the glycoproteins. In another embodiment, those hose cells that produce fucose or sialic acid can be modified to have reduced or eliminated fucosyltransferase activity or sialyltransferase activity. The glycoprotein compositions produced from the host of the present invention are, therefore, essentially free of fucose and sialic acid residues. A significant advantage of the present invention is that the host cells produce galactosylated, fucose-free and sialic acid-free gly coproteins without *ex vivo* modification with fucosidase and sialidase treatment.

Other preferred host cells include fungal hosts that lack mannosylphosphorylation with respect to glycans (USSN 11/020,808). Still other preferred host cells include fungal hosts that lack β-mannosylation with respect to glycans (USSN 60/566,736).

Another aspect of the present invention thus relates to a Pichia pastoris host strain expressing glycoproteins comprising modified *N*-glycans that resemble those made by human-cells. Preferably, glycosylation enzymes or catalytic domains and the like are targeted to a subcellular location along the host cell secretory pathway where they are capable of functioning, and preferably, where they are designed or selected to function most efficiently.

Examples of modifications to glycosylation which can be affected using a method according to this embodiment of the invention are: (1) engineering a Pichia pastoris host cell to trim mannose residues from Man₈GlcNAc₂ to yield a Man₅GlcNAc₂ *N-*glycan; (2) engineering Pichia pastoris host cell to add an *N-*acetylglucosamine (GlcNAc) residue to Man₅GlcNAc by action of GlcNAc transferase 1; (3) engineering a Pichia pastoris host cell to functionally express an enzyme such as an *N*-acetylglucosaminyl Transferase (GnTI, GnTII, GnTIII, GnTIV, GnTV, GnTVI, GnTIX), mannosidase II, fucosyltransferase (FT), galactosyl transferase (GaIT) or a sialyltransferase (ST).

By repeating the method, increasingly complex glycosylation pathways can be engineered into a target host. In one preferred embodiment, the host organism is transformed two or more times with DNA libraries including sequences encoding glycosylation activities. Selection of desired phenotypes may be performed after each round of transformation or alternatively after several transformations have occurred. Complex glycosylation pathways can be rapidly engineered in this manner.

### Target Glycoproteins

The methods described herein are useful for producing glycoproteins, especially glycoproteins used therapeutically in humans. Glycoproteins having specific glycoforms may be especially useful, for example, in the targeting of therapeutic proteins. For example, mannose-6-phosphate has been shown to direct proteins to the lysosome, which may be essential for the proper function of several enzymes related to lysosomal storage disorders such as Gaucher's, Hunter's, Hurler's, Scheie's, Fabry's and Tay-Sachs disease, to mention just a few. Likewise, the addition of one or more sialic acid residues to a glycan side chain may increase the lifetime of a therapeutic glycoprotein *in vivo* after administration. Accordingly, Pichia pastoris host cells may be genetically engineered to increase the extent of terminal sialic acid in glycoproteins expressed in the cells. Alternatively, sialic acid may be conjugated to the protein of interest *in vitro* prior to administration using a sialic acid transferase and an appropriate substrate. Changes in growth medium composition may be employed in addition to the expression of enzyme activities involved in human-like glycosylation to produce glycoproteins more closely resembling human forms (S. Weikert, et al., Nature Biotechnology, 1999, 17,1116-1121; Werner, Noe, et al 1998 Arzneimittelforschung 48(8):870-880; Weikert, Papac et al., 1999; Andersen and Goochee 1994 Cur. Opin. Biotechnol. 5: 546-549; Yang and Butler 2000 Biotechnol.Bioengin. 68(4): 370-380). Specific glycan modifications to monoclonal antibodies (e.g. the addition of a bisecting GlcNAc) have been shown to improve antibody dependent cell cytotoxicity (Umana P., et al. 1999), which may be desirable for the production of antibodies or other therapeutic proteins.

Therapeutic proteins are typically administered by injection, orally, pulmonary, or other means. Examples of suitable target glycoproteins which may be produced according to the invention include, without limitation: erythropoietin, cytokines such as interferon- a, interferon- b, interferon- g, interferon- w, and granulocyte-CSF, GM-CSF, coagulation factors such as factor VIII, factor IX, and human protein C, antithrombin III, thrombin, soluble IgE receptor a-chain, IgG, IgG fragments, IgG fusions, IgM, interleukins, urokinase, chymase, and urea trypsin inhibitor, IGF-binding protein, epidermal growth factor, growth hormone-releasing factor, annexin V fusion protein, angiostatin, vascular endothelial growth factor-2, myeloid progenitor inhibitory factor-1, osteoprotegerin, a-1-antitrypsin, a- feto proteins, DNase II, kringle 3 of human plasminogen, glucocerebrosidase, TNF binding protein **1,** follicle stimulating hormone, cytotoxic T lymphocyte associated antigen **4** - Ig, transmembrane activator and calcium modulator and cyclophilin ligand, soluble TNF receptor Fc fusion, glucagon like protein 1 and IL-2 receptor agonist.

### Secretory Signal Sequence

It is also preferred to associate a nucleic acid sequence encoding a secretory signal with a sequence of interest encoding the glycoprotein. The term 'secretory signal sequence' denotes a DNA sequence that encodes a polypeptide (a 'secretory peptide') that, as a component of a larger polypeptide, directs the larger polypeptide through a secretory pathway of a cell in which it is synthesized. The larger polypeptide is commonly cleaved to remove the secretory peptide during transit through the secretory pathway. To direct a polypeptide into the secretory pathway of a host cell, a secretory signal sequence (also known as a leader sequence, prepro sequence or pre sequence) is provided in an expression vector. The secretory signal sequence may be that of, without limitation, a wild-type sequence related to a glycoprotein, sequence encoding *S. cerevisiae* Suc2 signal sequence, sequence encoding *Pichia* Pho2 signal sequence, sequence encoding *Pichia* Prc1 signal sequence, sequence encoding *S. cerevisiae* alpha-mating factor (αMF) signal sequence, sequence encoding bovine lysozyme C signal sequence. The secretory signal sequence is operably linked to a nucleic acid sequence, i.e., the two sequences are joined in the correct reading frame and positioned to direct the newly synthesized polypeptide into the secretory pathway of the host cell. Secretory signal sequences are commonly positioned 5' to the DNA sequence encoding the polypeptide of interest, although certain secretory signal sequences may be positioned elsewhere in the DNA sequence of interest (See, e.g., Welch et al., U.S. Pat. No. 5,037,743; Holland et al., U.S. Pat. No. 5,143,830).

Alternatively, the secretory signal sequence contained in the polypeptides of the present invention is used to direct other polypeptides into the secretory pathway. The present invention provides for such fusion polypeptides. The secretory signal sequence contained in the fusion polypeptides of the present invention is preferably fused amino-terminally to an additional peptide to direct the additional peptide into the secretory pathway. Such constructs have numerous applications known in the art. For example, these novel secretory signal sequence fusion constructs can direct the secretion of an active component of a normally non-secreted protein, such as a receptor. Such fusions may be used *in vivo* or *in vitro* to direct peptides through the secretory pathway.

Glycoproteins produced by the methods of the present invention can be isolated by techniques well-known in the art. The desired glycoproteins are purified and separated by methods such as fractionation, ion exchange, gel filtration, hydrophobic chromatography and affinity chromatography.

The following are examples which illustrate the compositions and methods of this invention. These examples should not be construed as limiting: the examples are included for the purposes of illustration only.

### EXAMPLE 1

### Construction of promoter cassettes and expression vectors

The 800bp promoter for the *PpOCH1* gene was amplified using primers RCD48 (SEQ ID NO:15) (5'-TATGCG-GCCGCGGCTGATGATATTTGCTACGA-3') and RCD 134 (SEQ ID NO:16) (5'-CCTCTCGAGTGGACACAGGAGACTCAGAAACAG-3') and the 400bp promoter for the *PpSEC4* gene was amplified using primers RCD156 (SEQ ID NO: 17) (5'-CTTCTCGAGGAAGTAAAGTTGGCGAAACTT-3') and RCD157 (SEQ ID NO:18) (5'-CTTAGCGGCCGCGATTGTTCGTTTGAGTAGTTT-3'). The PCR products were cloned into the **pCR2.1** cloning vector (Invitrogen) and sequenced. The OCH1 and *SEC4* promoters were then subcloned into the vector **pJN261** (Nett et al., Yeast. 2003 Nov;20(15):1279-90) in place of the GAPDH promoter using the introduced *Xho*I/*Not*I restriction sites to create plasmids **pRCD360** and **pRCD362,** respectively.

The *PpHIS3* promoter was PCR amplified using primers RCD152 (SEQ ID NO: 19) (5'-CTTCTCGAGGGCAT-TCAAAGAAGCCTTGGG-3') and RCD153 (SEQ ID NO:20) (5'-CTTAGCGGCCGCTGAGTGGTCATGTGGGAACTT-3'), cloned into plasmid pCR2.1 and sequenced. The *Xho*I/*Not*I sites were then used to subclone the *PpHIS3* promoter into plasmid pTA18 to replace the *PpPMA1* strong promoter with the weaker *PpHIS3* promoter and create plasmid **pRCD351,** which is a NAT^{R} plasmid that rolls into the *PpHIS3* promoter.

A portion of the *PpHIS3* gene was amplified using primers RCD301 (SEQ ID NO:21) (5'-CCTGGATCCAACA-GACTACAATGACAGGAG-3') and RCD302 (SEQ ID NO:22) (5'-CCTGCATGCCTCGAGCTTGCCGGCGTCTAAATAGCCGTTGAAG-3') and inserted into **pUC19** using the *Bam-*HI/*Sph*I restriction sites to create plasmid **pRCD391.** This vector contains a 1.2 Kb portion of the *PpHIS3* locus as well as *Xho*I and NgoMIV sites engineered into the primer RCD302 (SEQ ID NO:22). The G418^{R} gene was inserted as a *Bgl*II/*Sac*I fragment from **pUG6** (Wach et al., 1994) into the *Bam*HI/*Sac*I sites of **pRCD391** to create **pRCD392.**

A 1.2 Kb portion of the *PpTRP1* gene was amplified from *P. pastoris* genomic DNA with primers RCD307 (SEQ ID NO:23) (5'-CCTGTCGACGCTGCCGGCAAG CTCGAGTTTAAGCGGTGCTGC-3') and RCD308 (SEQ ID NO:24) (5'-CCT GGATCCTTTGGCAAAAACCAGCCCTGGTGAG-3'). The amplified fragment was inserted into **pUC19** using *Bam*HI/*Sal*I sites to create plasmid **pRCD399.** The PAT gene conferring resistance to phosphinothricin was released from plasmid **pAG29** (Goldstein and McCusker, 1999) using *Bgl*II/*Sac*I and inserted into **pRCD399** digested with *Bam*HI/*Sac*I to create the *PpTRP1*/PAT roll-in plasmid **pRCD401.**

### EXAMPLE 2

### Cloning of Galactose Transporters

### Schizosaccharomyces pombe UDP Galactose Transporter

The *S. pombe* gene encoding the UDP Galactose Transporter (*SpGMS1*+, Genbank AL022598) referred to as *SpUGT* was PCR amplified from *S. pombe* genomic DNA (ATCC24843) in two pieces to eliminate a single intron. Primers RCD164 (SEQ ID NO:7) (5'-CCTTGCGGCCGCATGGCTGTCAAGGGCGACGATGTCAAA-3') and RCD177 (SEQ ID NO:8) (5'-ATTCGAGAATAGTTAAGTGTCAAAATCAATGCACTATTTT-3') were used to amplify the 5'96bp of the gene and primers RCD176 (SEQ ID NO:9) (5'-AAAATAGTGCATTGATTTTGACACTTAACTATTCTCGAAT-3') and RCD165 (SEQ ID NO: 10) (5'-CCTTTTAATTAATTAATGCTTATGATCAACGTCCTTAGC-3') to amplify the 3'966bp. Subsequently, primers RCD164 (SEQ ID NO:7) and RCD165 (SEQ ID NO: 10) were used to overlap the two amplified products into a single PCR fragment comprising one contiguous open reading frame with *Not*I and *Pac*I sites introduced at the ends. This PCR product was cloned into the **pCR2.1** vector (Invitrogen) and sequenced. The *Not*I and *Pac*I sites were then used to subclone this gene into plasmid **pJN335**, which contains a cassette that fuses a gene downstream of the *P. pastoris* GAPDH promoter. The 400bp *PpOCH1* transcriptional terminator was then PCR amplified using primers RCD202 (SEQ ID NO:25) (5'-TCCTTAATTAAAGAAAGCTAGAGTAAAATAGAT-3') and RCD203 (SEQ ID NO:26) (5'-TCCCTCGAGGATCAT-GTTGATCAACTGAGACCG-3') and cloned into **pCR2.1.** Subsequently a triple ligation was performed to insert the GAPDH promoter/ *SpUGT* gene fusion as an *Xho*I/*Pac*I fragment and the *PpOCH1-TT* as a *Pac*I/*Xho*I fragment into a single *Xho*I site in plasmid **pTA18** to create plasmid **pRCD257.** The new plasmid, **pRCD257,** is a NAT^{R} containing vector that contains the *GAPDH-SpGALE-OCH1TT* fusion along with a second cassette that contains a truncated version of the human GnTII gene fused to the *ScVAN1* transmembrane domain driven by the *PpPMAI* promoter.

The *SpUGT* gene was also inserted into the *Not*I/*Pac*I sites of **pRCD360** with the *OCH1* promoter and **pRCD362** with the *SEC4* promoter to create plasmids **pRCD385** and **pRCD387,** respectively. The *P_{OCH1}-SpUGT-PpCYC1TT* cassette from **pRCD385** and *P_{SEC4}-SpUGT-PpCYC1*TT cassette from **pRCD387** were inserted into the **pRCD392** HIS3/G418^{R} roll-in vector using *Xho*I/*Ngo*MIV to create *P. pastoris HIS3* / G418^{R} roll-in expression plasmids **pRCD393** and **pRCD394,** respectively.

### Drosophila melanogaster UDP Galactose Transporter

The *D. melanogaster* gene encoding the UDP Galactose Transporter (Genbank BAB62747) referred to as *DmUGT* was PCR amplified from a *D. melanogaster* cDNA library (UC Berkeley Drosophila Genome Project, ovary 1-ZAP library GM) and cloned into the **pCR2.1** PCR cloning vector and sequenced. Primers DmUGT-5' (SEQ ID NO:11) (5'-GGCTCGAGCGGCCGCCACCATGAATAGCATACACATGAACGCCAATACG-3') and DmUGT-3' (SEQ ID NO:12) (5'-CCCTCGAGTTAAT-TAACTAGACGCGCGGCAGCAGCTTCTCCTCATCG-3') were used to amplify the gene, which introduced *Not*I and *Pac*I sites at the 5' and 3' ends, respectively. The *Not*I and *Pad* sites were then used to subclone this gene fused downstream of the *PpOCHI* and promoter at the *Not*I/*Pac*I sites in **pRCD393** to create plasmid **pSH263.**

### Homo sapiens UDP Galactose Transporter

The *H*. *Sapiens* genes encoding the UDP Galactose Transporter 1 (Genbank #BAA95615) and UDP Galactose Transporter 2 (Genbank #BAA95614) referred to as *hUGT1* and *hUGT2,* respectively, were amplified from human prostate cDNA (marathon ready dDNA, Clontech). The *hUGT1* gene was amplified with primers hUGT1-5' (SEQ ID NO:27) (5'-GGCTCGAGCGGCCGCCACCATG-GCAGCGGTTGGGGCTGGTGGTTC-3') and hUGT1-3' (SEQ ID NO:28) (5'-CC-CTCGAGTTAATTAATCACTTCACCAGCACTGACTTTGGCAG-3') and the *hUGT2* gene was amplified with primers hUGT2-5' (SEQ ID NO:29) (5'- GGCTC-GAGCGGCCGCCACCATGGCAGCGGTTGGGGCTGGTGGTTC-3') and hUGT2-3' (SEQ ID NO:30) (5'-CCCTCGAGTTAATTAACTAGGAACCCTTCACCTTG-GTGAGCAAC-3'). The PCR products were cloned into the **pCR2.1** vector (Invitrogen, Carlsbad, CA) and sequenced. The *hUGT1* and *hUGT2* genes were subsequently inserted into **pRCD393** downstream of the *PpOCH1* promoter using *Not*I/ *Pac*I to create plasmids **pSH264** and **pSH262,** respectively.

### EXAMPLE 3

### Cloning UDP-Galactose-4-Eplmerase Genes

### S. cerevisiae UDP-galactose 4-epimerase

The *S*. *cerevisiae* gene encoding UDP-galactose 4-epimerase (*ScGAL10*) was PCR amplified from *S. cerevisiae* genomic DNA using primers RCD270 (SEQ ID NO:31) (5'-TAGCGGCCGCATGACAGCTCAGTTACAAAGTGAAAG-3') and RCD271 (SEQ ID NO:32) (5'-CGTTAATTAATCAGGAAAATCTGTAGACAATCTTGG-3'). The resulting PCR product was cloned into **pCR2.1** and sequenced.

The *ScGAL10* gene was then subcloned using the *Not*I/*Pac*I sites into plasmids **pRCD393** and **pRCD394** to create plasmids **pRCD395** and **pRCD396,** respectively and also into plasmids **pRCD402** and **pRCD403** to create plasmids **pRCD404** and **pRCD405,** respectively. Plasmids **pRCD402** and **pRCD403** are expression vectors containing the *P. pastoris OCH1* and *SEC4* promoters, respectively, and the *PpCYC1* terminator and convenient restriction sites that were used to fuse the epimerases with these promoters and create a cassette that could be collectively moved into another plasmid.

### Homo sapiens UDP-g galactose 4-epimerase

The *H. sapiens* gene encoding UDP-galactose 4-epimerase (Thoden et al., (2001) JBC Vol. 276 (18) 15131-15136.), referred to as *hGALE* was PCR amplified from human kidney cDNA (marathon ready cDNA, Clontech) using primers GD7 (SEQ ID NO: 33) and GD8 (SEQ ID NO:34) with *NotI* and *PacI* sites respectively, cloned into **pCR2.1** and sequenced. The *hGALE* gene was then subcloned using *Not*I/*Pac*I sites into plasmids **pRCD406** and **pRCD407** to create plasmids **pRCD427** and **pRCD428,** respectively.

### S. pombe UDP-galactose 4-epimerase

Primers GALE2-L (SEQ ID NO:35) and GALE2-R (SEQ ID NO:36) were used to amplify the *SpGALE* gene from *S. pombe* (ATCC24843) genomic DNA. The amplified product was cloned into **pCR2.1** and sequenced. Sequencing revealed the presence of an intron (175bp) at the +66 position.

To eliminate the intron, upstream primer GD1 (SEQ ID NO:37) (94 bases) was designed. It has a *NotI* site, 66 bases upstream of the intron, followed by 20 bases preceding the intron. GD2 (SEQ ID NO:38) is the downstream primer and has a *Pac*I site. Primers GD1 (SEQ ID NO:37) and GD2 (SEQ ID NO:38) were used to amplify the *SpGALE* intronless gene from the **pCR2.1** subclone and the product cloned again into **pCR2.1** and sequenced.

### EXAMPLE 4

### Cloning of b-1,4-Galactosyltransferase Genes

### Homo sapiens b-1?4-galactosyltransferase I

The *H. sapiens* b-1,4-galactosyltransferase I gene (*hGalTI,* Genbank AH003575) was PCR amplified from human kidney cDNA (marathon ready cDNA, Clontech) using primers RCD192 (SEQ ID NO:1) (5'-GCCGCGACCTGAGCCGCCTGCCCCAAC-3') and RCD186 (SEQ ID NO:2) (5'-CTAGCTCGGTGTCCCGATGTC-CACTGT-3'). This PCR product was cloned into **pCR2.1** vector (Invitrogen, Carlsbad, CA) and sequenced. From this clone, a PCR overlap mutagenesis was performed for three purposes: 1) to remove a *Not*I site within the open reading frame while maintaining the wild-type protein sequence, 2) to truncate the protein immediately downstream of the endogenous transmembrane domain, 3) and to introduce *Asc*I and *Pac*l sites at the 5' and 3' ends for modular cloning. To do this, the 5' end of the gene up to the *Not*I site was amplified using primers RCD198 (SEQ ID NO:3) (5'-CTTAGGCGCGCCGGCCGCGACCTGAGCCGCCTGCCC-3') and RCD201 (SEQ ID NO:4) (5'-GGGGCATATCT-GCCGCCCATC-3') and the 3' end was amplified with primers RCD200 (SEQ ID NO:5) (5'-GATGGGCGGCAGATAT-GCCCC-3') and RCD199 (SEQ ID NO:6) (5'-CTTCTTAATTAACTAGCTCGGTGTCCCGATGTCCAC-3'). The products were overlapped together with primers 198 and 199 to resynthesize the ORF with the wild-type amino acid sequence while eliminating the *Not*I site. The new truncated *hGalTI* PCR product was cloned into **pCR2.1** vector (Invitrogen, Carlsbad, CA) and sequenced. The introduced *Asc*I/*Pac*I sites were then used to subclone the fragment into plasmid **pRCD259,** which is a *PpURA3*/*HYG^{R}* roll-in vector, to create **pRCD260.** A library of yeast targeting sequence transmembrane domains as described in WO 02/00879, which is incorporated by reference, was ligated into the *Not*I/*Asc*I sites located upstream of the *hGalTI* gene to create plasmids **pXB20-pXB67.**

### Homo sapiens b-1,4-galactosyltransferase II

A truncated form of the *H. sapiens* b-1,4-galactosyltransferase II gene (*hGalTII,* Genbank AF038660) was PCR amplified from human kidney cDNA (marathon ready cDNA, Clontech) using primers RCD292 (SEQ ID NO:39) (5'-CTTAGGCGCGCCCAGCACCTGGCCTTCTTCAGC-3') and RCD293 (SEQ ID NO:40) (5'-CTTGTTAATTAAT-CAGCCCCGAGGGGGCCACGACGG-3'), cloned into plasmid **pCR2.1** and sequenced. This truncated clone, which eliminates part of the gene encoding the N-terminal transmembrane domain, was subcloned using the introduced As*c*I/*Pac*I sites into vector **pXB53** in place of *hGalTI* to create plasmid **pRCD378.** The plasmid, containing the gene fusion of the truncated *hGalTII* with the transmembrane domain/leader sequence-encoding portion of the *S. cerevisiae MNN2* gene is driven by the *PpGAPDH* promoter.

### Homo sapiens b-1,4-galactosyltransferase III

A truncated form of the *H. Sapiens* b-1,4-galactosyltransferase III gene (*hGalTIII,* Genbank AF038661) was PCR amplified from human kidney cDNA (marathon ready cDNA, Clontech) using primers RCD294 (SEQ ID NO:41) (5'-CTTAGGCGCGCCCGAAGTCTCAGTGCCCTATTTGGC-3') and RCD295 (SEQ ID NO:42) (5'-CTTGTTAATTAAT-CAGTGTGAACCTCGGAGGGCTGT-3'), cloned into plasmid **pCR2.1** and sequenced. This truncated clone, which eliminates part of the gene encoding the N-terminal transmembrane domain, was subcloned using the introduced *Asc*I/*Pac*I sites into vector **pXB53** in place of *hGalTI* to create plasmid **pRCD381.** This plasmid now contains a gene fusion of the truncated *hGalTIII* with the transmembrane domain/leader sequence-encoding portion of the *S. cerevisiae MNN2* gene driven by the *PpGAPDH* promoter.

### EXAMPLE 5

### Expression of hGalTI with SpUGT in a strain producing complex N-glycans

The **pRCD257** plasmid containing the human GnTII gene and the *SpGMS1* + gene (SpUGT) was introduced into strain **RDP27. RDP27** is a mutant strain of *P. pastoris* that has *och1* and *alg3* deletions, and that has been transformed with plasmids **pSH49** and **pPB104** which contain active fusion constructs of mouse Mannosidase IB and human GnTI, respectively as well as plasmid **pPB103**, which contains the *K. lactis* UDP-GlcNAc transporter and **pBK64** which contains the reporter protein K3 (Choi et al. 2003). After selection on nourseothricin, 16 transformants were selected to determine the glycosylation of the expressed reporter protein K3. In two of these transformants, the expected complex human glycosylation structure GlcNAc₂Man₃GlcNAc₂ was observed and these strains were designated **RDP30-10 (****Figure 8A****)** and **RDP30-13.** A portion of the *hGalTI* gene/leader fusion plasmid library was transformed into strain **RDP30-10** and transformants were selected on minimal medium containing hygromycin. N-glycans released from K3 secreted by the resulting strains were analyzed on MALDI-TOF MS. A molecular shift in mass consistent with the addition of one galactose sugar was observed on N-glycans from transformants of two different leader constructs, **pXB53** and **pXB65.** The first, **pXB53** consists of *hGalTI* fused to the ScMnn2(s) leader (referred to here as ScMnn2(s)/hGalTI) and the other was a fusion with the ScMnn1(m) leader. Analysis of the N-glycans released from K3 from **RDP37 (RDP30-10** transformed with **pXB53)** by MALDI-TOF revealed approximately 10-20% GlcNAc₂Man₃GlcNAc₂ being converted to Gal GlcNAc₂Man₃GlcNAc₂ and a lesser amount (1-2%) to Gal₂GlcNAc₂Man₃GlcNAc₂ (p**XB53,** **Figure 8B**). A lesser amount of conversion (3-5%) to GalGlcNAc₂Man₃ GlcNAc₂ but no observable Gal₂ GlcNAc₂ Man₃ GlcNAc₂ was observed for the second fusion (**pXB65**).

### EXAMPLE 6

### Expression of hGalTI and ScGAL10 in a strain producing hybrid N-glycans

The *ScGAL10* gene encoding UDP-galactose 4-epimerase was subcloned with *Not*I/*Pac*I into the NAT^{R} vectors **pTA18** and **pRCD351** in place of hGnTII, which inserts the epimerase gene in front of the strong PMAI promoter and the weaker *PpHIS3* promoter, respectively, to create plasmids **pRCD331** (*P_{PMA1}-ScGAL10*) and **pRCD352** (*P_{H1S3}-ScGAL10*)*,* respectively. The plasmids were linearized (**pRCD331** with *Sac*I in the *PpPMA1* promoter and **pRCD352** with *Bgl*II in the *PpHIS3* promoter) and transformed into strain **PBP-3** (US Pat. Appl. No. 20040018590). Strain **PBP-3** is a mutant strain of *P. pastoris,* which has an *och1* deletion and has been transformed with plasmids **pSH49** and **pPB104** which contain active fusion constructs of mouse Mannosidase IB and human GnTI, respectively as well as plasmid **pPB103**, which contains the *K. lactis* UDP-GlcNAc transporter and plasmid **pBK64** which contains the reporter protein K3 (Choi et al.). This strain produces hybrid N-glycans of the structure GlcNAcMan₅GlcNAc₂ on secreted proteins. Resulting transformants selected on YPD medium containing Nourseothricin were analyzed by PCR with primers RCD285 (SEQ ID NO:43) (5'-TACGAGATTCCCAAATATGATTCC-3') and RCD286 (SEQ ID NO:44) (5'-ATAGTGTCTCCATATGGCTTGTTC-3') and by expressing the reporter protein K3 and analyzing the released N-glycans to ensure that the strains maintained the hybrid GlcNAcMan₅GlcNAc₂ glycan structure. One strain transformed with the **pR CD352 (*****P**_{HIS3}-ScGAL10*) construct was designated **RDP38-18.** This strain was transformed with the plasmid **pXB53** (containing the Mnn2(s)/hGalTI fusion construct and the HYG^{R} and *PpURA3* genes) after linearization with *SalI* (located in *PpURA3*). Transformants were selected on YPD medium with Hygromycin and screened by expressing K3 and determining the size of the N-glycans. A large portion (-2/3) of the N-glycans released from K3 purified from **RDP39-6** strains (**Figure 10A**) contained one additional hexose (HexGlcNAcMan₅ GlcNAc₂) as compared with those from **RDP38-18,** which were mostly GlcNAcMan₅ GlcNAc₂. Furthermore, the additional hexose residue could be removed by subsequent incubation with soluble b-1,4-galactosidase, but not a-1,3-galactosidase or a-1,2-mannosidase, indicating that the addition of a single galactose to the terminal GlcNAc with a specific linkage (b-1,4) was catalyzed by *hGalTI* in this strain.

### EXAMPLE 7

### Expression of hGalTI and SeGAL10 in a strain producing complex N-glycans

The *P. pastoris* strain **YSH-44** was constructed, which displays complex N-glycans with a GlcNAc₂Man₃GlcNAc₂ structures. **YSH-44** is a mutant strain of *P. pastoris* deleted for *och1* and transformed with plasmids **pSH49, pPB104, pKD53,** and **pTC53** which contain active fusion constructs of mouse Mannosidase IB, human GnTI, *D. melanogaster* Mannosidase II, and human GnTII, respectively as well as plasmid **pPB103,** which contains the *K. lactis* UDP-GlcNAc transporter and plasmid **pBK64** which contains the reporter protein K3 (Hamilton et al., Science. 2003 Aug 29;301(5637):1244-6.). This strain was transformed with the **pXB53** plasmid containing a Mnn2(s)/hGalTI fusion construct and transformants were selected on YPD medium with hygromycin. Several transformants were analyzed by purifying K3 and analyzing the released N-glycans by MALDI-TOF MS. Each of the transformants analyzed yielded a majority of N-glycans with a GlcNAc₂Man₃GlcNAc₂ structure and a minority (∼5%) consistent with a single hexose addition **(YSH-71**). However, although this peak always correlated with the introduction of *hGalTI,* it was completely recalcitrant to b-1,4-galactosidase. Subsequently, several of these strains were transformed with plasmids **pRCD395** and **pRCD396** (*PpHIS3*/G418^{R} plasmids containing *P_{OCH1}-ScGAL10* and *P_{SEC4}-ScGAL10,* respectively) after linearization with *Bgl*II*,* selected on G418, and the resulting strains were named **YSH-83** and **YSH-84,** respectively. N-glycans released from secreted K3 were analyzed by MALDI-TOF MS.. The resulting transformants were selected on YPD medium containing G418 and N-glycans released from purified, secreted K3 from these strains were analyzed by MALDI-TOF MS. A majority of N-glycans from these transformants were of three structures, Gal₂GlcNAc₂Man₃GlcNAc₂(∼0-25%) or GalGlcNAc₂Man₃GlcNAc₂ (∼40-50%), with the rest of the N-glycans retaining the GlcNAc₂Man₃GlcNAc₂ structure displayed by the parental **YSH-44** strain. The relative amount of N-glycans remained unchanged irrespective of whether the ***ScGAL10*** epimerase gene was driven by the *PpOCH1* promoter (**YSH-83**) or the *PpSEC4* promoter **(YSH-84).** **Figure 9B** shows a MALDI-TOF MS of the N-glycans released from **YSH-84.**

### EXAMPLE 8

### Construction of a Epimerase/Transferase Fusion Construct;

The *SpGALE* gene was amplified using primers RCD326 (SEQ ID NO:45) (5'-CTTGGCGCGCCATGACTGGT-GTTCATGAAGGGACT-3') and RCD329 (SEQ ID NO:46) 5'-CCTGGATCCCTTATATGTCTTGGTATGGGTCAG-3'), cloned into the **pCR2.1** vector (Invitrogen) and sequenced. A truncated portion of the *hGalTI* gene eliminating the first 43 amino acid (hGalTIΔ43) was amplified using primers RCD328 (SEQ ID NO:47) (5'-CTTGGATCC GGTGGT-GGCCGCGACCTGAGC-CGCCTGCCC-3') and RCD199 (SEQ ID NO:48) (5'-CTTCTTAATTAA CTAGCTCGGTGTC-CCGATGTCCAC-3') cloned into the **pCR2.1** vector (Invitrogen) and sequenced. The *SpGALE* clone was then digested with *Asc*I/*Bam*HI and the hGalTI clone digested with *Bam*HI/*Pac*I and both were inserted into **pRCD452** digested with *AscI*/*PacI.* The plasmid **pRCD452** contains the G418 resistance marker and *GAPDHICYC1* cassette with the *ScMNN2(s)*/*hGalTI* fusion. The *Asc*I/*Bam*HI *SpGALE* and *Bam*HI/*Pac*I hGalTI∧43 fragments were ligated in place of the *Asc*I/*Pac*I released *hGalTI* to create **pRCD461.** This new plasmid, **pRCD461** contains a *ScMNN2* (s)*lSpGALE*/*hGalTI* fusion where the *Sp*GalE and hGalTI proteins are encoded in a single polypeptide separated by a four amino acid (GSGG) linker containing the *Bam* HI site, and driven by the *PpGAPDH* promoter.

### EXAMPLE 9

### Expression of a Galactosyl transferase, epimerase and transporter in a strain producing complex N-glycans

Plasmids **pXB53,** containing the active *hGalTI*-53 gene fusion, and **pRCD378,** containing an *hGalTII*-53 fusion, were linearized with *Xho*I adjacent to the HYG^{R} marker and blunted with T4 DNA polymerase (New England Biolabs, Beverly, MA). Plasmid **pRCD381**, containing a *hGalTIII*-53 gene fusion, was linearized with *Hind*III adjacent to the *URA3* gene and blunted with T4 polymerase. The three epimerase genes *ScGAL10, SpGALE* and *hGALE* were then digested from plasmids **pRCD404, pRCD406,** and **pRCD427,** respectively, with *XhoI*/*Sph*I*,* blunted with T4 DNA polymerase, and inserted into the three linearized transferase plasmids. This generated nine new double transferase/epimerase HYG^{R} plasmids: **pRCD424** with *hGalTI-*53 and *ScGAL10,* **pRCD425** with *hGalTI*-53 and *SpGALE,* **pRCD438** with *hGalTI-*53 and *hGALE,* **pRCD439** with *hGalTII*-53 and *ScGAL10,* **pRCD440** with *hGalTII*-53 and *SpGALE,* **pRCD441** with *hGalTII*-53 and *hGALE,* **pRCD442** with *hGalTIII*-53 and *ScGAL10,* **pRCD443** with *hGalTIII-53* and *SpGALE,* and **pRCD447** with *hGalTIII-*53 and *hGALE.* Subsequently, the strain **YSH44** was transformed sequentially with these double HYG^{R} plasmids (linearized with *Xba*I) and the G418^{R} plasmids **pRCD393, pSH262, pSH263** and **pSH264** containing the *SpUGT, hUGT2, DmUGT,* and *hUGT1* UDP-Gal transporter encoding genes, respectively (linearized with *Age*I). Thus, a series of strains was created that each contained a different combination of transferase, epimerase and transporter. First, the different UDP-Gal transporters were compared in strains that contained *hGalTI*-53 and *SpGALE.* The introduction of the *DmUGT* gene resulted in virtually all of the complex glycans having two terminal galactose residues (Gal₂GlcNAc₂Man₃GlcNAc₂), whereas the other three transporter genes resulted in a profile of complex glycans virtually identical to that obtained with only the transferase and epimerase (**Figure 11A-11E**). Second, the epimerase genes were compared in strains with the *hGalTI*-53 fusion and active *DmUGT* gene by introducing **pSH263** into strains with **pRCD424, pRCD425** or **pRCD438.** The combinations of Gal genes with each of the three epimerase genes were equivalent in generating Gal₂GlcNAc₂Man₃GlcNAc complex N-glycans on secreted K3. Finally, the three human transferase fusion constructs *hGalTI-53, hGalTII-53,* and *hGalTII-53* were compared in strains with *DmUGT* and *SpGALE* by introducing **pRCD425, pRCD440** and **pRCD443** into strains transformed with **pSH263.** Here, *hGalTII-53* was slightly less efficient in transferring Gal as approximately 10% of the complex N-glycans in the strain with *hGalTI-53* had only a single galactose (GalGlcNAc₂Man₃GlcNAc₂) where as all the observable complex N-glycans in the strain with *hGalTI-*53 were bi-galactosylated Gal₂GlcNAc₂Man₃GllcNAc₂ **(****Figure 12A - 12B**). Moreover, *hGalTIII-53,* was significantly less efficient than either *hGalTI*-53 or *hGalTII-53* as 60-70% of the complex N-glycans contained 0-1 galactose residues (GlcNAc₂Man₃GlcNAc₂ or GalGllcNAc₂Man₃GlcNAc₂) whereas only 30-40% were Gal₂GlcNAc₂Man₃GlcNAc₂(**Figure 12A - 12C**).

### EXAMPLE 10

### Expression of a Galactosyl transferase, epimerase and transporter using a single plasmid construct

The G418^{R} plasmid containing *P_{OCH1}-DmUGT,* **pSH263,** was linearized by digesting with *Sac*I, then blunted with T4 DNA polymerase (□□□□□□□□□□□New England Biolabs). The *P_{SEC4}-SpGALE* gene was digested from plasmid **pRCD405** with *Xho*I/ *Sph*I and blunted with T4 DNA polymerase. The blunt *SpGALE* was then inserted into the blunt *Sac*I site of **pSH263** to create plasmid **pRCD446**, which is a double transporter/epimerase G418^{R} plasmid. **pRCD446** was then linearized with *Eco*RI and blunted with T4 DNA polymerase. The *P_{GAPDH} ScMNN2*(s)/*hGalTI* fusion construct was released from **pXB53** with *BglII*/*BamHI* and blunted with T4 DNA polymerase. The blunt *ScMNN2*(s)/*hGalTI* was then inserted into the blunt *EcoRI* site of **pRCD446** to create plasmid **pRCD465,** which is a triple G418^{R} plasmid containing *ScMNN2*(s)/ *hGalTI, SpGALE,* and *DmUGT. P. pastoris* **YSH-44,** transformed with **pRCD465** was designated **RDP80**. The N-glycan profile showed a single peak at 1663 m/z corresponding to the mass of Gal₂GlcNAc₂Man₃GlcNAc₂ [C] (**Figure 14A**).

The HYG^{R} plasmid containing *hGalTI*-53 and *SpGALE,* **pRCD425,** was linearized with *Afl*II and blunted with T4 DNA polymerase. The *DmUGT* gene was released from **pSH263** with *NotI*/*PacI* and inserted into plasmid **pRCD405** digested with *NotI*/*PacI* to create plasmid **pRCD468,** which contains a *P_{SEC4}-DmUGT-CYC1-TT* fusion that can be released as a single cassette. **pRCD468** was digested with *XhoI*/*SalI* to release the *DmUGT* cassette and blunted with T4 DNA polymerase. The blunted *DmUGT* was inserted into the blunt *Afl*II site of **pRCD425** to create plasmid **pRCD466,** which is a HYG^{R} triple plasmid with *hGalTI-53, SpGALE,* and *DmUGT.*

The HYG^{R} plasmid containing *hGalTI*-53 and *hGALE* **pRCD438** was linearized with AflII and blunted with T4 DNA polymerase. **pRCD468** was digested with *Xho*I/ *Sal*I to release the *DmUGT* cassette and blunted with T4 DNA polymerase. The blunted *DmUGT* was inserted into the blunt *Afl*II site of **pRCD438** to create plasmid **pRCD467,** which is a HYG^{R} triple plasmid with *hGalTI-53, hGALE,* and *DmUGT.*

### In vitro b-galactosidase Digest

N-glycans (2µg) from *P. pastoris* strain **RDP80** were incubated with 3mU β1,4 galactosidase (QA bio, San Mateo, CA) in 50 mM NH 4 HCO 3, pH6.0 at 37°C for 16-20 hours. N-glycan analysis in **Figure 14B** shows a predominant peak at 1430 m/z [A], which corresponds to the mass of the N-glycan GlcNAc₂Man₃GlcNAc₂, confirming galactose transfer in **Figure 14A****.**

### In vitro Sialyltransferase Reaction

K3 purified from strain **RDP80** (200 µg) was incubated with 50 mg CMP-sialic acid and 15 mU rat recombinant a□(2,6)-(N)-sialyltransferase (Calbiochem) in 50 mM NH 4HCO 3, pH6.0 at 37°C for 16-20 hours. N-glycan were then released by PNGaseF digest and detected on MALDI-TOF MS. The spectrum of the glycans showed an increase in mass following sialyltransferase treatment (**Figure 14C**) when compared with those from **RDP80** (**Figure 14A**). The spectrum as shown in **Figure 14C** depicts a predominant peak at 2227 m/z [X], which corresponds to the mass of the N-glycan NANA₂Gal₂GlcNAc₂Man₃GlcNAc₂ further confirming that the N-glycans produced by strain **RDP80** is human-type Gal₂GlcNAc₂Man₃GlcNAc₂.

### Example 11

### Epimerase sequence alignment

Sequence alignment of epimerases was performed using CLUSTAL. The nucleotide sequences and/or amino acid sequences of the Sequence Listing were used to query sequences in the GenBank, SwissProt, BLOCKS, and Pima II databases. These databases, which contain previously identified and annotated sequences, were searched for regions of homology using BLAST (Basic Local Alignment Search Tool). (See, e.g., Altschul, S. F. (1993) J. Mol. Evol 36:290-300; and Altschul et al. (1990) J. Mol. Biol. 215:403-410.) BLAST produced alignments of both nucleotide and amino acid sequences to determine sequence similarity. Other algorithms could have been used when dealing with primary sequence patterns and secondary structure gap penalties. (See, e.g., Smith, T. et al. (1992) Protein Engineering 5:35-51.)

### EXAMPLE 12

### Materials

MOPS, sodium cacodylate, manganese chloride, UDP-galactose and CMP-N-acetylneuraminic acid were from Sigma. TFA was from Aldrich. b1,4-galactosyltransferase from bovine milk were from Calbiochem. Protein N-glycosidase F, mannosidases, and oligosaccharides were from Glyko (San Rafael, CA). DEAE ToyoPearl resin was from TosoHaas. Metal chelating 'HisBind' resin was from Novagen (Madison, WI). 96-well lysate-clearing plates were from Promega (Madison, WI). Protein-binding 96-well plates were from Millipore (Bedford, MA). Salts and buffering agents were from Sigma (St. Louis, MO). MALDI matrices were from Aldrich (Milwaukee, WI).

### Shake-flask cultivations

A single colony was picked from an YPD plate (<2 weeks old) containing the strain of interest and inoculated into 10 ml of BMGY media in a 50ml 'Falcon' centrifuge tube. The culture was grown to saturation at 24° C (approx. 48 hours). The seed culture is transferred into a 500ml baffled volumetric flask containing 150 ml of BMGY media and grown to OD₆₀₀ of 5±2 at 24° C (approx. 18 hours). The growth rate of the cells was determined as the slope of a plot of the natural logarithm of OD₆₀₀ against time. The cells were harvested from the growth medium (BMGY) by centrifugation at 3000g for 10 minutes, washed with BMMY and suspended in 15 ml of BMMY in a 250 ml baffled volumetric flask. After 24 hours, the expression medium flask is harvested by centrifugation (3000g for 10 minutes) and the supernatant analyzed for K3 production.

### Bioreactor Cultivations

A 500ml baffled volumetric flask with 150ml of BMGY media was inoculated with 1 ml of seed culture (see flask cultivations). The inoculum was grown to an OD₆₀₀ of 4-6 at 24° C (approx 18 hours). The cells from the inoculum culture was then centrifuged and resuspended into 50ml of fermentation media (per litre of media: CaSO₄.2H₂O 0.30g, K₂SO₄ 6.00g, MgSO₄.7H₂O 5.00g, Glycerol 40.0g, PTM₁ salts 2.0ml, Biotin 4x10⁻³ g, H₃PO₄ (85%) 30ml, PTM1 salts per litre: CuSO₄.H₂O 6.00, Nal 0.08g, MnSO₄.7H₂O 3.00g, NaMoO₄.2H₂O 0.20g, H₃BO₃ 0.02g, CoCl₂.6H₂O 0.50g, ZnCl₂ 20.0g, FeSO₄.7H₂O 65.0g, Biotin 0.20g, H₂SO₄(98%) 5.00ml).

Fermentations were conducted in 3 litre dished bottom (1.5 litre initial charge volume) Applikon bioreactors. The fermentors were run in a fed-batch mode at a temperature of 24° C, and the pH was controlled at 4.5±0.1 using 30% ammonium hydroxide. The dissolved oxygen was maintained above 40% relative to saturation with air at 1 atm by adjusting agitation rate (450-900 rpm) and pure oxygen supply. The air flow rate was maintained at 1 vvm. When the initial glycerol (40g/l) in the batch phase is depleted, which is indicated by an increase of DO, a 50% glycerol solution containing 12 ml/l of PTM₁ salts was fed at a feed rate of 12 ml/l/h until the desired biomass concentration was reached. After a half an hour starvation phase, the methanol feed (100% Methanol with 12 ml/l PTM₁) is initiated. The methanol feed rate is used to control the methanol concentration in the fermentor between 0.2 and 0.5%. The methanol concentration is measured online using a TGS gas sensor (TGS822 from Figaro Engineering Inc.) located in the offgass from the fermentor. The fermentors were sampled every eight hours and analyzed for biomass (OD₆₀₀, wet cell weight and cell counts), residual carbon source level (glycerol and methanol by HPLC using Aminex 87H) and extracellular protein content (by SDS page, and Bio-Rad protein assay).

### Reporter protein expression, purification and release of N-linked glycans

The K3 domain, under the control of the alcohol oxidase 1 (*AOX1*) promoter, was used as a model protein and was purified using the 6xHistidine tag as reported previously (Choi et al., Proc Natl Acad Sci USA. 2003 Apr 29;100(9):5022-7). The glycans were released and separated from the glycoproteins by a modification of a previously reported method (Papac and Briggs 1998). After the proteins were reduced and carboxymethylated, and the membranes blocked, the wells were washed three time with water. The protein was deglycosylated by the addition of 30 ml of 10 mM NH₄ HCO₃ pH 8.3 containing one milliunit of N-glycanase (Glyko). After 16 hr at 37°C, the solution containing the glycans was removed by centrifugation and evaporated to dryness.

### Protein Purification

Kringle 3 was purified using a 96-well format on a Beckman BioMek 2000 sample-handling robot(Beckman/Coulter Ranch Cucamonga, CA). Kringle 3 was purified from expression media using a C-terminal hexa-histidine tag. The robotic purification is an adaptation of the protocol provided by Novagen for their HisBind resin. Briefly, a 150uL (µL) settled volume of resin is poured into the wells of a 96-well lysate-binding plate, washed with 3 volumes of water and charged with 5 volumes of 50mM NiSO4 and washed with 3 volumes of binding buffer (5mM imidazole, 0.5M NaCl, 20mM Tris-HCL pH7.9). The protein expression media is diluted 3:2, media/PBS (60mM PO4, 16mM KCl, 822mM NaCl pH7.4) and loaded onto the columns. After draining, the columns are washed with 10 volumes of binding buffer and 6 volumes of wash buffer (30mM imidazole, 0.5M NaCl, 20mM Tris-HCl pH7.9) and the protein is eluted with 6 volumes of elution buffer (1M imidazole, 0.5M NaCl, 20mM Tris-HCl pH7.9). The eluted glycoproteins are evaporated to dryness by lyophilyzation.

### Release of N-linked Glycans

The glycans are released and separated from the glycoproteins by a modification of a previously reported method (Papac, et al. A. J. S. (1998) Glycobiology 8, 445-454). The wells of a 96-well MultiScreen IP (Immobilon-P membrane) plate (Millipore) are wetted with 100uL of methanol, washed with 3X150uL of water and 50uL of RCM buffer (8M urea, 360mM Tris, 3.2mM EDTA pH8.6), draining with gentle vacuum after each addition. The dried protein samples are dissolved in 30uL of RCM buffer and transferred to the wells containing 10uL of RCM buffer. The wells are drained and washed twice with RCM buffer. The proteins are reduced by addition of 60uL of 0.1M DTT in RCM buffer for 1hr at 37oC. The wells are washed three times with 300uL of water and carboxymethylated by addition of 60uL of 0.1M iodoacetic acid for 30min in the dark at room temperature. The wells are again washed three times with water and the membranes blocked by the addition of 100uL of 1% PVP 360 in water for 1hr at room temperature. The wells are drained and washed three times with 300uL of water and deglycosylated by the addition of 30uL of 10mM NH₄HCO₃ pH 8.3 containing one milliunit of N-glycanase (Glyko). After 16 hours at 37°C, the solution containing the glycans was removed by centrifugation and evaporated to dryness.

**Miscellaneous:** Proteins were separated by SDS/PAGE according to Laemmli (Laemmli 1970).

### Matrix Assisted Laser Desorption Ionization Time of Flight Mass Spectrometry

Molecular weights of the glycans were determined using a Voyager DE PRO linear MALDI-TOF (Applied Biosciences) mass spectrometer using delayed extraction. The dried glycans from each well were dissolved in 15uL of water and 0.5uL spotted on stainless steel sample plates and mixed with 0.5uL of S-DHB matrix (9mg/mL of dihydroxybenzoic acid, 1mg/mL of 5-methoxysalicilic acid in 1:1 water/acetonitrile 0.1% TFA) and allowed to dry.

Ions were generated by irradiation with a pulsed nitrogen laser (337nm) with a 4ns pulse time. The instrument was operated in the delayed extraction mode with a 125ns delay and an accelerating voltage of 20kV. The grid voltage was 93.00%, guide wire voltage was 0.10%, the internal pressure was less than 5 X 10-7 torr, and the low mass gate was 875Da. Spectra were generated from the sum of 100-200 laser pulses and acquired with a 2 GHz digitizer. Man₅GlcNAc₂ oligosaccharide was used as an external molecular weight standard. All spectra were generated with the instrument in the positive ion mode. The estimated mass accuracy of the spectra was 0.5%.

## Claims

1. A recombinant *Pichia pastoris* host cell producing human-like glycoproteins characterized as having a terminal β-galactose residue and essentially lacking fucose and sialic acid residues on the glycoprotein, wherein the host cell expresses: a nucleic acid molecule encoding β-galactosyltransferase activity and a nucleic acid molecule encoding UDP-galactose C4 epimerase activity.

2. The host cell of claim 1, wherein the β1,4-galactosyltransferase activity is selected from: human GalT I, GalT II, GalT III, GalT IV, GalT V, GalT VI, GalT VII, bovine GalTI, *X. leavis* GalT and *C. elegans* GalTII.

3. The host cell of claim 1 or 2 wherein the UDP-galactose C4 epimerase activity is encoded by a gene selected from SpGALE, ScGAL10 and hGALE.

4. The host cell of claim 1. 2, or 3 which expresses UDP-galactose transport activity.

5. The host cell of claim 4 wherein the UDP-galactosc transport activity is encoded by a gene selected from SpUGT, hUGT1, hUGT2, and DmUGT.

6. The host cell of any preceding claim which exhibits an elevated level of UDP-galactose.

7. The host cell of any preceding claim which is impaired in initiating 1,6 mannosyltransferase activity with respect to the glycan on the glycoprotein.

8. A method for producing human-like glycoproteins in a *Pichia pastoris* host cell as defined in any one of claims 1-7, the method comprising the step of catalyzing the transfer of a galactose residue from UDP-galactose onto an acceptor substrate in a β linkage by expression of a β1,4GalT activity and introducing into the host a UDP galactose 4-epimerase activity.

9. The method of claim 8 wherein a galactose residue is transferred-onto a glycan selected from GlcNAcMan3GlcNAc2, GlcNAc2Man3GlcNAc2, GlcNAc3Man3GlcNAc2, GlcNAc4ManGlcNAc, GlcNAc5Man3GlcNAc2 GlcNAc6Man3GlcNAc2, GlcNAcMan4GlcNAc2, GlcNAcMan5GlcNAc2, GlcNAc2ManSGlcNAc2 and GlcNAc3Man5GlcNAc2.

10. The method of claims 8 or 9 wherein at least 60 mole % of galactose is transferred onto a glycoprotein wherein said 60 mol % is determined with respect to % total neutral glycans.

## Patentansprüche

1. Eine rekombinante *Pichia-pastoris*-Wirtszelle, die menschenähnliche Glykoproteine erzeugt, **gekennzeichnet durch** das Vorliegen eines terminalen β-Galactoserests und im Wesentlichen das Fehlen von Fucose- und Sialinsäureresten am Glykoprotein, wobei die Wirtszelle exprimiert: ein Nukleinsäuremolekül, das β-Galactosyltransferaseaktivität kodiert, und ein Nukleinsäuremolekül, das UDP-Galactose-C4-Epimeraseaktivität kodiert.

2. Die Wirtszelle nach Anspruch 1, wobei die β1,4-Galactosyltransferaseaktivität ausgewählt ist aus: menschlicher GalT I, GalT II, GalT III, GalT IV, GalT V, GalT VI, GalT VII, boviner GalT I, *X.-leavis*-GalT und *C.-elegans*-GalTII.

3. Die Wirtszelle nach Anspruch 1 oder 2, wobei die UDP-Galactose-C4-Epimeraseaktivität durch ein Gen kodiert wird, ausgewählt aus SpGALE, ScGAL10 und hGALE.

4. Die Wirtszelle nach Anspruch 1, 2 oder 3, die UDP-Galactosetransportaktivität exprimiert.

5. Die Wirtszelle nach Anspruch 4, wobei die UDP-Galactosetransportaktivität durch ein Gen kodiert wird, ausgewählt aus SpUGT, hUGT1, hUGT2 und DmUGT.

6. Die Wirtszelle nach einem vorhergehenden Anspruch, die einen erhöhten UDP-Galactosespiegel aufweist.

7. Die Wirtszelle nach einem vorhergehenden Anspruch, dessen Initiierung von 1,6-Mannosyltransferaseaktivität in Bezug auf das Glykan am Glykoprotein beeinträchtigt ist.

8. Ein Verfahren zur Erzeugung von menschenähnlichen Glykoproteinen in einer *Pichia-pastoris*-Wirtszelle wie in einem der Ansprüche 1-7 definiert, wobei das Verfahren den Schritt umfasst: Katalysierens des Transfers eines Galactoserestes von UDP-Galactose auf ein Akzeptorsubstrat in einer β-Verknüpfung durch Expression einer β1,4-GalT-Aktivität und Einführen einer UDP-Galactose-4-Epimeraseaktivität in den Wirt.

9. Das Verfahren nach Anspruch 8, wobei ein Galactoserest auf ein Glykan, ausgewählt aus GlcNAcMan3GlcNAc2, GlcNAc2Man3GlcNAc2, GlcNAc3Man3GlcNAc2, GlcNAc4ManGlcNAc, GlcNAc5Man3GlcNAc2, GlcNAc6Man3GlcNAc2, GlcNAcMan4GlcNAc2, GlcNAcMan5GlcNAc2, GlcNAc2Man5GlcNac2 und GlcNAc3Man5GlcNAc2, transferiert wird.

10. Das Verfahren nach Anspruch 8 oder 9, wobei wenigstens 60 Mol-% Galactose auf ein Glykoprotein transferiert werden, wobei die 60 Mol-% bezogen auf die Gesamt-% an neutralen Glykanen ermittelt werden.

## Revendications

1. Cellule hôte *Pichia pastoris* recombinante produisant des glycoprotéines de type humain **caractérisée** comme ayant un résidu β-galactose terminal et dépourvues essentiellement de résidus de fucose et d'acide sialique sur la glycoprotéine; dans laquelle la cellule hôte exprime: une molécule d'acide nucléique codant une activité β1,4-galactosyltransférase et une molécule d'acide nucléique codant une activité UDP-galactose-C4 épimérase.

2. Cellule hôte selon la revendication 1, dans laquelle l'activité β1,4-galactosyltransférase est sélectionnée parmi GalTI, GalTII, GalTIII, GalTIV, GalTV, GalTVI, GalTVII humaines, GalTI bovine, GalT de *X. leavis* et GalTII de *C. elegans.*

3. Cellule hôte selon les revendications 1 ou 2, dans laquelle l'activité UDP-galactose-C4 épimérase est codée par un gène sélectionné parmi *SpGALE, ScGAL10 et hGALE.*

4. Cellule hôte selon les revendications 1, 2 ou 3, qui exprime une activité de transport d'UDP-galactose.

5. Cellule hôte selon la revendication 4, dans laquelle l'activité de transport d'UDP-galactose est codée par un gène sélectionné parmi *SpUGT, hUGT1, hUGT2* et *DmUGT.*

6. Cellule hôte selon l'une quelconque des revendications précédentes qui présente un taux élevé d'UDP-galactose.

7. Cellule hôte selon l'une quelconque des revendications précédentes qui est altérée dans l'induction de l'activité 1,6-mannosyltransférase par rapport au glycane sur la glycoprotéine.

8. Procédé de production de glycoprotéines de type humain dans une cellule hôte de *Pichia pastoris* telle que définie dans l'une quelconque des revendications 1 à 7, le procédé comprenant l'étape consistant à catalyser le transfert d'un résidu galactose d'une UDP-galactose sur un substrat accepteur dans une liaison β par l'expression d'une activité β1,4GalT et à introduire dans l'hôte une activité UDP-galactose-C4 épimérase.

9. Procédé selon la revendication 8, dans lequel un résidu galactose est transféré sur un glycane sélectionné parmi GlcNAcMan₃GlcNAc₂, GlcNAc₂Man₃GlcNAc₂, GlcNAc₃Man₃GlcNAc₂, GlcNAc₄Man₃GlcNAc₂, GlcNAc₅Man₃GlcNAc₂, GlcNAc₆Man₃GlcNAc₂, GlcNAcMan₄GlcNAc₂, GlcNAcMan₅GlcNAc₂, GlcNAc₂Man₅GlcNAc₂ et GlcNAc₃Man₅GlcNAc₂.

10. Procédé selon les revendications 8 ou 9, dans lequel au moins 60 % en moles de galactose sont transférés sur une glycoprotéine, où lesdits 60 % en moles sont déterminés par rapport au pourcentage total de glycanes neutres.
